# FASCICULE DE BREVET EUROPEEN

(11) **EP 2 195 323 B1**
(45) Date de publication et mention de la délivrance du brevet: **28.12.2011**
(21) Numéro de dépôt: 08863264.1
(22) Date de dépôt: 02.10.2008
(51) Int. Cl.: C07D 493/10, C07D 493/20, A61K 31/35, A61P 33/00

(54) **Composés polyspiranniques, leur application dans le traitement du paludisme ou de la toxoplasmose et leur procédé de préparation**
Polyspiranverbindungen, ihre Anwendung bei der Behandlung von Malaria oder Toxoplasmose sowie Verfahren zu ihrer Herstellung
Polyspirane compounds, use thereof in the treatment of malaria or toxoplasmosis and method for their preparation

(30) Priorité: 03.10.2007 FR 0706929
(43) Date de publication de la demande: 16.06.2010
(73) Titulaire: Université Joseph Fourier, 38041 Grenoble Cedex 09 (FR)
(72) Inventeur: WONG, Yung-Sing, F-38400 Saint Martin-d´Heres (FR); PEUCHMAUR, Marine, Aimée, F-38140 Apprieu (FR); MARECHAL, Eric, F-38000 Grenoble (FR); BOTTE, Cyrille, F-38100 Grenoble (FR); VIAL, Henri, Joseph, F-34080 Montpellier (FR); SAIDANI, Nadia, F-44300 Nantes (FR)
(74) Mandataire: Grosset-Fournier, Chantal Catherine
(86) Numéro de dépôt international: PCT/FR2008/001378
(87) Numéro de publication internationale: WO 2009/077677

(56) Documents cités:
- MARINE PEUCHMAUR ET AL.: "Studies towards the synthesis of Aculeatin C" SYNLETT., vol. 18, 19 octobre 2007 (2007-10-19), pages 2902-2906, XP002483975 DETHIEME INTERNATIONAL, STUTTGART.
- JÖRG HEILMANN ET AL.: "Antiprotozoal activity and cytotoxicity of novel 1,7-dioxadispiro(5.1.5.2)pentadeca-9,12-di en-11-one derivatives from amomum aculeatum" HELVETICA CHIMICA ACTA., vol. 83, 2000, pages 2939-2945, XP002483976 CHVERLAG HELVETICA CHIMICA ACTA, BASEL. cité dans la demande

## Description

La présente invention concerne de nouveaux composés polyspiranniques, leur application dans le traitement du paludisme ou de la toxoplasmose et leur procédé de préparation.

Le phylum des apicomplexes compte plusieurs milliers d'espèces de parasites unicellulaires responsables de maladies infectieuses majeures, dont le paludisme (causé par des parasites du genre *Plasmodium*), la toxoplasmose (*Toxoplasma gondii*), la pyroplasmose (*Babesia*), la néosporose (*Neospora*)...

Le paludisme (ou malaria) est la maladie parasitaire la plus répandue dans le monde. Tous les pays d'Europe, non endémiques, connaissent un paludisme dit d'importation.

Il existe plus d'une centaine d'espèces de *Plasmodium,* seules quatre espèces parasitent l'homme : *Plasmodium malariae, Plasmodium vivax, Plasmodium ovale* et *Plasmodium falciparum,* de loin la plus virulente (80 % des cas mortels). L'infection est, elle, véhiculée par un moustique femelle du genre Anophèle (*Anopheles gambiae*).

Dans les années 1950-1960, l'utilisation intensive d'insecticides, tel que le dichlorodiphényltrichloroéthane (DDT), permit de réduire considérablement les zones endémiques.

Cette stratégie fut cependant abandonnée en 1969 : en plus des risques environnementaux et liés à la santé des populations, l'utilisation intensive d'insecticides a favorisé l'apparition de souches de moustiques résistantes.

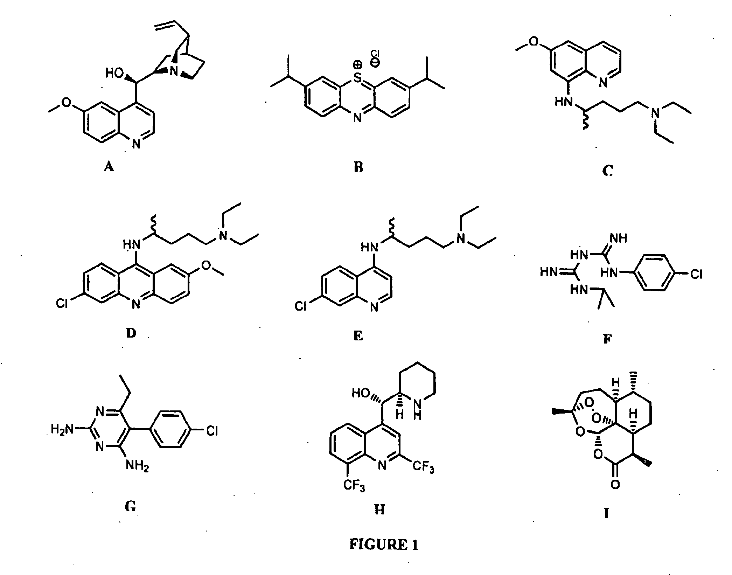

En terme de traitement et prophylaxie (Wiesner J., Ortmann R., Jomaa H., Schlitzer M., New antimalarial drugs. Angew. Chem. Int. Ed. 2003, 42, 5274-5293 ; Biagini G. A., O'Neill P. M., Bray P. G., Ward S. A., Current drug development portfolio for antimalarial therapies. Curr. Opin. Pharm. 2005, 5, 473-478), dès le 18^{ème} siècle, en Amérique latine, l'écorce de quinquina (*Cinchona*) est utilisée dans le traitement des fièvres, mais ce n'est qu'en 1820 que la molécule active est isolée : la quinine (figure 1). En 1891, la première molécule synthétique est utilisée en traitement sur l'homme pour cette maladie : il s'agit du bleu de méthylène. Les années de recherche qui suivirent donnèrent lieu à la synthèse de nombreux composés, telles que la pamaquine, la mépacrine et la chloroquinc (1946). Cependant, rapidement, les premiers cas de résistance (Hyde J. E., Drug-resistant malaria. Trends in parasitology 2005, 21, 494-498) à la chloroquine sont apparus à cause de son utilisation excessive et probablement de doses insuffisantes. La seconde guerre mondiale et la guerre du Viêt-Nam ont donné lieu à un nouvel élan dans la recherche de molécules actives originales aboutissant au développement notamment du proguanil, puis de la pyriméthamine et de la méfloquine. Enfin, en 1972, l'artémisinine, une endopéroxyde-lactone sesquiterpènique, a été isolée des parties aériennes de l'armoise annuelle *Artemisia annua,* plante utilisée en Chine dès 340 de notre ère dans le traitement des fièvres.

Les échecs des nouveaux médicaments contre le paludisme sont survenus à un taux alarmant, par exemple, la résistance à la méfloquine est apparue seulement 5 ans après son introduction comme traitement prophylactique dans certaines parties de la Thaïlande, tandis que la résistance à l'atovaquone a été encore plus rapide, intervenant la même année que son lancement (Biagini G. A., O'Neill P. M., Bray P. G., Ward S. A., Current drug development portfolio for antimalarial therapies. Curr. Opin. Pharm. 2005, 5, 473-478).

L'artémisinine et ses dérivés sont les seuls composés actuels n'ayant donné lieu à aucune résistance de la part du parasite.

L'OMS (http://www.who.int/topics/malaria/fr/. 10 Avril 2007) préconise donc une utilisation de ces molécules en combinaison avec d'autres composés afin d'éviter l'apparition de nouveaux phénomènes de résistance. Ces molécules présentent cependant des inconvénients, notamment leur très faible solubilité.

Les phénomènes de résistance, spécifique ou croisée, chez le parasite se développant de plus en plus rapidement (Hyde J. E., Drug-resistant malaria. Trends in parasitology 2005, 21, 494-498), il est indispensable de trouver de nouvelles molécules possédant à la fois des structures originales et agissant sur de nouvelles cibles biologiques.

Tout comme le paludisme, la toxoplasmose est une maladie parasitaire dont l'agent est un protozoaire de forme arqué, *Toxoplasma gondii.* Présente partout dans le monde, la toxoplasmose (Montoya J. G., Liesenfeld O., Toxoplasmosis. Lancet 2004, 363, 1965-1976) est l'infection parasitaire la plus commune chez les animaux à sang chaud, mammifères et oiseaux.

La toxoplasmose est elle aussi une maladie à trois acteurs : le parasite (*Toxoplasma gondii,* la seule espèce connue à ce jour), l'homme (l'hôte intermédiaire) et le félin, généralement un chat domestique ou sauvage (l'hôte définitif). Le cycle de développement de *Toxoplasma gondii* peut être indirect, en passant par un ou plusieurs hôtes intermédiaires, mais il peut également être direct, c'est-à-dire sans hôte intermédiaire.

La toxoplasmose est généralement asymptômatique ; malgré cela, le parasite *Toxoplasma gondii* reste essentiellement présenté comme un pathogène opportuniste pouvant provoquer des maladies graves chez les patients immunodéprimés, encéphalites cérébrales ou autres complications psychiatriques (désorientation, anxiété, dépression, psychoses...), ou chez le foetus.

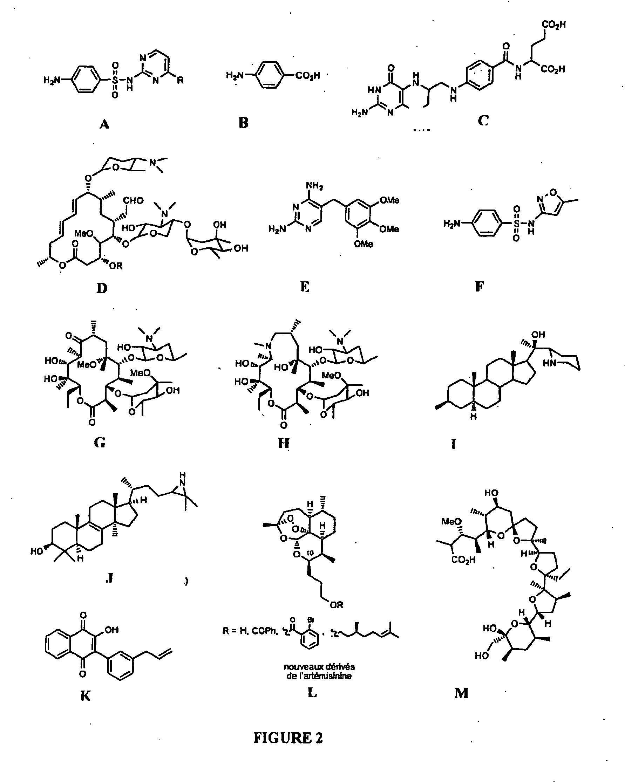

Classiquement, lorsque la toxoplasmose touche des personnes immunocompétentes, aucun traitement n'est utilisé à moins que les symptômes ne se révèlent intenses ou persistants : les traitements existants ne visent en effet qu'à supprimer la prolifération du parasite, pendant la phase aiguë, jusqu'à ce que l'immunité soit acquise. Il n'existe actuellement aucun traitement contre la toxoplasmose chronique puisque aucun médicament n'est capable d'éliminer les kystes tissulaires. Finalement, très peu de molécules sont présentes sur le marché (figure 2) :
- les sulfonamides. Elles sont actives contre *Toxoplasma gondii,* parmi elles, les plus utilisées sont la sulfadiazine et la sulfamérazine. Le mode d'action de ces composés est identique sur la plupart des microorganismes : ils entrent en compétition avec l'acide para-aminobenzoïque (PABA, ex-vitamine B10) et inhibent ainsi la synthèse des acides nucléiques.
- la pyriméthamine. Utilisé dans le traitement du paludisme, cet agent antifolinique est également actif contre *Toxoplasma gondii.*

La sulfadiazine et la pyriméthamine agissent en synergie et constituent donc le traitement de choix contre le parasite humain : seuls les trophozoïtes sont touchés par cette bithérapie. Cependant de nombreux effets secondaires (réactions allergiques touchant 5 à 15 % des patients) peuvent résulter de cette chimiothérapie. C'est le cas par exemple de la thrombocytopénie et de la leucopénie : pour remédier à ces problèmes, un acide folinique (leucovérine) peut être simultanément administré.

D'autres thérapies alternatives sont également utilisées (pyriméthamine en combinaison avec des sulfones, des tétracyclines ou des macrolides, comme la spiramycine), mais elles restent moins efficaces. Malgré cela, des controverses (Kravetz J. D., Federman D. G., Toxoplasmosis in pregnancy. Am. J. Med. 2005, 118, 212-216; The SYROCOT (Systematic Review on Congenital Toxoplasmosis) study group, Effectiveness of prenatal treatment for congenital toxoplasmosis: a meta-analysis of individual patient's data. Lancet 2007, 369, 115-122) persistent concernant l'efficacité des thérapies ou chimioprophylaxies existantes, notamment pour les traitements néonatals.

Les traitements actuels présentant de nombreux inconvénients et étant inefficaces contre les formes enkystées de la toxoplasmose, il semble nécessaire de trouver des médicaments à la fois moins toxiques et actifs contre les stades chroniques du parasite.

A priori, aucun phénomène de résistance (Petersen E., Toxoplasmosis. Semin. Fetal. Neonatal Med. 2007, 12, 214-223) n'a été détecté à ce jour malgré la pression médicamenteuse sur l'homme. Des cas de résistance de *T. gondii* aux sulfonamides ont pu être induits en laboratoire, cependant les risques semblent faibles.

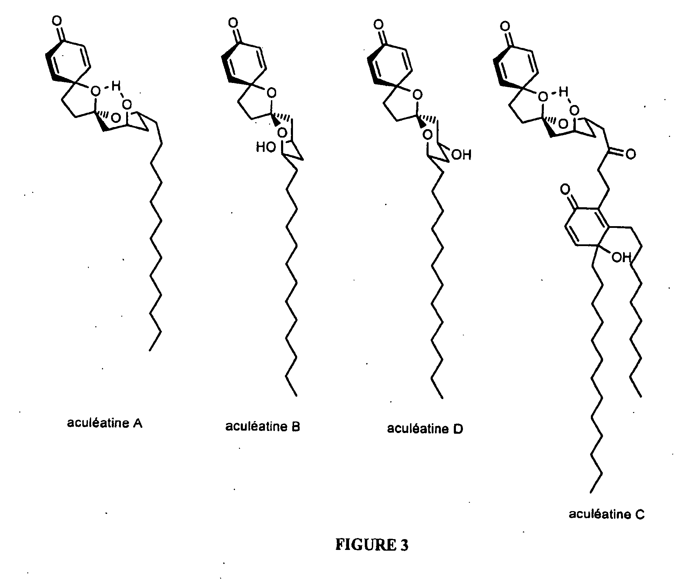

Les aculéatines naturelles (figure 3) ont été isolées en 2000 par Heilman et son équipe (J. Heilmann, S. Mayr, R. Brun, T. Rali, O. Sticher, Antiprotozoal activity and cytotoxicity of novel 1,7-dioxadispiro[5.1.5.2]pentadeca-9,12-dien-11-one derivatives from Amomum aculeatum. Helv. Chim. Acta 2000, 83, 2939-2945; J. Heilmann, R. Brun, S. Mayr, T. Rali, O. Sticher, Minor cytotoxic and antibacterial compounds from the rhizomes of Amomum aculeatum. Phytochemistry 2001, 57, 1281-1285) : ce sont des molécules possédant une structure tridimensionnelle complexe et qui présentent des propriétés anticancéreuses et antiprotozoaires moyennes (notamment antipaludiques).

Un des buts de la présente invention est de fournir de nouveaux composés polyspiranniques pour la préparation d'un médicament destiné à la prévention ou au traitement de pathologies impliquant les parasites appartenant au phylum des apicomplexes.

Un autre but de l'invention est de fournir des compositions pharmaceutiques contenant au moins un composé polyspirannique pour la prévention ou le traitement de pathologies telles que le paludisme ou la toxoplasmose.

Un autre but de l'invention est de fournir un procédé de préparation des composés polyspiranniques.

Par conséquent, la présente invention concerne l'utilisation d'au moins un composé polyspirannique, ou des sels de celui-ci physiologiquement acceptables, de formule (I) suivante : 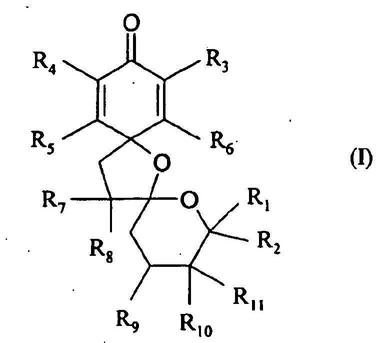 dans laquelle :
- R₁ représente l'hydrogène et R₂ représente un groupe -CH₂COX-R', dans lequel X= N, O, C ;
   lorsque X=O, R' représente un alkyle saturé ou non saturé, linéaire ou ramifié de 1 à 20 atomes de carbone ou un cycloalkyle de 3 à 8 atomes de carbone;
   lorsque X=CH₂, R' représente l'hydrogène, un alkyle saturé ou non saturé, linéaire ou ramifié de 1 à 20 atomes de carbone ou un cycloalkyle de 3 à 8 atomes de carbone;
   lorsque X=N, N peut être monosubstitué par R' ou disubstitué par R' et R" qui représentent, indépendamment l'un de l'autre, l'hydrogène ou un alkyle saturé ou non saturé, linéaire ou ramifié de 1 à 20 atomes de carbone ou un cycloalkyle de 3 à 8 atomes de carbone;

R₉ représente :
   - un groupe OH,
   - un groupe O-allyle, un groupe O-benzyle, un groupe O-CO-alkyle, un groupe O-COCH₂CO-alkyle, ledit résidu alkyle étant saturé ou non saturé, linéaire ou ramifié de 1 à 20 atomes de carbone, ou un groupe O-CO-cycloalkyle comportant 3 à 8 atomes de carbone,
ou,
- R₁ et R₂ représentent ensemble un groupe G-1: 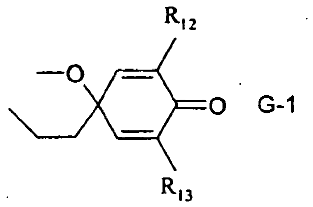
   R₁₂ et R₁₃ représentent indépendamment l'un de l'autre l'hydrogène, un groupe alkyle, saturé ou non saturé, linéaire ou ramifié de 1 à 6 atomes de carbone, un groupe cycloalkyle de 3 à 8 atomes de carbone, un groupe O-alkyle, saturé ou non saturé, linéaire ou ramifié de 1 à 6 atomes de carbone, un groupe O-cycloalkyle de 3 à 8 atomes de carbone, un atome d'halogène tel que le chlore, le fluor, le brome et l'iode ;
   R₉ représente :
      - un groupe OH,
      - un groupe alkyle saturé ou non saturé, linéaire ou ramifié de 1 à 20 atomes de carbone, un groupe cycloalkyle de 3 à 8 atomes de carbone,
      - un groupe S(O)-R" dans lequel R" est un alkyle saturé ou non saturé, linéaire ou ramifié de 1 à 20 atomes de carbone ou un groupe cycloalkyle de 3 à 8 atomes de carbone,
      - un groupe O-alkyle, un groupe O-CO-alkyle, un groupe O-COCH₂CO-alkyle, ledit résidu alkyle étant saturé ou non saturé, linéaire ou ramifié de 1 à 20 atomes de carbone, un groupe O-cycloalkyle de 3 à 8 atomes de carbone, un groupe O-CH₂-CH₂-N(CH₃)₂ ; un groupe O-CH₂-CCH,
      - un groupe 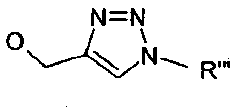 dans lequel R"' est un alkyle saturé ou non saturé, linéaire ou ramifié de 1 à 20 atomes de carbone,
      - un groupe N(R")(R"'), R" et R"' étant tels que définis ci-dessus, et quels que soient R₁ et R₂:
- R₃, R₄, R₅, R₆ représentent indépendamment l'un de l'autre l'hydrogène, un groupe alkyle, saturé ou non saturé, linéaire ou ramifié de 1 à 6 atomes de carbone, un groupe cycloalkyle de 3 à 8 atomes de carbone, un groupe O-alkyle, saturé ou non saturé, linéaire ou ramifié de 1 à 6 atomes de carbone, un groupe O-cycloalkyle de 3 à 8 atomes de carbone, un atome d'halogène tel que le chlore, le fluor, le brome et l'iode ;
- R₇ et R₈ représentent indépendamment l'un de l'autre l'hydrogène, un groupe alkyle, saturé ou non saturé, linéaire ou ramifié de 1 à 6 atomes de carbone, un groupe cycloalkyle de 3 à 8 atomes de carbone, un groupe O-alkyle, saturé ou non saturé, linéaire ou ramifié de 1 à 6 atomes de carbone, un groupe O-cycloalkyle de 3 à 8 atomes de carbone, un groupe NH-alkyle saturé ou non saturé, linéaire ou ramifié de 1 à 6 atomes de carbone, un groupe NH-cycloalkyle de 3 à 8 atomes de carbone, un groupe NHCO-R, dans lequel R peut être un groupe alkyle, saturé ou non saturé, linéaire ou ramifié de 1 à 6 atomes de carbone, un groupe cycloalkyle de 3 à 8 atomes de carbone,
- R₁₀ et R₁₁ représentent indépendamment l'un de l'autre l'hydrogène, un groupe hydroxyle, un groupe O-alkyle, le résidu alkyle étant saturé ou non saturé, linéaire ou ramifié de 1 à 8 atomes de carbone, un groupe O-cycloalkyle de 3 à 8 atomes de carbone, une amine tertiaire ;
   pour la préparation d'un médicament destiné à la prévention ou au traitement de pathologies impliquant les parasites appartenant au phylum des apicomplexes.

Un composé spirannique est un composé possédant deux cycles connectés par un même carbone. Le terme « polyspirannique » désigne par conséquent une molécule dans laquelle il existe plusieurs cycles qui sont connectés deux à deux par un même carbone.

Les composés de la formule générale (I), lorsque R₁ représente l'hydrogène et R₂ représente un groupe -CH₂COX-R', sont des composés dispiranniques dans lesquels deux carbones (C₆ et C₈) de l'hétérocycle à cinq atomes (**B**) sont connectés à deux autres cycles (**A**) et (**C**) (formule (II)):

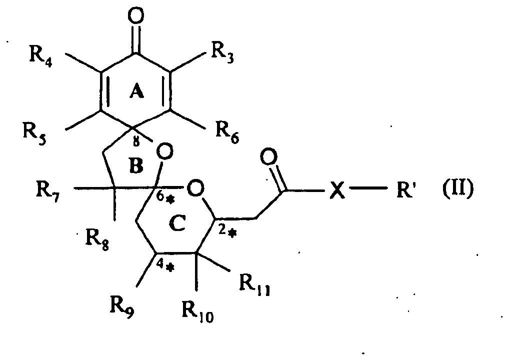

Lesdits composés dispiranniques possèdent au moins trois centres asymétriques, signalés par une étoile au niveau des carbones C₂, C₄ et C₆ du cycle (C).

La présente invention concerne par conséquent aussi bien :
- le mélange de tous les diastéréoisomères,
- le mélange des couples de diatéréoisomèrés *syn* [(2*S*, 4*R*, 6*R*) et (2*R*, 4*S*, 6*S*)] composés **10** et [(2*S*, 4*R*, 6*S*) et (2*R*, 4*S*, 6*R*)] composés **11** : 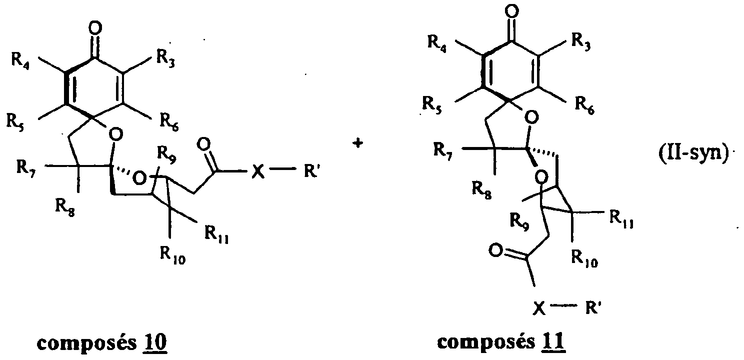
- le mélange des couples de diastéréoisomères *anti* [(2*S*, 4*S*, 6*R*) et (2*R*, 4*R*, 6*S*)] composés **12** et [(2*S*, 4*S*, 6*S*) et (2*R*, 4*R*, 6*R*)] composés **13** : 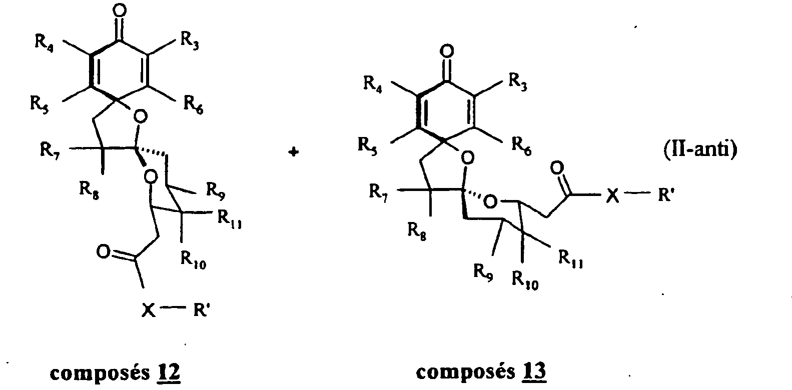
- le mélange racémique *syn* [(2*S*, 4*R*, 6*R*) et (2*R*, 4*S*, 6*S*)] composés **10,**
- le mélange racémique *syn* [(2*S*, 4*R*, 6*S*) et (2*R*, 4*S*, 6*R*)] composés **11**,
- le mélange racémique *anti* [(2*S*, 4*S*, 6*R*) et (2*R*, 4*R*, 6*S*)] composés **12,**
ou le mélange racémique *anti* [(2*S*, 4*S*, 6*S*) et (2*R*, 4*R*, 6*R*)] composés **13.**

Les composés de la formule générale (I), lorsque R₁ et R₂ représentent ensemble un groupe G-1 sont des composés tétraspiranniques dans lesquels deux carbones (C₈ et C₁₀) de l'hétérocycle à six atomes (**C**) sont connectés à deux autres cycles (**B**) et (**D**), possédant eux-mêmes chacun un atome de carbone, C6 (**B**) et C12 (**D**), connectés au cycle (**A**) et (**E**) respectivement (formule (III)):

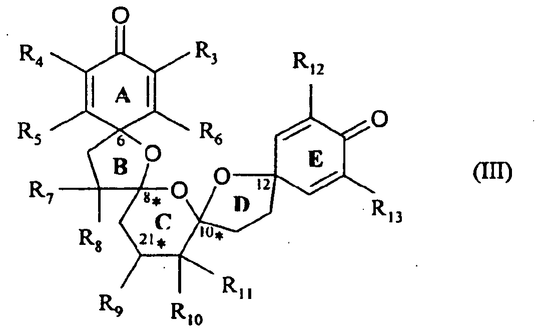

De la même manière, lesdits composés tétraspiranniques possèdent au moins trois centres asymétriques, signalés par une étoile au niveau des carbones C₈, C₁₀ et C₂₁:

Comme pour les composés dispiranniques, la présente invention concerne par conséquent aussi bien :
- le mélange de tous les diastéréoisomères,
- le mélange des couples de diastéréoisomères *syn* [(8*R*, 10*S,* 21*S*) et (8*S*, 10*R*, 21*R*)] et [(8*S*, 10*S*, 21*S*) et (8*R*, 10*R*, 21*R*)],
   ou le mélange des composés *méso* (8*R*, 10*R*, 21*S*) et (8*S*, 10*R*, 21*S*),
- le mélange racémique *syn* [(8*R*, 10*S*, 21*S*) et (8*S*, 10*R*, 21*R*)],
- le mélange racémique *syn* [(8*S*, 10*S*, 21*S*) et (8*R*, 10*R*, 21*R*)].
- le composé *méso* (8*R*, 10*R*, 21*S*),
ou le composé *méso* (8*S*, 10*S*, 21*R*),

Les composés de formules (III) lorsque R₇ et R₈, R₁₀ et R₁₁ représentent l'hydrogène, possèdent un plan de symétrie, dans le cas ou le composé est *méso* (c'est-à-dire 8*R*, 10*R*, 21*S*), au niveau du cycle portant le résidu R₉, par conséquent il n'existe dans ce type de structure que les composés suivants concernés par l'invention :
- le mélange d'un couple de diastéréoisomères *syn* [(8*R*, 10*S*, 21*S*) et (8*S*, 10*R*, 21*R*)] composés **16** et le composé *méso* [(8*R*, 10*R,* 21*S*)] composés **16:** 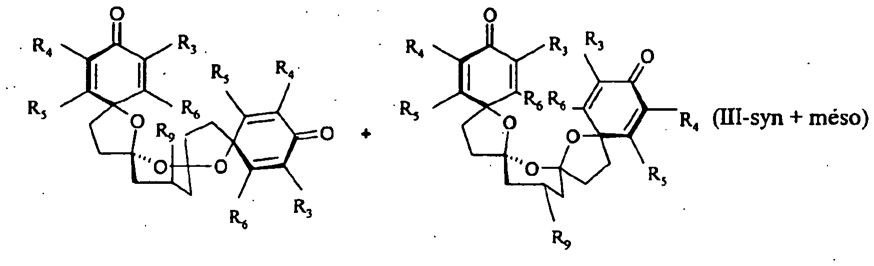
- le mélange racémique *syn* [(8*R*, 10*S*, 21*S*) et (8*S*, 10*R*, 21*R*)],
- le composé *méso* [(8*R*, 10*R*, 21*S*)].

L'expression « sels physiologiquement acceptables» signifie que les composés de la formule I, définie ci-dessus, lorsqu'ils possèdent un radical R₇ et/ou R₈ et/ou R₉ représentant une amine, peuvent exister sous forme d'ammonium quaternaire par réaction d'un acide inorganique, d'un acide organique ou d'un halogénure d'alkyle, sur l'amine.

Des exemples d'acides inorganiques permettant l'obtention de sels pharmacologiquement acceptables incluent sans être limités à ceux-ci l'acide chlorhydrique, l'acide bromhydrique, l'acide nitrique, l'acide carbonique, l'acide formique, l'acide monohydrogénocarbonique, l'acide phosphorique, l'acide monohydrogénophosphorique, l'acide dihydrogénophosphorique, l'acide perchlorique, l'acide sulfurique, l'acide monohydrogénosulfurique, l'acide iodhydrique.

Des exemples d'acides organiques permettant l'obtention de sels pharmacologiquement acceptables incluent sans être limités à ceux-ci l'acide acétique, l'acide lactique, l'acide propionique, l'acide butyrique, l'acide isobutyrique, l'acide palmique, l'acide maléique, l'acide glutamique, l'acide hydroxymaléique, l'acide malonique, l'acide benzoïque, l'acide succinique, l'acide glycolique, l'acide subérique, l'acide fumarique, l'acide mandélique, l'acide phthalique, l'acide salicylique, l'acide benzènesulfonique, l'acide p-toluènesulfonique, l'acide citrique, l'acide tartrique, l'acide méthanesulfonique, l'acide hydroxynaphthoïque.

Les sels d'acides aminés, tels que les arginates et leurs équivalents sont également inclus ainsi que les sels d'acides organiques tels que l'acide glucuronique ou l'acide galacturonique et leurs équivalents (voir, par exemple, Berge et al, "Pharmaceutical Salts", Journal of Pharmaceutical Science, 1977, 66, 1-19).

Les halogénures d'alkyle permettant l'obtention de sels pharmacologiquement acceptables incluent sans être limités à ceux-ci les bromure, iodure, fluorure ou chlorure d'alkyle dans lesquels ledit résidu alkyle est saturé ou non saturé, linéaire ou ramifié de 1 à 20 atomes de carbone, ou un groupe O-cycloalkyle de 3 à 8 atomes de carbone.

Le terme «apicomplexes» désigne des parasites possédant une combinaison caractéristique d'organelles appelée complexe apical.

Selon un autre mode de réalisation, la présente invention concerne l'utilisation d'au moins un composé polyspirannique, de formule générale (II) suivante : 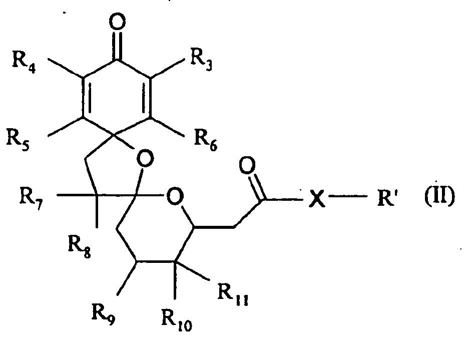 dans laquelle R₃ à R₈, R₁₀ à R₁₁, X et R' sont tels que définis ci-dessus,
et R₉ représente un groupe OH, un groupe O-allyle, un groupe O-benzyle, un groupe O-CO-alkyle, un groupe O-COCH₂-CO-alkyle, ledit résidu alkyle étant saturé ou non saturé, linéaire ou ramifié de 1 à 20 atomes de carbone, ou un groupe O-CO-cycloalkyle comportant 3 à 8 atomes de carbones.

Dans un mode de réalisation préféré, la présente invention concerne l'utilisation d'au moins un composé polyspirannique, de formule générale (III) suivante : 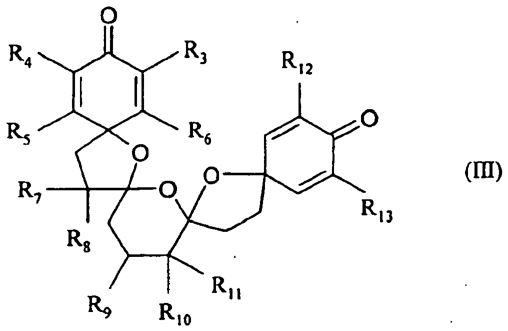 dans laquelle R₃ à R₈ et R₁₀ à R₁₃ sont tels que définis ci-dessus,
R₉ représente :
- un groupe OH,
- un groupe alkyle saturé ou non saturé, linéaire ou ramifié de 1 à 20 atomes de carbone, un groupe cycloalkyle de 3 à 8 atomes de carbone,
- un groupe S(O)-R" dans lequel R" est un alkyle saturé ou non saturé, linéaire ou ramifié de 1 à 20 atomes de carbone ou un groupe cycloalkyle de 3 à 8 atomes de carbone,
- un groupe O-alkyle, un groupe O-CO-alkyle, un groupe O-COCH₂CO-alkyle, ledit résidu alkyle étant saturé ou non saturé, linéaire ou ramifié de 1 à 20 atomes de carbone, un groupe O-cycloalkyle de 3 à 8 atomes de carbone, un groupe O-CH₂-CH₂-N(CH₃)₂ ; un groupe O-CH₂-CCH,
- un groupe 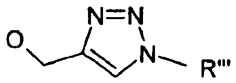 dans lequel R"' est un alkyle saturé ou
- non saturé, linéaire ou ramifié de 1 à 20 atomes de carbone,
- un groupe N(R")(R"'), R" et R"' étant tels que définis ci-dessus,

Selon un mode de réalisation préféré, lesdits parasites définis ci-dessus sont de l'espèce des *Plasmodium,* notamment *Plasmodium malariae, Plasmodium vivax, Plasmodium ovale* et *Plasmodium falciparum,* particulièrement *Plasmodium falciparum.*

Le *Plasmodium* est un genre de protozoaires parasites, composé, de plusieurs espèces.

*P. falciparum* est le plus dangereux de ces parasites causant la malaria car il entraîne le taux de mortalité le plus élevé. En outre, il représente 80 % de toutes les infections malariques humaines et 90 % des décès.

Selon un autre mode de réalisation préféré, lesdits parasites sont du genre *Toxoplasma* et notamment de l'espèce *Toxoplasma gondii.*

Le *Toxoplasma* est un genre de protozoaires parasites dont *Toxoplasma gondii* est une des espèces. L'hôte définitif du parasite de l'espèce *Toxoplasma gondii* est le chat, bien que le parasite puise être porté par une grande variété d'animaux à sang chaud dont l'homme.

Selon encore un autre mode de réalisation préféré, lesdits troubles sont le paludisme ou la toxoplasmose.

Les différentes espèces de *Plasmodium* causent le paludisme chez l'homme. Le parasite est transmis à l'homme par une piqûre d'anophèle (moustique des régions chaudes et marécageuses) et infecte les globules rouges et les cellules du foie au cours du cycle parasitaire.

L'espèce *Toxoplasma gondii* provoque la toxoplasmose chez l'homme, maladie qui est habituellement bénigne mais qui peut provoquer des affections sévères et même fatales sur le foetus dont la mère a contracté pour la première fois la maladie durant la grossesse.

Selon un autre aspect, l'invention concerne un composé polyspirannique de formule (I) suivante : 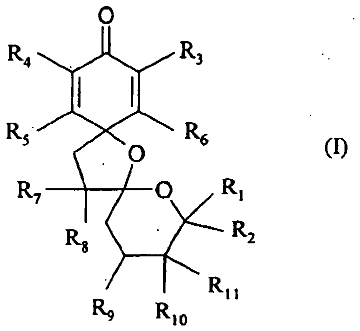 dans laquelle :
- R₁ représente l'hydrogène et R₂ représente un groupe -CH₂COX-R', dans lequel X= N,O,C;
   lorsque X=O, R' représente un alkyle saturé ou non saturé, linéaire ou ramifié de 1 à 20 atomes de carbone ou un cycloalkyle de 3 à 8 atomes de carbone;
   lorsque X=CH₂, R' représente l'hydrogène, un alkyle saturé ou non saturé, linéaire ou ramifié de 1 à 20 atomes de carbone ou un cycloalkyle de 3 à 8 atomes de carbone;
   lorsque X=N, N peut être monosubstitué par R' ou disubstitué par R' et R" qui représentent, indépendamment l'un de l'autre, l'hydrogène ou un alkyle saturé ou non saturé, linéaire ou ramifié de 1 à 20 atomes de carbone ou un cycloalkyle de 3 à 8 atomes de carbone;
   R₉ représente :
   - un groupe OH,
   - un groupe O-allyle, un groupe O-benzyle, un groupe O-CO-alkyle, un groupe O-COCH₂CO-alkyle, ledit résidu alkyle étant saturé ou non saturé, linéaire ou ramifié de 1 à 20 atomes de carbone, ou un groupe OCO-cycloalkyle de 3 à 8 atomes de carbone,
      ou,
      - R₁ et R₂ représentent ensemble un groupe G-1: 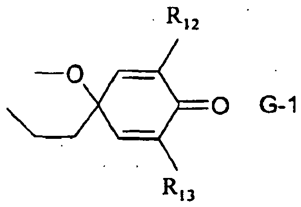 R₁₂ et R₁₃ représentent indépendamment l'un de l'autre l'hydrogène, un groupe alkyle, saturé ou non saturé, linéaire ou ramifié de 1 à 6 atomes de carbone, un groupe cycloalkyle de 3 à 8 atomes de carbone, un groupe O-alkyle, saturé ou non saturé, linéaire ou ramifié de 1 à 6 atomes de carbone, un groupe O-cycloalkyle de 3 à 8 atomes de carbone, un atome d'halogène tel que le chlore, le fluor, le brome et l'iode ;
   R₉ représente :
   - un groupe OH,
   - un groupe alkyle saturé ou non saturé, linéaire ou ramifié de 1 à 20 atomes de carbone, un groupe cycloalkyle de 3 à 8 atomes de carbone,
   - un groupe S(O)-R" dans lequel R" est un alkyle saturé ou non saturé, linéaire ou ramifié de 1 à 20 atomes de carbone ou un groupe cycloalkyle de 3 à 8 atomes de carbone,
   - un groupe O-alkyle, un groupe O-CO-alkyle, un groupe O-COCH₂CO-alkyle, ledit résidu alkyle étant saturé ou non saturé, linéaire ou ramifié de 1 à 20 atomes de carbone, un groupe O-cycloalkyle de 3 à 8 atomes de carbone, un groupe O-CH₂-CH₂-N(CH₃₎₂ ; un groupe O-CH₂-CCH,
   - un groupe 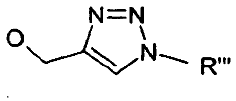 dans lequel R"' est un alkyle saturé ou non saturé, linéaire ou ramifié de 1 à 20 atomes de carbone,
   - un groupe N(R")(R"'), R" et R"' étant tels que définis ci-dessus,
   et quels que soient R₁ et R₂ :
- R₃, R₄, R₅, R₆ représentent indépendamment l'un de l'autre l'hydrogène, un groupe alkyle, saturé ou non saturé, linéaire ou ramifié de 1 à 6 atomes de carbone, un groupe cycloalkyle de 3 à 8 atomes de carbone, un groupe O-alkyle, saturé ou non saturé, linéaire ou ramifié de 1 à 6 atomes de carbone, un groupe O-cycloalkyle de 3 à 8 atomes de carbone, un atome d'halogène tel que le chlore, le fluor, le brome et l'iode;
- R₇ et R₈ représentent indépendamment l'un de l'autre l'hydrogène, un groupe alkyle, saturé ou non saturé, linéaire ou ramifié de 1 à 6 atomes de carbone, un groupe cycloalkyle de 3 à 8 atomes de carbone, un groupe O-alkyle, saturé ou non saturé, linéaire ou ramifié, de 1 à 6 atomes de carbone, un groupe O-cycloalkyle de 3 à 8 atomes de carbone, un groupe NH-alkyle saturé ou non saturé, linéaire ou ramifié de 1 à 6 atomes de carbone, un groupe NH-cycloalkyle de 3 à 8 atomes de carbone, un groupe NHCO-R, dans lequel R peut être un groupe alkyle, saturé ou non saturé, linéaire ou ramifié de 1 à 6 atomes de carbone, un groupe cycloalkyle de 3 à 8 atomes de carbone,
- R₁₀ et R₁₁ représentent indépendamment l'un de l'autre l'hydrogène, un groupe hydroxyle, un groupe O-alkyle, le résidu alkyle étant saturé ou non saturé, linéaire ou ramifié de 1 à 8 atomes de carbone, un groupe O-cycloalkyle de 3 à 8 atomes de carbone, une amine tertiaire.

Dans un mode de réalisation préféré, le composé polyspirannique défini ci-dessus est de formule générale (II) suivante : 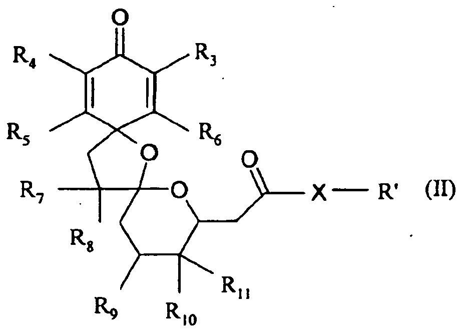 dans laquelle R₃ à R₈, R₁₀ à R₁₁, X et R'sont tels que définis ci-dessus et, R₉ représente un groupe OH, un groupe O-allyle, un groupe O-benzyle, un groupe O-CO-alkyle, ledit alkyle étant saturé ou non saturé, linéaire ou ramifié de 1 à 20 atomes de carbone, ou un groupe O-CO-cycloalkyle comportant 3 à 8 atomes de carbone.

Selon un autre mode de réalisation, le composé polyspirannique défini ci-dessus est de formule générale (III) suivante : 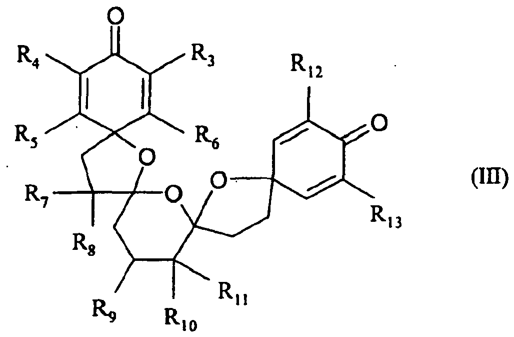 dans laquelle R₃ à R₈ et R₁₀ à R₁₃ sont tels que définis ci-dessus lorsque R₁ et R₂ forment ensemble un groupe G-1, et
R₉ représente :
- un groupe OH,
- un groupe alkyle saturé ou non saturé, linéaire ou ramifié de 1 à 20 atomes de carbone, un groupe cycloalkyle de 3 à 8 atomes de carbone,
- un groupe S(O)-R" dans lequel R" est un alkyle saturé ou non saturé, linéaire ou ramifié de 1 à 20 atomes de carbone ou un groupe cycloalkyle de 3 à 8 atomes de carbone,
- un groupe O-alkyle, un groupe O-CO-alkyle, un groupe O-COCH₂CO-alkyle, ledit résidu alkyle étant saturé ou non saturé, linéaire ou ramifié de 1 à 20 atomes de carbone, un groupe O-cycloalkyle de 3 à 8 atomes de carbone, un groupe O-CH₂-CH₂-N(CH₃)₂; un groupe O-CH₂-CCH,
- un groupe 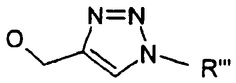 dans lequel R"' est un alkyle saturé ou non saturé, linéaire ou ramifié de 1 à 20 atomes de carbone,
un groupe N(R")(R"'), R" et R"' étant tels que définis ci-dessus,

Selon un mode de réalisation préféré, les substituants R₃ à R₈ et R₁₀ à R₁₁ du composé polyspirannique de formule générale (II), défini ci-dessus, représentent l'hydrogène, le substituant R₉ représente OH, X=O et le substituant R' est un alkyle saturé, linéaire à 3 atomes de carbone, à 10 atomes de carbone ou à 18 atomes de carbone.
- Le composé à 3 atomes de carbone peut correspondre au mélange des diastéréoisomères (2*S*,4*R*,6*R*) et (2*R*,4*S*,6*S*) 10a et (2*S*,4*R*,6*S*)- et (2*R*,4*S*,6*R*) 11a ou au mélange racémique 10a ou 11a, ou à l'un des énantiomères (2*S*,4*R*,6*R*) ou (2*R*,4*S*,6*S*) ou (2*S*,4*R*,6*S*) ou encore (2*R*,4*S*,6*R*).
   Les composés **10a** et **11a** sont de formule suivante : 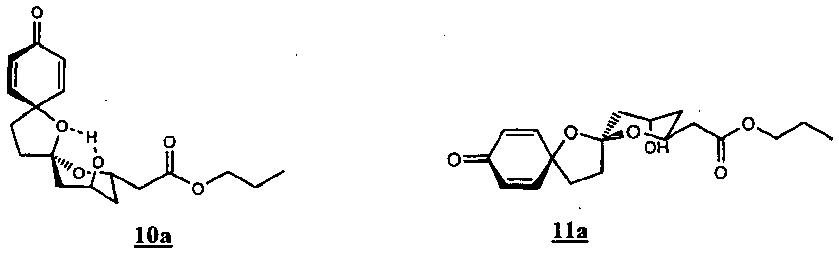
- Le composé à 10 atomes de carbone correspond au mélange des diastéréoisomères (2*S*,4*R*,6*R*) et (2*R*,4*S*,6*S*) 10b et (2*S*,4*R*,6*S*)- et (2*R*,4*S*,6*R*) 11b ou au mélange racémique 10b ou 11b, ou à l'un des énantiomères (2*S*,4*R*,6*R*) ou (2*R*,4*S*,6*S*) ou (2*S*,4*R*,6*S*) ou encore (2*R*,4*S*,6*R*).
   Les composés 10b et 11b sont de formule suivante : 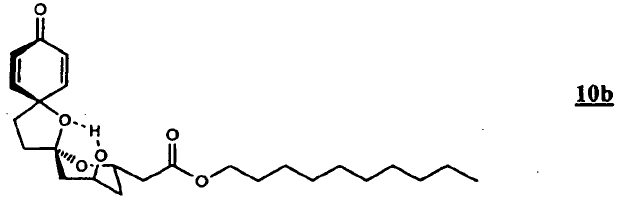 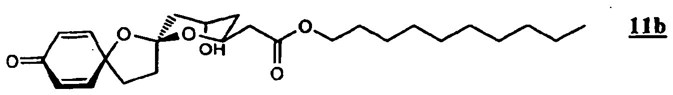
- Le composé à 18 atomes de carbones correspond au mélange des diastéréoisomères (2*S*,4*R*,6*R*) et (2*R*,4*S*,6*S*) 10c et (2*S*,4*R*,6*S*)- et (2*R*,4*S*,6*R*) 11c ou au mélange racémique 10c ou 11c, ou à l'un, des énantiomères (2*S*,4*R*,6*R*) ou (2*R*,4*S*,6*S*) ou (2*S*,4*R*,6*S*) ou encore (2*R*,4*S*,6*R*).
   Les composés **10c** et **11c** sont de formule suivante:

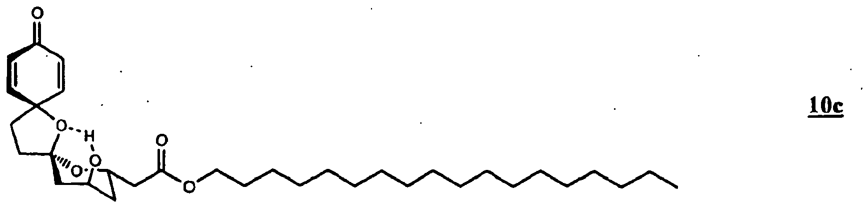

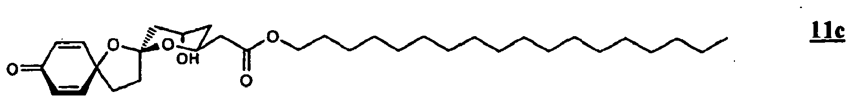

Selon un mode de réalisation préféré, les substituants R₃ à R₈ et R₁₀ à R₁₃ du composé polyspirannique de formule générale (III), défini ci-dessus, représentent l'hydrogène et le substituant R₉ est un groupe OH, O-CO-alkyle, dans lequel l'alkyle est linéaire et possède 7 atomes de carbone, 14 atomes de carbone ou 16 atomes de carbone ou un groupe alkyle dans lequel l'akyle est un alkyle linéaire 2-oxo et possède 15 atomes de carbone.
- Le composé dans lequel R₉ est un groupe OH correspond au mélange des diastéréoisomères **4a** et **4b**: 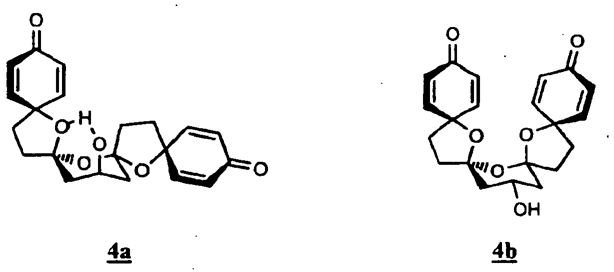
- Le composé dans lequel R₉ est un groupe O-CO-alkyle, à 7 atomes de carbone, correspond au mélange des diastéréoisomères **16a** et **16b**: 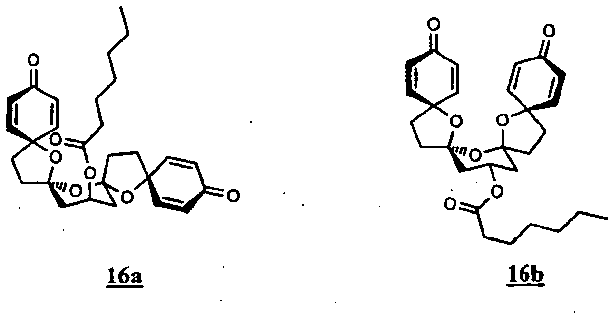
- Le composé dans lequel R₉ est un groupe O-CO-alkyle, à 14 atomes de carbone, correspond au mélange des diastéréoisomères **16c** et **16d**: 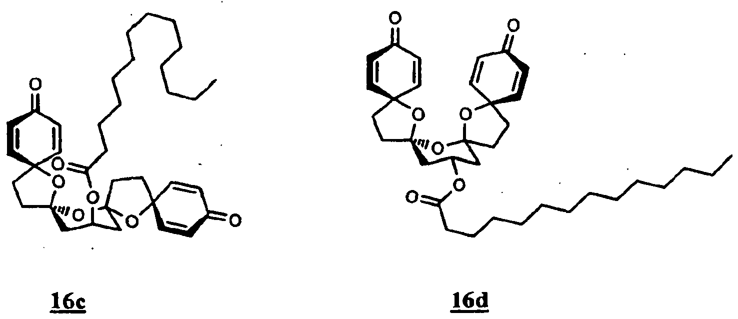
- Le composé dans lequel R₉ est un groupe O-CO-alkyle, à 16 atomes de carbone, correspond au mélange racémique 16e : 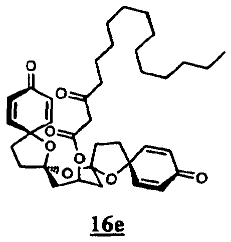
- Le composé dans lequel R₉ est un groupe alkyle, à 15 atomes de carbone, correspond au mélange des diastéréoisomères **23c** et **23d**:

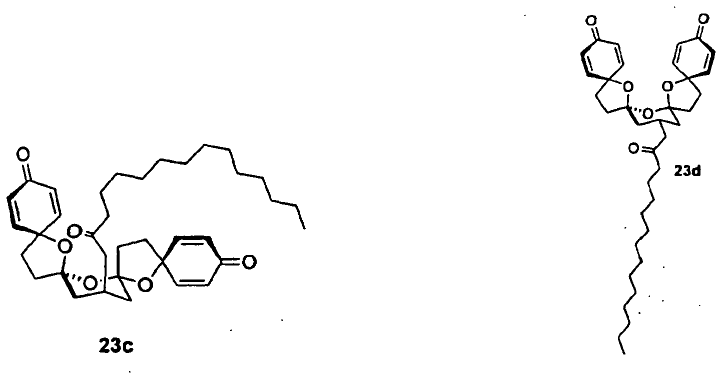

Selon encore un mode de réalisation préféré, le composé polyspirannique de formule générale (III), défini ci-dessus, est constitué d'un mélange d'énantiomères (8*R*, 10*S*, 21*S*) et (8*S*, 10*R*, 21*R*).

Le mélange d'énantiomères (8*R*, 10*S*, 21*S*) et (8*S*, 10*R*, 21*R*) correspond aux composés **4a**, ou **16a**, ou **16c**, ou **16e** ou **23c** ci-dessus représentés.

Dans un autre mode de réalisation plus préféré, le composé polyspirannique de formule générale (III), défini ci-dessus, est constitué du composé *méso* (8*R*, 10*R*, 21*S*), en mélange avec l'un des énantiomères (8*R*, 10*S*, 21*S*) ou (8*S*, 10*R*, 21*R*).

Dans un autre mode de réalisation encore plus préféré, le composé polyspirannique de formule générale (III), défini ci-dessus, est constitué du composé *méso* (8*R*, 10*R*, 21*S*), ou de l'énantiomère (8*R*, 10*S*, 21*S*) ou encore de l'énantiomère (8*S*, 10*R*, 21*R*).

Le composé *méso* (8*R,* 10*R*, 21*S*) correspond aux composés **4b** ou **16b**, ou **16d** ou **23d** ci-dessus représentés.

Selon un autre aspect, l'invention concerne une composition pharmaceutique comprenant comme substance active au moins un composé polyspirannique de formule générale (I), en association avec un véhicule pharmaceutiquement acceptable.

Dans un mode de réalisation préféré, ladite composition pharmaceutique se présente sous forme administrable par voie orale à raison de 0,1 mg/kg/j à 100 mg/kg/j de substance active, préférentiellement de 0,5 mg/kg/j à 10 mg/kg/j et plus préférentiellement de 1 mg/kg/j à 5 mg/kg/j.

Selon encore un autre aspect, l'invention concerne un procédé de préparation des composés de formule (I) :
comprenant une étape d'oxydation phénolique avec un oxydant tel que le PIFA ou le PIDA, et notamment le PIFA, d'un composé de formule (I-Z) suivante : 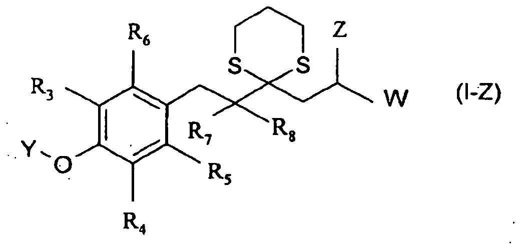 dans laquelle :
   - soit W représente un groupe de formule (II-W) suivante: 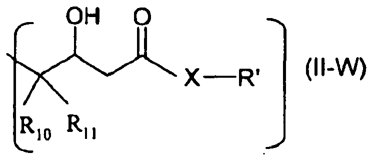 et X = O ou N,
      Y représente l'hydrogène,
      et Z représente :
      - un groupe OH,
      R₃ à R₈, R₁₀, R₁₁, X, R' et R"' étant tels que définis ci-dessus,
      pour obtenir un composé de formule générale (II) dans laquelle X = O ou N et R₉ représente un groupe OH,
      et comprenant ensuite optionnellement une étape d'acylation ou d'alkylation pour obtenir un composé de formule générale (II) dans lequel R₉ est un groupe O-CO-alkyle, un groupe O-allyle, un groupe O-benzyle, un groupe O-COCH₂CO-alkyle, ledit résidu alkyle étant saturé ou non saturé, linéaire ou ramifié de 1 à 20 atomes de carbone, ou un groupe O-CO-cycloalkyle comportant 3 à 8 atomes de carbone,
   - soit W représente un groupe de formule (II'-W) suivante : 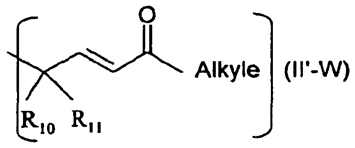 ledit alkyle étant saturé ou non saturé, linéaire ou ramifié de 1 à 17 atomes de carbone ou un cycloalkyle de 3 à 8 atomes de carbone, et X = C ;
      Y représente un groupe (Pr₃)Si-,
      et Z représente un groupe (Pr₃)Si-O-,
      pour obtenir un composé de formule générale (II) dans laquelle X = C et R₉ représente un groupe OH,
      et comprenant ensuite optionnellement une étape d'acylation ou d'alkylation pour obtenir un composé de formule générale (II) dans lequel R₉ est un groupe O-CO-alkyle, un groupe O-allyle, un groupe O-benzyle, un groupe O-COCH₂CO-alkyle, ledit résidu alkyle étant saturé ou non saturé, linéaire ou ramifié de 1 à 20 atomes de carbone, ou un groupe O-CO-cycloalkyle comportant 3 à 8 atomes de carbone,
   - soit W représente un groupe III-W : 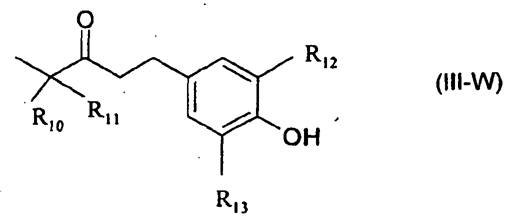 et Z représente :
      - un groupe OH,
      - un groupe alkyle saturé ou non saturé, linéaire ou ramifié de 1 à 20 atomes de carbone, un groupe cycloalkyle de 3 à 8 atomes de carbone,
      - un groupe S-R" dans lequel R" est un alkyle saturé ou non saturé, linéaire ou ramifié de 1 à 20 atomes de carbone ou un groupe cycloalkyle de 3 à 8 atomes de carbone,
      - un groupe O-alkyle, l'alkyle étant saturé ou non saturé, linéaire ou ramifié de 1 à 20 atomes de carbone, un groupe O-cycloalkyle de 3 à 8 atomes de carbone, un groupe O-CH₂-CH₂-N(CH₃)₂ ; un groupe O-CH₂-CCH,
      - un groupe N(R")(R"'), R" et R"' étant tels que définis ci-dessus,
      R₃ à R₈ et R₁₀ à R₁₃ étant tels que définis ci-dessus,
      pour obtenir un composé de formule générale (III) dans lequel R₉ représente :
      - un groupe OH,
      - un groupe alkyle saturé ou non saturé, linéaire ou ramifié de 1 à 20 atomes de carbone, un groupe cycloalkyle de 3 à 8 atomes de carbone,
      - un groupe S(O)-R" dans lequel R" est un alkyle saturé ou non saturé, linéaire ou ramifié de 1 à 20 atomes de carbone ou un groupe cycloalkyle de 3 à 8 atomes de carbone,
      - un groupe O-alkyle, l'alkyle étant saturé ou non saturé, linéaire ou ramifié de 1 à 20 atomes de carbone, un groupe O-cycloalkyle de 3 à 8 atomes de carbone, un groupe O-CH₂-CH₂-N(CH₃)₂ ; un groupe O-CH₂-CCH,
      - un groupe N(R")(R"'), R" et R"' étant tels que définis ci-dessus,
      et, lorsque Z est un groupe OH (composé (III-1), comprenant optionnellement une étape d'acylation ou d'alkylation pour obtenir un composé de formule générale (III) dans lequel R₉ est un groupe O-alkyle, un groupe O-cycloalkyle, un groupe O-CO-alkyle, un groupe O-COCH₂CO-alkyle, un groupe O-CH₂-CH₂-N(CH₃)₂ ; un groupe O-CH₂-CCH,
      et/ou, lorsque Z représente un groupe O-CH₂-CCH, comprenant optionnellement une réaction d'addition avec N₃-R"' pour obtenir un composé de formule générale (III) dans lequel R₉ est un groupe 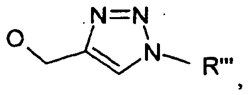 R'" étant tel que défini ci-dessus.

Le PIFA (phényliodonium bis(trifluoroacétate) et le PIDA (phényliodonium diacétate) sont tous deux des réactifs commerciaux.

La réaction est effectuée à température ambiante dans un mélange de solvant tel que l'acétone et l'eau.

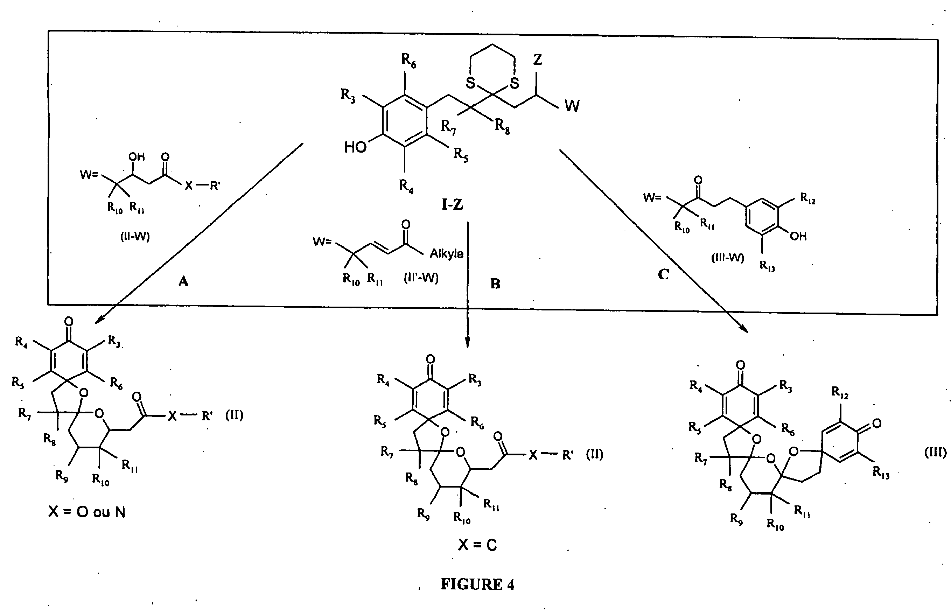

La figure 4 résume les différentes possibilités d'obtention des composés (II), dans lesquels X = O, N ou X = C, ou des composés (III).

L'intermédiaire (I-Z) permet ainsi d'obtenir tous les composés de l'invention, en fonction de Z et de W.

Lorsque le résidu R₉ des composés de formule (II) est un groupe OH, il est possible d'alkyler ou d'acyler lesdits composés dans des conditions faiblement basiques, telles que la diméthylaminopyridine (DMAP) par réaction avec un anhydride, un chlorure d'acide dans le cas de l'acylation ou un halogénure d'allyle ou de benzyle dans le cas de l'alkylation, dans un solvant notamment le dichlorométhane, pour obtenir des composés de formule (II) dans laquelle R₉ est un groupe O-allyle, un groupe O-benzyle, un groupe O-CO-alkyle, un groupe O-COCH₂CO-alkyle, ou un groupe O-CO-cycloalkyle.

Par conséquent, cesdits composés sont obtenus en deux étapes, par oxydation phénolique des composés de formule I-Z dans laquelle Z=OH, puis alkylation ou acylation des composés de formule (II) obtenu dans laquelle R₉ est un groupe OH.

Selon un mode de réalisation préféré, le procédé de préparation, ci-dessus défini, des composés de formule (II) dans lequel X = O, N comprend les étapes suivantes :
- Réaction d'un aldéhyde de formule (IV) suivante : 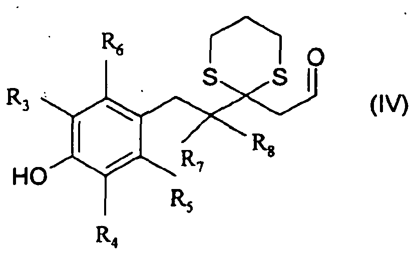 R₃ à R₈ étant tels que définis ci-dessus,
   avec un composé de formule (VII) suivante : 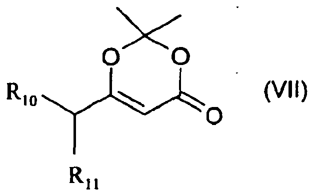 pour obtenir le composé de formule (V) suivante : 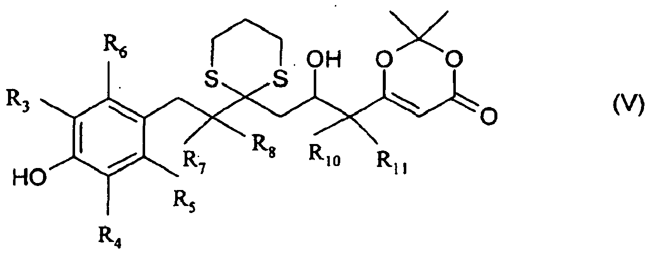 R₃ à R₈ et R₁₀ à R₁₁ étant tels que définis ci-dessus,
- réaction du composé (V) obtenu avec un composé R'-X-H , (X= O) ou (R')(R")-X-H (X= N), R' et R" étant tels que définis ci-dessus, puis réduction avec un réducteur, notamment NaBH₄ ou NaBH₄ et Et₃B, ou Me₄NBH(OAc)₃, pour obtenir un composé de formule (I-Z) dans lequel W représente le groupe (II-W);
- oxydation phénolique du composé (I-Z) obtenu avec un oxydant tel que le PIFA et le PIDA, notamment le PIFA, afin d'obtenir un composé II dans lequel R₉ est OH ;
- éventuellement, réaction d'acylation ou d'alkylation du composé II dans lequel R₉ est OH, pour obtenir un composé de formule générale II dans lequel R₉ représente un groupe O-allyle, un groupe O-benzyle, un groupe O-CO-alkyle, ou un groupe O-CO-cycloalkyle tels que définis ci-dessus.

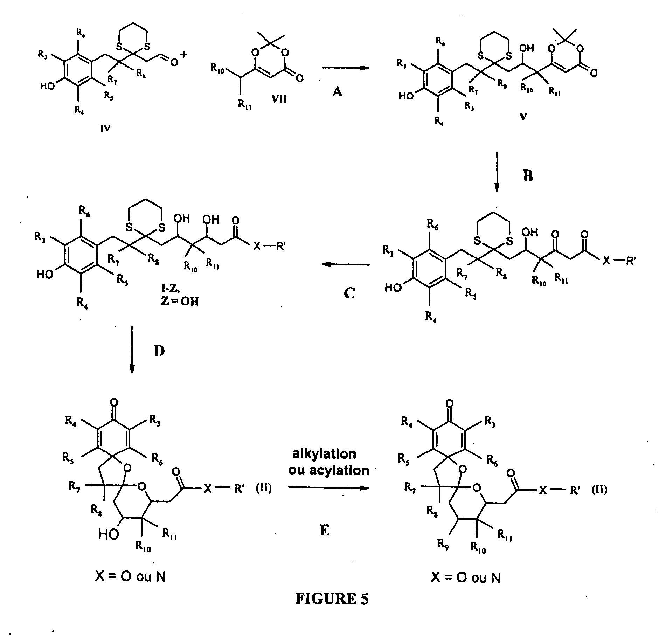

La figure 5 présente le procédé ci-dessus détaillé.

La réaction de l'aldéhyde de formule (IV) avec un composé de formule (VII) est effectuée dans les conditions basiques classiques au moyen d'une base telle que le LDA (diisopropylamidure de lithium) préparé par réaction du n-butyllithium avec la diisopropylamine, dans un solvant polaire tel que le THF à une température de -78°C.

L'ouverture du cycle 1,3-dioxin-4-one par un alcool ou une amine se fait dans des conditions classiques par chauffage au reflux d'un solvant tel que le toluène.

Le contrôle de la stéréochimie peut être effectué au niveau de l'étape de réduction en fonction du réducteur utilisé :
- l'emploi d'un réducteur tel que NaBH₄ et Et₃B conduit au composé (I-Z) dans lequel le diol obtenu est un mélange racémique de configuration *syn*,
- l'emploi d'un réducteur tel que Me₄NBH(OAc)₃ conduit au composé (I-Z) dans lequel le diol obtenu est un mélange racémique de configuration *anti*,

Selon un autre mode de réalisation, le procédé de préparation, ci-dessus défini, des composés de formule (II) dans lequel X = C comprend les étapes suivantes :
- Réaction d'un ester de formule (VIII) suivante : 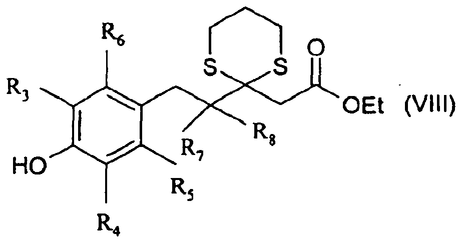 R₃ à R₈ étant tels que définis ci-dessus,
   avec un acétate de *tert*-butyle de formule 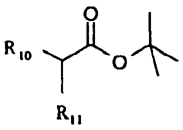 en présence d'une base telle que le LDA, pour obtenir le composé de formule (IX) suivante : 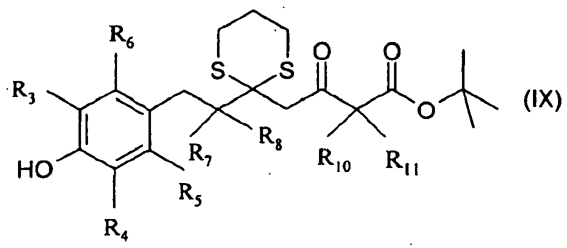 R₃ à R₈ étant tels que définis ci-dessus,
- réduction du composé (IX) obtenu puis réaction avec le chlorure de tripropylsilane pour conduire au composé de formule (X) suivante : 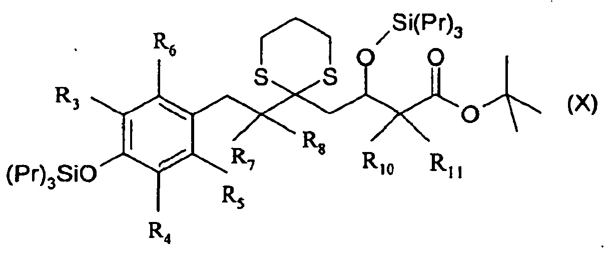
- réduction du composé (X) obtenu avec un réducteur tel que le DIBAL-H pour conduire au composé de formule (XI) suivante : 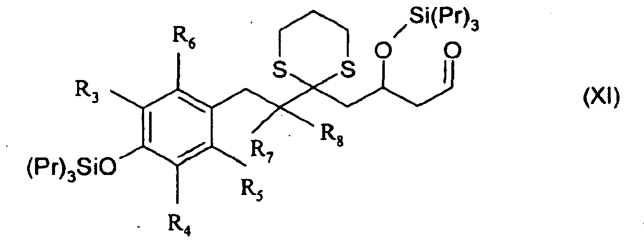
- réaction de Wittig sur le composé (XI) obtenu pour obtenir un composé de formule (I-Z) dans lequel W représente le groupe (II'-W) et Y = (Pr)₃Si-;
- oxydation phénolique du composé (I-Z) obtenu avec un oxydant tel que le PIFA et le PIDA, notamment le PIFA afin d'obtenir un composé (II) dans lequel R₉ est OH et X =C;
- éventuellement, réaction d'acylation ou d'alkylation du composé (II) dans lequel R₉ est OH et X=C, pour obtenir un composé de formule générale (II) dans lequel X=C et R₉ représente un groupe O-allyle, un groupe O-benzyle, un groupe O-CO-alkyle, ou un groupe O-CO-cycloalkyle tels que définis ci-dessus.

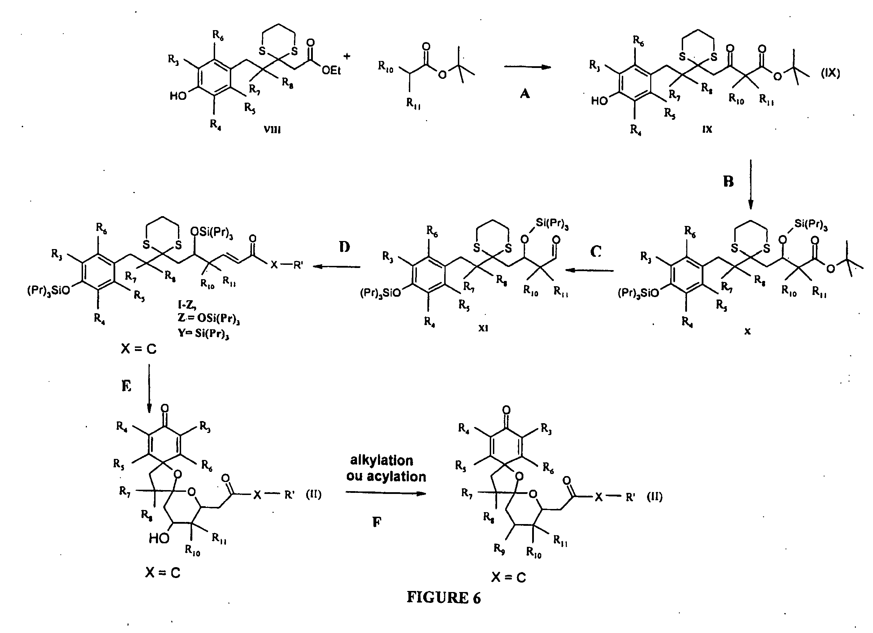

La figure 6 présente le procédé de préparation détaillé des composés de formule (II) dans laquelle X = C ;

La réaction de l'ester de formule (VIII) avec un acétate de *tert*-butyle est effectuée dans les conditions basiques classiques au moyen d'une base telle que le LDA (diisopropylamidure de lithium) préparé par réaction de *n*-butyllithium avec la diisopropylamine, dans un solvant polaire tel que le THF à une température de -78°C.

La réduction du composé (IX) obtenu est effectuée au moyen d'un réducteur tel que NaBH₄ dans un solvant protique polaire tel que le méthanol à 0°C. La fonction alcool ainsi que le phénol du composé (X) obtenu sont protégés par la suite par un groupe protecteur des fonctions alcool et phénol, notamment par réaction du chlorure de tri-n-propylsilane dans un solvant tel que le diméthylformamide.

La réduction de la fonction ester du composé (X) est effectuée par un réducteur tel que le DIBAL-H (hydrure de diisobutylaluminium) dans un solvant tel que le toluène à -78°C.

Les composés désirés de formule (I-Z) dans laquelle W représente le groupe (II'-W) et Y = (Pr)₃Si- sont obtenus par réaction de Wittig sur le composé (XI).

Les réactifs de Wittig (sels de triphénylphosphine) sont soit commerciaux, soit aisément disponibles par des techniques connues de l'homme du métier. La réaction de Wittig est effectuée dans un solvant tel que le dichlorométhane à température ambiante.

Selon un mode de réalisation préféré, le composé (I-Z) du procédé de préparation, ci-dessus défini, des composés de formule (III), est obtenu par addition d'un nucléophile par addition de type Michael sur un composé de formule (III-3) : 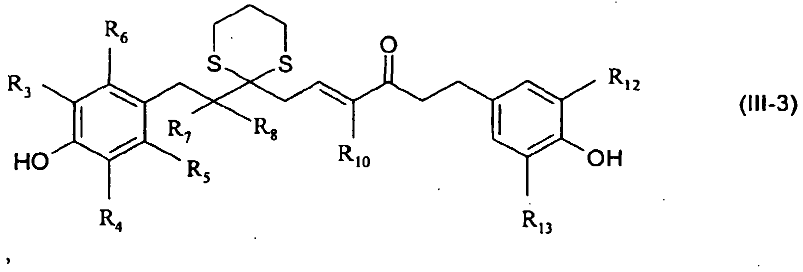 W représentant le groupe III-W et Z représentant :
- un groupe alkyle saturé ou non saturé, linéaire ou ramifié de 1 à 20 atomes de carbone, un groupe cycloalkyle de 3 à 8 atomes de carbone,
- un groupe S-R" dans lequel R" est un alkyle saturé ou non saturé, linéaire ou ramifié de 1 à 20 atomes de carbone ou un groupe cycloalkyle de 3 à 8 atomes de carbone,
- un groupe O-alkyle tel que défini ci-dessus, un groupe O-cycloalkyle de 3 à 8 atomes de carbone, un groupe O-CH₂-CH₂-N(CH₃)₂ ; un groupe O-CH₂-CCH,
- un groupe N(R")(R"'), R" et R"' étant tels que définis ci-dessus,
ledit composé (III-3) étant obtenu par déshydratation d'un composé (I-Z) dans lequel Z= OH de formule (III-1) suivante: 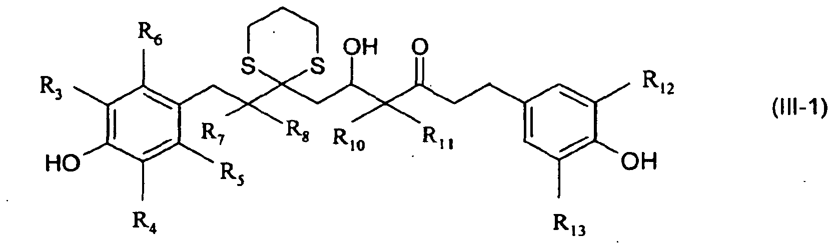 dans laquelle R₁₁ représente l'hydrogène.
R₃ à R₈, R₁₀ à R₁₃, R" et R"'étant tels que définis ci-dessus.

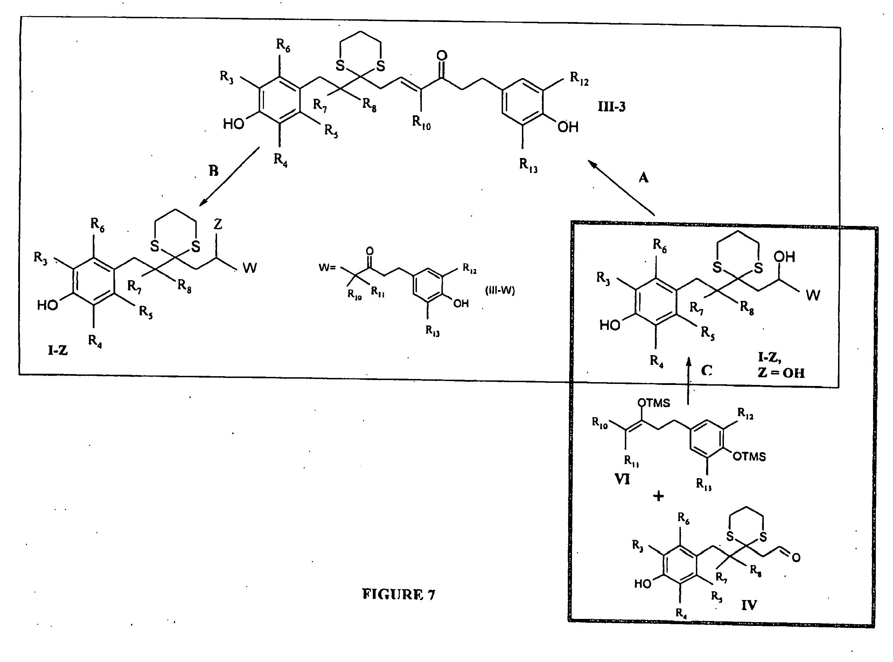

La figure 7 (cadre en traits noirs) présente le procédé de préparation détaillé des composés de formule (I-Z) (Z étant différent de OH).

Le composé (I-Z) dans lequel Z = OH est donc un intermédiaire clé permettant l'accès aux composés (III-3) ainsi que l'accès direct aux composés de formule III dans laquelle R₉ représente OH.

La réaction de déshydratation des composés de formule (III-1) est effectuée par chauffage au reflux dans un solvant tel que l'acétonitrile en présence d'un acide, notamment l'acide *para*-toluène sulfonique.

Les différentes additions de nucléophiles par addition de type Michael sont effectuées selon les techniques bien connues de l'homme du métier :
- addition des groupements alkyles via un organocuprate (Cluzeau J., Lubell W. D. Conformationally constrained dipeptide surrogates with aromatic side-chains : synthesis of 4-aryl indolizidin-9-one amino acids by conjugate addition to a common a,g-diaminoazelate enone intermediate J. Org. Chem. 2004, 69, 1504-1512),
- addition des thiols (Fehr C., Galindo J. Aldols by Michael addition: application of the retro-Michael addition to the slow release of enones Helv. Chim. Acta 2005, 88, 2005),
- addition des alcools (Stewart I. C., Bergman R. G., Toste F. D. Phosphinecatalyzed hydration and hydroalkoxylation of activated olefins: use of a strong nucleophile to generate a strong base J. Am. Chem. Soc. 2003, 125, 8696-8697),
- addition des amines (Bartoli G., Bartolacci M., Giuliani A., Marcantoni E., Massaccesi M., Torregiani E. Improved heteroatom nucleophilic addition of electron-poor alkenes promoted by CeCl3.7xH2O/NaI system supported on Alumina in solvent-free conditions J. Org. Chem. 2004, 70, 169-174).

Selon un autre mode de réalisation, le composé (I-Z), dans le cas où Z est un groupe OH du procédé de préparation, ci-dessus défini, d'un composé de formule (III), est obtenu par réaction d'un aldéhyde de formule (IV) suivante : 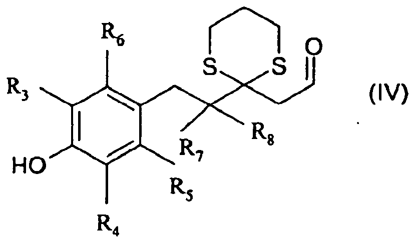 R₃ à R₈ étant tels que définis ci-dessus,
avec un composé de formule (VI) suivante : 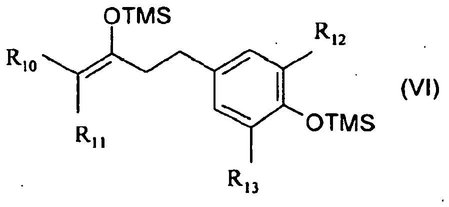 R₁₀ à R₁₃ étant tels que définis ci-dessus.

La figure 7 (cadre en traits gris épais) présente le procédé de préparation détaillé des composés de formule (I-Z) dans laquelle Z représente un groupe OH.

Les composés de formule (VI) sont préparés par les techniques conventionnelles de la chimie et notamment par réaction du LDA dans le THF à -78°C puis addition d'un silane tel que le chlorure de triméthylsilane.

Les composé (I-Z) désirés sont obtenus par réaction des composés de formule (IV) avec les composés de formule (VI) dans un solvant aprotique tel que le dichlorométhane en présence de BF₃.OEt₂ à -78°C. Après isolement des produits intermédiaires ainsi obtenus, la déprotection des triméthylsilylanes est effectuée par réaction de fluorure de tétrabutylammonium (TBAF) dans un solvant aprotique tel que le dichlorométhane.

Selon un mode de réalisation préféré, le procédé de préparation ci-dessus défini, des composés de formule (III) dans laquelle R₉ représente :
- un groupe OH,
   un groupe O-alkyle, un groupe O-CO-alkyle, un groupe O-COCH₂CO-alkyle, ledit alkyle étant saturé ou non saturé, linéaire ou ramifié de 1 à 20 atomes de carbone, un groupe O-CO-cycloalkyle de 3 à 8 atomes de carbone,
   comprend les étapes suivantes :
   - réaction d'un aldéhyde de formule (IV) suivante : 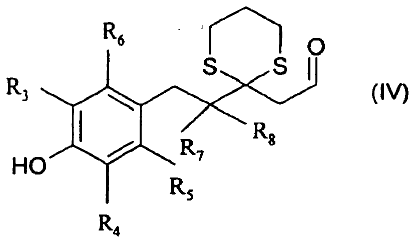 R₃ à R₈ étant tels que définis ci-dessus,
      avec un composé de formule (VI) suivante : 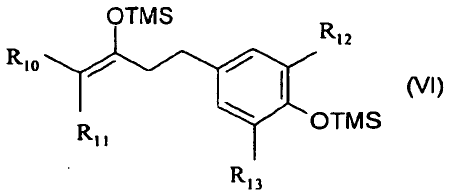 R₁₀ à R₁₃ étant tels que définis ci-dessus,
      pour obtenir, après traitement par le TBAF, un composé (I-Z) dans lequel Z=OH de formule (III-1) suivante: 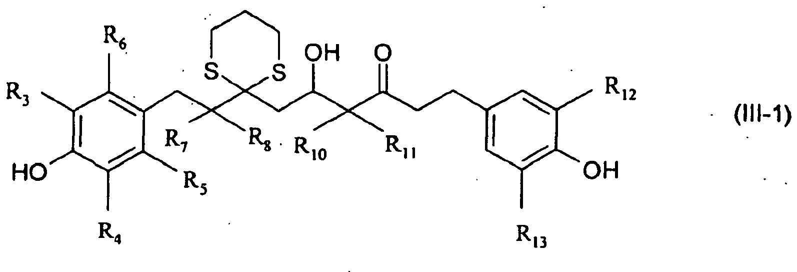
   - oxydation phénolique avec un oxydant tel que le PIFA et le PIDA, notamment le PIFA, du composé (III-1) obtenu, afin d'obtenir un composé (III) dans lequel R₉ est OH,
   - éventuellement, réaction d'acylation ou d'alkylation du composé (III) obtenu, dans lequel R₉ est un groupe OH, pour obtenir un composé de formule (III) désiré dans lequel R₉ est un groupe O-allyle, un groupe O-benzyle, un groupe O-CO-alkyle, un groupe O-COCH₂CO-alkyle, ou un groupe O-CO-cycloalkyle.

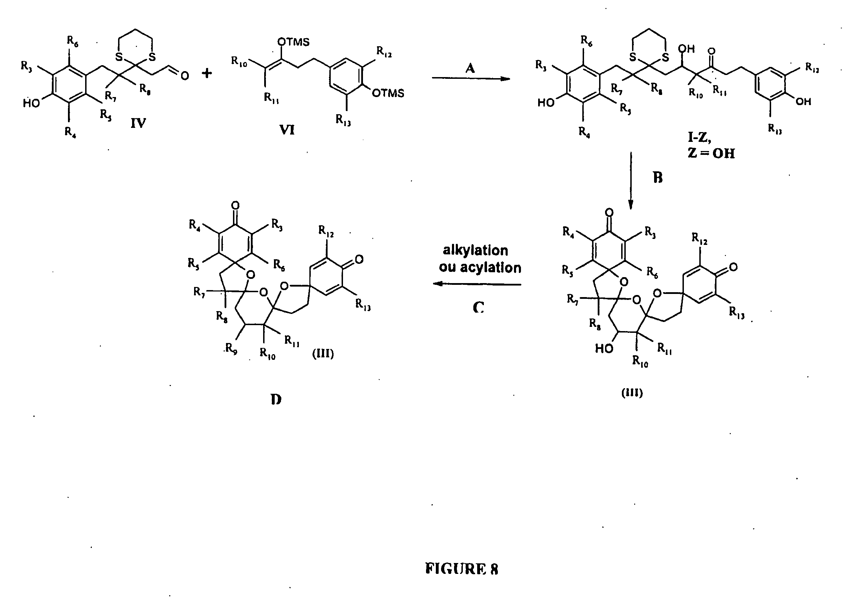

La figure 8 présente le procédé de préparation détaillé des composés de formule (III) dans laquelle R₉ est un groupe OH, ou R₉ est un groupe O-allyle, un groupe O-benzyle, un groupe O-CO-alkyle, un groupe O-COCH₂CO-alkyle, ou un groupe O-CO-cycloalkyle.

Selon un mode de réalisation encore plus préféré, le procédé de préparation, ci-dessus défini, des composés de formule (III) où R₉ est un groupe alkyle tel que défini ci-dessus, comprend les étapes suivantes :
- addition d'un nucléophile CuXRa par addition de type Michael, Ra représentant un groupe alkyle tel que défini ci-dessus, sur le composé (III-3) ci-dessus obtenu pour conduire au composé (I-Z) dans lequel Z=Ra de formule (III-4) suivante : 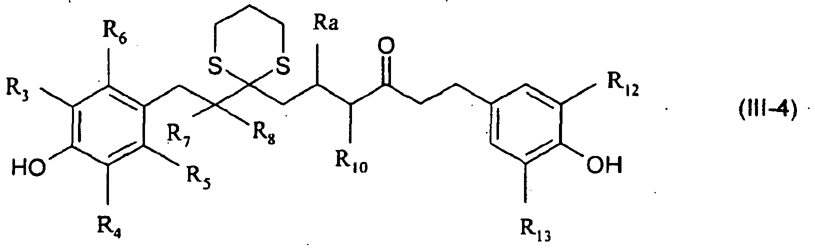 dans laquelle R₃ à R₈, R₁₀, R₁₂ et R₁₃ sont tels que définis ci-dessus et Ra représente un groupe alkyle tel que défini ci-dessus.
- oxydation phénolique avec un oxydant tel que le PIFA et le PIDA, notamment le PIFA, du composé (III-4) obtenu, afin d'obtenir un composé (III) dans lequel R₉ est un groupe alkyle tel que défini ci-dessus.

Selon un mode de réalisation encore plus préféré, le procédé de préparation, ci-dessus défini, des composés de formule (III) dans lesquels R₉ est un groupe N(R")(R"'), R" et R"' étant tels que définis ci-dessus, comprend les étapes suivantes :
- addition d'un nucléophile HN(R")(R"') par addition de type hétéro-Michael sur le composé (III-3) ci-dessus obtenu pour conduire au composé (I-Z) dans lequel Z=N(R")(R"') de formule (III-5) suivante : 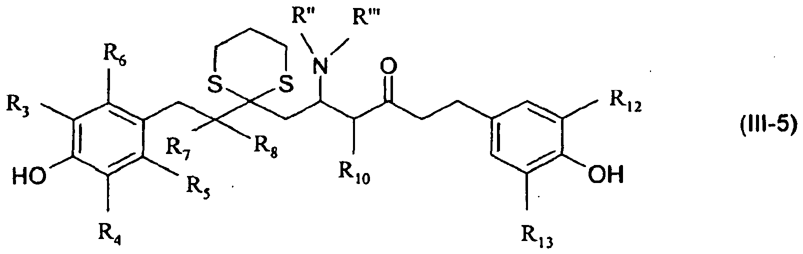 dans laquelle R₃ à R₈, R₁₀, R₁₂ et R₁₃ sont tels que définis ci-dessus,
- oxydation phénolique avec un oxydant tel que le PIFA et le PIDA, notamment le PIFA, du composé (III-5) obtenu, afin d'obtenir un composé (III) dans lequel R₉ est un groupe N(R")(R"') tel que défini ci-dessus.

Selon un autre mode de réalisation, le procédé de préparation, ci-dessus défini, des composés de formule (III) dans lesquels R₉ est un groupe S(O)-R", R" étant tel que défini ci-dessus, comprend les étapes suivantes :
- addition d'un nucléophile R"SH par addition de type hétéro-Michael, R" étant tel que défini ci-dessus, sur le composé (III-3) ci-dessus obtenu pour conduire au composé (I-Z) dans lequel Z= SR" de formule (III-6) suivante : 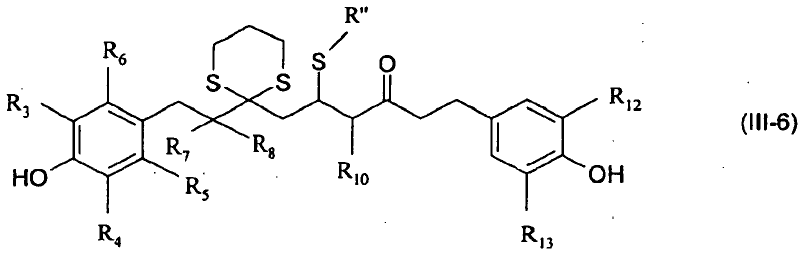 dans laquelle R₃ à R₈, R₁₀, R₁₂, R₁₃ et R"sont tels que définis ci-dessus.
- oxydation phénolique avec un oxydant tel que le PIFA et le PIDA, notamment le PIFA, du composé (III-6) obtenu, afin d'obtenir un composé (III) dans lequel R₉ est un groupe -S(O)R", R" étant tel que défini ci-dessus.

Selon un mode de réalisation préféré, le procédé de préparation, ci-dessus défini, des composés de formule (III) dans lesquels R₉ est un groupe O-alkyle tel que défini ci-dessus, un groupe O-CH₂-CH₂-N(CH₃)₂ ; un groupe O-CH₂-CCH,
un groupe 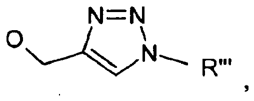 R"' étant tel que défini ci-dessus comprend les étapes suivantes :
- addition d'un nucléophile ROH par addition de type hétéro-Michael, R étant un alkyle tel que défini ci-dessus, un groupe -CH₂-CH₂-N(CH₃)₂ ; un groupe -CH₂-CCH, sur le composé (III-3) ci-dessus obtenu pour conduire au composé (I-Z) dans lequel Z=OR de formule (III-2) suivante: 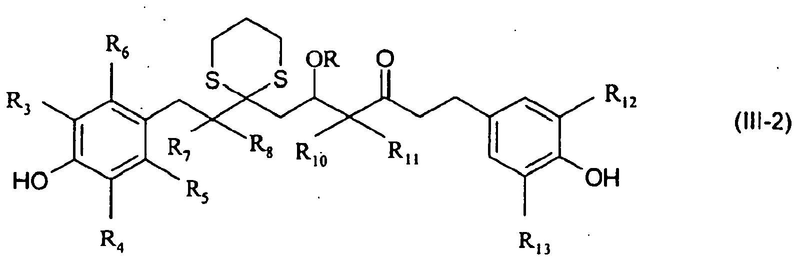 dans laquelle R₃ à R₈ et R₁₀, R₁₂ et R₁₃ sont tels que définis ci-dessus R₁₁ représente l'hydrogène,
- oxydation phénolique avec un oxydant tel que le PIFA et le PIDA, notamment le PIFA, du composé (III-2) obtenu, afin d'obtenir un composé (III) dans lequel R₉ est un groupe O-alkyle tel que défini ci-dessus, un groupe O-CH₂-CH₂-N(CH₃)₂ ; un groupe O-CH₂-CCH,
- éventuellement, réaction d'addition du composé (III) obtenu ci-dessus dans lequel R₉ est un groupe O-CH₂-CCH, avec N₃-R"' pour obtenir le composé de formule (III) désiré dans lequel R₉ est un groupe 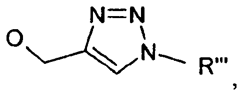 R"' étant tel que défini ci-dessus.

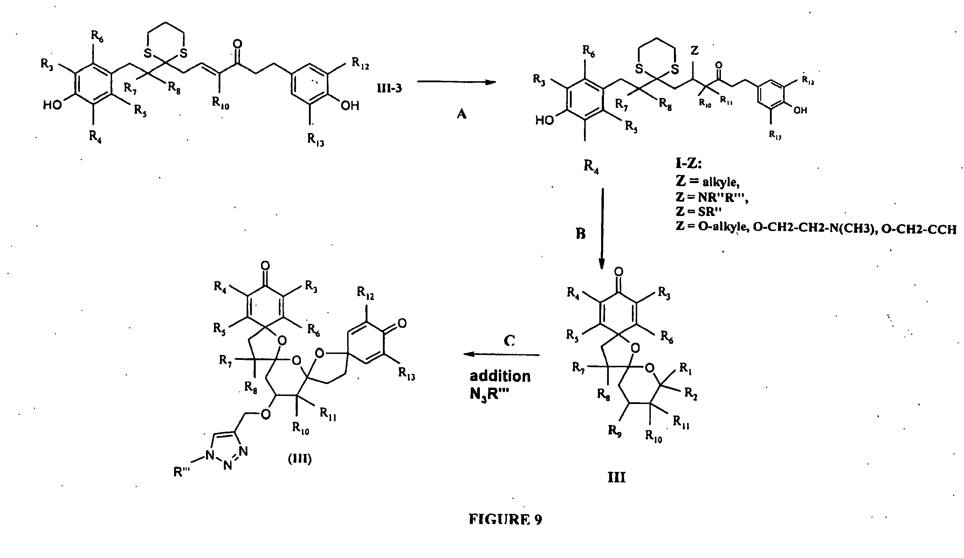

La figure 9 présente le procédé de préparation détaillé des composés de formule (III) dans laquelle R₉ est un groupe alkyle, un groupe NR"R"', un groupe S(O)R", un groupe O-alkyle, un groupe O-CH₂-CH₂-N(CH₃)₂ ; un groupe O-CH₂-CCH ou un groupe 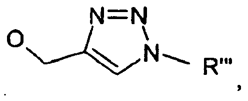 R"' étant tel que défini ci-dessus.

Les composés de formule générale (III) dans lequel R₉ est un groupe 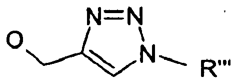 sont obtenus par addition d'un azide (N₃-R"') en présence de sulfate de cuivre et d'ascorbate de sodium. (Kolb H. C., Finn M. G., Sharpless K. B. Click chemistry: diverse chemical function from a few good reactions Angew Chem. Int. Ed. 2001, 40, 2004-2021; Bock V. D., Hiemstra H., van Maarseveen J. H. Cu1-catalysed alkyne-azide "Click" cycloaddition from a mechanistic and synthetic perspective Eur. J. Org. Chem. 2006, 51-68).

### DESCRIPTION DES FIGURES

La figure 1A à 1I représente des molécules antipaludiques utilisées auparavant :
1A : quinine
1B : bleu de méthylène
1C : pamaquine
1D : mépacrine
1E : chloroquine
1F : proguanil
1 G : pyriméthamine
1H : méfloquine
1I : artémisinine

La figure 2A à 2M représente des molécules utilisées ou en cours d'étude pour le traitement de la toxoplasmose :
2A : R=H, sulfadiazine ; R = Me, sulfamérazine
2B : PABA
2C : acide folinique
2D : R=H, spiramicine I ; R=COMe, spiramicine II, R=H, spiramicine III
2E : triméthropine
2F : sulfaméthoxazole
2G : clarithromycine
2H : azithromycine
2I : 22,26-azastérol (AZA)
2J : 24,25 (R,S)-épiminolanostérol (EIL)
2K : PHNQ-6
2L : R=H, COPh, COPh(2-Br), 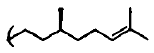
2M : monensine

La figure 3 représente la structure des aculéatines A, B, C et D.

La figure 4 représente les différentes possibilités d'obtention des composés de l'invention :
A : obtention des composés (II), dans lesquels X = O, N par oxydation phénolique,
B : obtention des composés (II) dans lesquels X = C, par oxydation phénolique,
C : obtention des composés (III), par oxydation phénolique.

La figure 5 représente le procédé de synthèse des composés de formule (II) dans lesquels X = O ou N :
A : addition du composé (VII) sur le composé (IV) par traitement avec le LDA dans le THF à -78°C,
B et C : addition d'une amine ou d'un alcool sur le composé (V) par chauffage au reflux dans le toluène puis réduction de la fonction cétone du composé obtenu par NaBH₄, NaBH₄ et Et₃B, ou Me₄NBH(OAc)₃,
D : oxydation phénolique par un oxydant tel que le PIFA ou le PIDA, à température ambiante dans un mélange de solvant tel que l'acétone et l'eau.
E : optionnellement, réaction d'acylation dans le dichlorométhane par réaction avec un anhydride, un chlorure d'acide ou d'alkylation avec un chlorure d'allyle ou de benzyle dans un solvant, notamment le dichlorométhane.

La figure 6 représente le procédé de synthèse des composés de formule (II) dans lesquels X = C :
A : addition d'un acétate de *tert*-butyle sur le composé (VIII) par traitement avec le LDA dans le THF à -78°C,
B : réduction de la fonction cétone du composé (IX) par NaBH₄ dans le méthanol à 0°C puis protection de la fonction alcool et phénol par un groupe protecteur, notamment le chlorure de tripropylsilane.
C : réduction de la fonction ester par le DIBAL-H dans le toluène à -78°C,
D : réaction de Wittig du composé (XI) dans le dichlorométhane à température ambiante avec un sel de triphénylphosphine,
E : oxydation phénolique par un oxydant tel que le PIFA ou le PIDA, à température ambiante dans un mélange de solvant tel que l'acétone et l'eau.

La figure 7 représente :
- cadre en traits noirs : le procédé de préparation des composés de formule (I-Z) (Z étant différent de OH) à partir de l'intermédiaire clé (I-Z) dans lequel Z = OH :
   A : déshydratation du composé (I-Z) (Z = OH) par chauffage au reflux dans un solvant tel que l'acétonitrile en présence d'un acide, notamment l'acide *para*-toluène sulfonique,
   B : réaction d'addition d'un nucléophile (CuX-alkyle, HS-R", HO-alkyle, ou HN(R")(R"')) par addition de type Michael sur le composé de formule (III-3).
- cadre en traits gris épais : le procédé de préparation des composés de formule (I-Z) dans laquelle Z représente un groupe OH :
   C : réaction d'addition d'un composé de formule (VI) sur un composé de formule (IV) dans un solvant aprotique tel que le dichlorométhane en présence de BF₃.OEt₂ à -78°C. Après isolement des produits intermédiaires ainsi obtenus, la déprotection des triméthylsilylanes est effectuée par réaction de fluorure de tétrabutylammonium (TBAF) dans un solvant aprotique tel que le dichlorométhane.

La figure 8 présente le procédé de préparation des composés de formule (III) dans laquelle R₉ est un groupe OH, ou R₉ est un groupe O-allyle, un groupe O-benzyle, un groupe O-CO-alkyle, un groupe O-COCH₂CO-alkyle, ou R₉ est un groupe O-CO-cycloalkyle:
A : addition du composé (VI) sur l'aldéhyde (IV) dans le THF à -78°C,
B : oxydation phénolique par un oxydant tel que le PIFA ou le PIDA, à température ambiante dans un mélange de solvant tel que l'acétone et l'eau,
C : optionnellement, réaction d'acylation dans le dichlorométhane par réaction avec un anhydride, un chlorure d'acide ou d'alkylation avec un chlorure d'allyle ou de benzyle dans un solvant, notamment le dichlorométhane.

La figure 9 présente le procédé de préparation des composés de formule (III) dans laquelle R₉ est un groupe alkyle, un groupe NR"R"', un groupe S(O)R", un groupe O-alkyle, un groupe O-CH₂-CH₂-N(CH₃)₂ ; un groupe O-CH₂-CCH ou un groupe 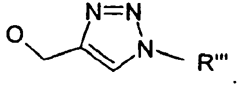
A: addition d'un nucléophile (nucléophile (CuX-alkyle, HS-R", HO-alkyle, ou HN(R")(R'").
B : oxydation phénolique par un oxydant tel que le PIFA ou le PIDA, à température ambiante dans un mélange de solvant tel que l'acétone et l'eau,
C : optionnellement, addition d'un azide (**N₃-R**"') en présence de sulfate de cuivre et d'ascorbate de sodium.

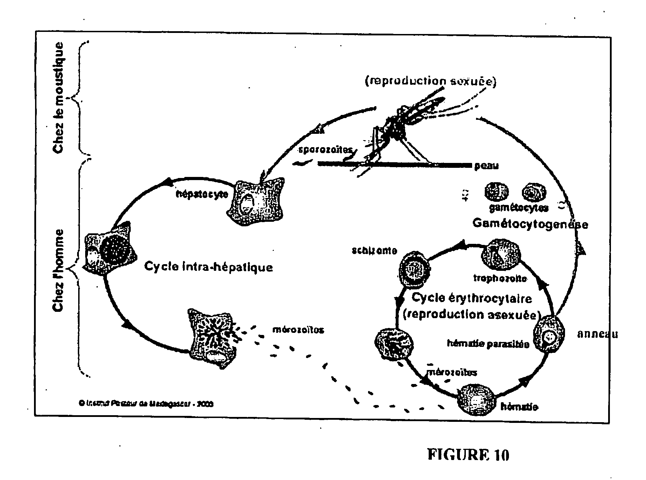

La figure 10 présente les différents cycles biologiques de *Plasmodium* Spp.

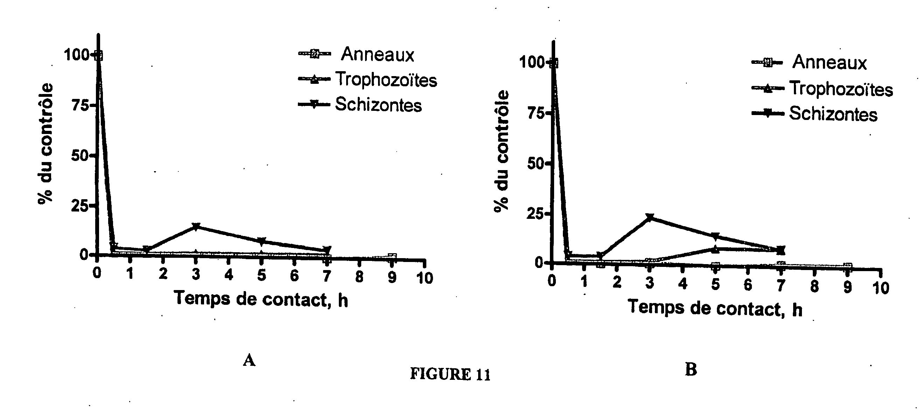

La figure 11 présente les résultats obtenus avec des composés de l'invention sur les différents stades sanguins (anneaux, trophozoïtes et schizontes) du développement du parasite:
A : composé **16c**
B : composé **16d**

### PARTIE EXPERIMENTALE :

### I) SYNTHESE DES COMPOSES

### Acide 3-(4-henzyloxyphényl)propionique (2)

A une solution aqueuse de soude 1N (301 mL) est ajoutée, à température ambiante, une solution d'acide 3-(4-hydroxyphényl)propionique (20,0 g, 0,12 mol) et de bromure de benzyle (21,5 mL, 0,18 mol) dans 155 mL de THF. Le catalyseur de transfert de phase, Bu₄NHSO₄ (155 mg, 0,45 mmol), est enfin ajouté. Le milieu réactionnel est agité une nuit avant d'être chauffé 1 h à 80°C. Après refroidissement, 150 mL d'une solution aqueuse 1N de HCl est ajouté lentement. Après extraction au CH₂Cl₂, les phases organiques sont séchées sur MgSO₄, filtrées et concentrées sous vide. La purification est effectuée sur gel de silice (CH₂Cl₂ jusque CH₂Cl₂/MeOH 95:5) pour donner le composé **2** (30,4 g, 0,12 mol, 99 %) sous forme d'un solide blanc. 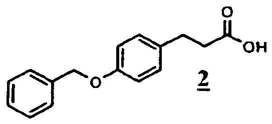 C₁₆H₁₆O3
M = 256,3 g.mol⁻¹
**RMN ¹H (400 MHz, CDCl₃) :** δ = 2,63 (t, *J* = 7,7 Hz, 2H) ; 2,88 (t, *J* = 7,7 Hz, 2H) ; 5,02 (s, 2H) ; 6,90 (d, *J* = 8,6 Hz, 2H) ; 7,11 (d, *J* = 8,6 Hz, 2H) ; 7,28-7,44 (m, 5H).
**RMN ¹³C (100 MHz, CDCl₃) :** δ = 29,9 ; 36,1 ; 70,2 ; 115,1 ; 127,7 ; 128,1 ; 128,8 ; 129,4 ; 132,7; 137,2 ; 157,5 ; 179,6.
**SMHR (ESI+) :** *m*/*z* calculée pour C₁₆H₁₆O₃Na 279,0997, trouvée 279,0999.

### 5-(4-Benzyloxyphényl)-3-oxopentanoate d'éthyle (3)

A une solution d'acide **2** (30,41 g, 0,12 mol) dans 300 mL de CH₂Cl₂ anhydre, est ajouté, goutte à goutte, sous argon et à -78°C, le chlorure d'oxalyle (20,4 mL, 0,24 mol). Dans le cas où la réaction ne démarre pas d'elle-même, 2 gouttes de DMF sont ajoutées. Après 3 h d'agitation à température ambiante, le milieu réactionnel est concentré sous vide pour fournir une huile jaune. Simultanément, à une solution d'acide de Meldrum's (17,10 g, 0,12 mol) dans 115 mL de CH₂Cl₂ anhydre, est ajouté, goutte à goutte, sous argon et à 0°C, la pyridine anhydre (19,2 mL, 0,24 mol). Après 1 h d'agitation à 0°C, le chlorure d'acyle précédemment obtenu en solution dans 55 mL de CH₂Cl₂ anhydre est ajouté lentement. Le milieu réactionnel est ajouté 16 h à 0°C puis 2 h à température ambiante avant d'être quenché par ajout d'une solution aqueuse 1N de HCl. Après extraction au CH₂Cl₂, les phases organiques sont séchées sur MgSO₄, filtrées et concentrées sous vide. L'huile orange ainsi obtenue, en solution dans 180 mL d'éthanol absolu, est portée 2,5 h à reflux. Le milieu réactionnel est ensuite concentré sous vide. La purification est effectuée sur gel de silice (cyclohexane/AcOEt 9:1) pour donner le composé **3** (23,74 g, 0,07 mol, 61 %) sous forme d'une huile incolore. 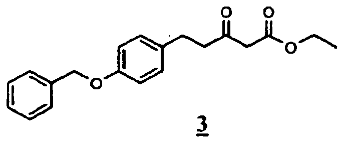 C₂₀H₂₂O₄
M = 326,4 g.mol⁻¹
**RMN ¹H (400 MHz, CDCl₃) :** δ = 1,26 (t, *J* = 7,2 Hz, 3H) ; 2,79-2,92 (m, 4H) ; 3,41 (s, 2H) ; 4,17 (q, *J* = 7,2 Hz, 2H) ; 5,03 (s, 2H) ; 6,89 (d, *J* = 8,4 Hz, 2H) ; 7,09 (d, *J* = 8,4 Hz, 2H) ; 7,28-7,45 (m, 5H).
**RMN ¹³C (100 MHz, CDCl₃) :** δ = 14,2 ; 28,7 ; 44,8 ; 49,6 ; 61,5 ; 70,1 ; 115,0 ; 127,6 ; 128,0 ; 128,7 ; 129,4 ; 133,0 ; 137,2 ; 157,4 ; 167,2 ; 202,1.
**Analyse élémentaire :** calculée pour C₂₀H₂₂O₄ : C 73,60, H 6,79 ; trouvée : C 73,88, H 6,99.

### 5-(4-Hydroxyphényl)-3-oxopentanoate d'éthyle (4)

A une solution de phénol protégé **3** (23,74 g, 0,07 mol) dans 320 mL de AcOEt est ajouté, à température ambiante, 2,0 g de Pd/C 10 %. Le milieu réactionnel est agité une nuit sous atmosphère de H₂ puis il est filtré sur Célite^{®}, lavé à l'AcOEt. Le filtrat est ensuite concentré sous vide pour fournir le produit attendu **4** (17,02 g, 0,07 mol, 99%) sous forme d'huile incolore. Le produit brut est utilisé dans l'étape suivante sans autre purification. 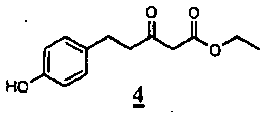 C₁₃H₁₆O₄
M = 236,3 g.mol⁻¹.
**RMN ¹H (400 MHz, CDCl₃) :** δ = 1,26 (t, *J* = 7,1 Hz, 3H) ; 2,80-2,86 (m, 4H); 3,43 (s, 2H) ; 4,18 (q, *J* = 7,1 Hz, 2H) ; 6,75 (d, *J*= 8,4 Hz, 2H) ; 7,01 (d, *J* = 8,4 Hz, 2H).
**RMN ¹³C (100 MHz, CDCl₃) :** δ = 14,2 ; 28,8 ; 45,0 ; 49,6 ; 61,8 ; 115,6 ; 129,6 ; 132,4 ; 154,4; 167,6; 202,8.
**Masse (IE)** : *m*/*z* (%) 236 [M]⁺ (94), 190 (42), 164 (66), 147 (92), 108 (100).
**SMHR (IE) :** *m*/*z* calculée pour C₁₃H₁₆O₄ 236,1049, trouvée 236,1070.

### {2-[2-(4-Hydroxyphényl)-éthyl]-[1,3]-dithian-2-yl}-acétate d'éthyle (4')

A une solution de β-cétoester **4** (17,02 g, 0,07 mol) et de 1,3-propanedithiol (7,30 mL, 0,07 mol) dans 90 mL de CH₂Cl₂ anhydre, est ajouté, goutte à goutte, sous argon et à température ambiante, 10,1 mL de BF₃.OEt₂. Après une nuit d'agitation, le milieu réactionnel est quenché par ajout de 90 mL d'une solution aqueuse de NaOH 1N. Après extraction au CH₂Cl₂, les phases organiques sont séchées sur MgSO₄, filtrées et concentrées sous vide. La purification est effectuée sur gel de silice (cyclohexane/AcOEt 85:15) pour donner le composé **4**' (18,64 g, 0,06 mol, 79 %) sous forme d'une huile incolore. 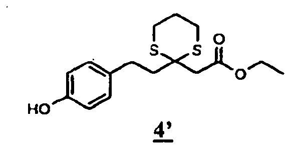 C₁₆H₂₂O)S₂
M = 326,5 g.mol⁻¹
**RMN ¹H (400 MHz, CDCl₃) :** δ = 1,29 (t, *J* = 7,2 Hz, 3H) ; 1,84-1,96 (m, 1H) ; 2,05-2,14 (m, 1H) ; 2,30-2,36 (m, 2H) ; 2,73-2,84 (m, 4H) ; 3,04 (ddd, *J* = 14,0, 10,8, 4,8 Hz, 2H) ; 3,12 (s, 2H) ; 4,18 (q, *J* = 7,2 Hz, 2H) ; 6,76 (d, *J* = 8,8 Hz, 2H) ; 7,07 (d, *J* = 8,8 Hz, 2H).
**RMN ¹³C (100 MHz, CDCl₃) :** δ = 14,4 ; 25,1 ; 26,6 ; 27,1 ; 41,9 ; 43,0 ; 50,2 ; 61,1 ; 115,5 ; 129,8 ; 133,6; 154,2 ; 169,4.
**Masse** (**IE**) : *m*/*z* (%) 326 [M]⁺ (37), 251 (10), 239 (20), 219 (85), 205 (42), 145 (52), 107 (100).
**SMHR (IE) :** *m*/*z* calculée pour C₁₆H₂₂O)S₂ 326,1010, trouvée 326,1034.

### Voie de syhtbèse alternative du composé 4' :

### 3-(4 Hydroxy-phényl)-propionique acide éthyl ester (4'-1)

A une solution d'acide 3-(4 Hydroxy-phényl)-propionique commercial (13,08 g ; 78,79 mmol) dans 100 mL d'éthanol sont ajoutées, sous agitation, 4 gouttes d'HCl concentré. Le milieu réactionnel est chauffé à reflux dans un bain à 145°C pendant une nuit. L'éthanol est évaporé et de l'acétate d'éthyle est ajouté. La phase organique est lavée par une solution saturée NaHCO₃ et par une solution NaCl. Après séchage sur MgSO₄ et évaporation, une huile est obtenue. Cette dernière est distillée par Kugelrohr (150-175°C ; 1,2 mmbar). Une huile transparente qui cristallise est obtenue pour donner le composé attendu **4'-1** (13,8 g ; 71,1 mmol ; 90%) sous forme d'un solide blanc. 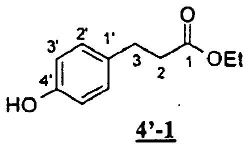 C₁₁H₁₄O₃
M = 194 g.mol⁻¹
**R_{f}** = 0.31 (1:4 ethyl acetate/cyclohexane)
**UV :** 278, 224, 199 nm.
**IRvₘₐₓ (film, cm⁻¹):** 3391, 2983, 1709, 1615, 1517, 1447, 1374, 1225, 1036.
**¹H NMR (400 MHz, CDCl₃)** : δ = 1.22 (t, *J* = 7.2 Hz, 3H, -OCH₂*CH₃*), 2.59 (t, *J* = 7.9 Hz, 2H, 2xH3), 2.86 (t, *J* = 7.9 Hz, 2H, 2xH2), 4.12 (q, *J* = 7.2 Hz, 2H, -O*CH₂*CH₃), 6.75 (d, *J* = 8.5 Hz, 2H, 2xH2'), 7.01 (d, *J*= 8.5 Hz, 2H, 2xH3').
**¹³C NMR (100 MHz, CDCl₃) :** δ = 14.2 (-OCH₂*CH₃*), 30.21 (C3), 36.5 (C2), 60.9 (-O*CH₂*H₃), 115.5 (2xC2'), 129.4 (2xC3'), 132.1 (C1'), 154.5 (C4'), 174.2 (C1).
**LRMS (ESI+):** *m*/*z* (%) 217 (26) [*M*+Na]⁺, 107 (100).

### 5-(4-Hydroxyphényl)-3-oxopentanoate de tert-butyle (4'-2)

Dans un ballon de 100 mL est placée la diisopropyle amine (4,65 mL ; 33,0 mmol ; 5 equiv.) dans du THF anhydre (25 mL) sous atmosphère d'azote. La solution est refroidie à -20°C et le *n*-BuLi à 2,5 M dans l'hexane (13,5 mL ; 33,0 mmol ; 5 equiv.) est ajouté goutte-à-goutte. La solution est alors agitée pendant 15 min à 0°C puis refroidie à -78°C. Le *t*-butyl acétate (2,74 mL ; 33,0 mmol ; 5 equiv.) est injecté goutte-à-goutte. Après 30 min, l'ester 4'-1 (1,29 g; 6,6 mmol), dans du THF anhydre (15 mL), est injecté goutte-à-goutte par une seringue. Le mélange réactionnel est agité pendant 30 min à -78°C, puis encore 30 min à 0°C. La réaction est stoppée par l'ajout de 15 mL d'acide acétique. La solution est lavée avec 50 mL d'une solution saturée en K₂CO₃. Le produit est extrait avec 3x100 mL d'acétate d'éthyle, séché avec MgSO₄ et le solvant est ensuite évaporé. Après une colonne chromatographique (AcOEt/cyclohexane 1:3), le produit **4'-2** (1,36 g ; 78%) est isolé sous forme d'huile jaune. 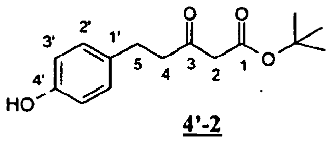 C₁₅H₂₀O₄
M = 264 g.mol⁻¹
R_{f} = 0.23 (1:3 ethyl acetate/cyclohexane)
**IR vₘₐₓ (film, cm⁻¹):** 3457, 3403, 3331, 2978, 2932, 1738, 1713, 1614, 1597, 1516, 1454, 1368, 1323, 1263, 1161, 1080, 951, 831, 768 cm⁻¹.
**UV:** 286, 280, 224, 205 nm.
**¹H NMR (300 MHz, CDCl₃):** δ = 1.47 (s, 9H, -OC(CH₃)₃), 2.78-2.82 (m, 4H, 2xH4, 2xH5), 3.34 (s, 2H, 2xH2), 6.75 (d, *J* = 8.3 Hz, 2H, 2xH3'), 6.99 (d, *J* = 8.3 Hz, 2H, 2xH2').
**¹³C NMR (75 MHz, CDCl₃):** δ = 27.8 (-OC(*C*H₃)₃), 28.5 (C5), 44.7 (C4), 50.6 (C2), 81.6 (-O*C*(CH₃)₃), 115.3(2xC3'), 129.2 (2xC2'), 131.9 (C4'), 154.3 (C1'), 166.7 (C1).
**LRMS (DCI, NH₃+isobutane):** *m*/*z* (%) 282 (6) [*M*+NH₄]⁺, 265 (5) [*M*+H]⁺, 226 (39), 209 (43), 107 (100).

### {2-[2-(4-Hydroxyphényl)-éthyl]-[1,3]-dithian-2-yl}-acétate d'éthyle (4')

Dans un ballon de 100 mL sont ajoutés l'ester **4'-2** (1,118 g, 4,23 mmol) dans CH₂Cl₂ anhydre (11 mL), le 1,3 propanedithiol (456 mg, 4,23 mmol) et 0,82 mL de BF₃OEt₂, sous argon et à température ambiante. Après 2 h d'agitation (durée maximale à ne pas dépasser, afin de ne pas obtenir un produit parasite), la réaction est stoppée par l'ajout de 8 mL de NaOH à 1N. Le milieu est réacidifié par 8 mL de HCl à 1 N. Après extraction au CH₂Cl₂ puis à AcOEt, les phases organiques sont séchées sur MgSO₄, filtrées et concentrées sous vide.

Le produit est utilisé directement pour l'étape suivante

L'acide obtenu ci-dessus est introduit dans un ballon avec 40 mL d'éthanol ainsi que 10 gouttes de HCL concentré. Le mélange réactionnel est agité et chauffé à reflux dans un bain à 145°C pendant 2 nuits. Après évaporation et colonne chromatographique (AcOEt/cyclohexane 1,5:8,5), l'ester **4'** (0,709 g) est isolé sous forme d'une huile incolore, avec un rendement de 51,3% à partir de la molécule **4'-2.**

### {2-[2-(4-Hydroxyphényl)-éthyl]-[1,3]-dithian-2-yl}-acéthaldéhyde (1)

A une solution d'ester **4'** (3,32 g, 10,2 mmol) dans 80 mL de toluène anhydre, est ajoutée, goutte à goutte, sous argon et à -78°C, une solution commerciale de DIBAL-H 1,5 M dans le toluène (14,2 mL, 21,3 mmol). Après 20 min d'agitation à cette température, le milieu réactionnel est quenché par ajout de 17 mL de MeOH. Après retour à température ambiante (1h); une solution aqueuse de NaOH 1N est ajoutée. Après extraction à l'AcOEt, les phases organiques sont séchées sur MgSO₄, filtrées et concentrées sous vide. La purification est effectuée sur gel de silice (cyclohexane/AcOEt 8:2) pour donner l'aldéhyde **1** (2,09 g, 7,4 mmol, 73 %) sous forme d'un solide blanc. 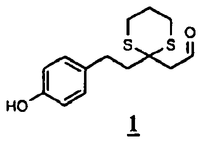 C₁₄H₁₈O₂S₂
M = 282,4 g.mol⁻¹
**RMN ¹H (400 MHz, CDCl₃) :** δ = 1,92-2,18 (m, 2H) ; 2,22-2,30 (m, 2H) ; 2,72-2,80 (m, 2H) ; 2,85-2,93 (m, 4H) ; 2,94 (d, *J =* 2,6 Hz, 2H) ; 6,77 (d, *J =* 8,4 Hz, 2H) ; 7,04 (d, *J =* 8,4 Hz, 2H) ; 9,82 (t, *J* = 2,6 Hz, 1H).
**RMN ¹³C (100 MHz, CDCl₃) :** δ = 24,7 ; 26,3 ; 30,0 ; 42,7 ; 49,3 ; 50,5 ; 115,6 129,7 133,2 ; 154,2 ; 200,5.
**Masse (IE) :** *m*/*z (%)* 282 [M]⁺ (8), 264 [M-H₂O]⁺ (8), 207 (5), 175 (21), 107 (100).
**Analyse élémentaire :** calculée pour C₁₄H₁₈O₂S₂ : C 59,54, H 6,42 ; trouvée : C 59,32, H 6,51.

### Exemple 1 : Préparation du 6-(2-Hydroxy-3-{2-[2-(4-hydroxyphényl)-éthyl]-[1,3]-dithian-2-yl}-propyl)-2,2-diméthyl-[1,3]-dioxin-4-one (5)

L'aldéhyde **1** utilisé dans cette préparation possède la structure suivante :

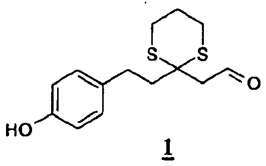

A une solution de diisopropylamine (0,25 mL, 1,77 mmol) dans 5 mL de THF anhydre sont ajoutés goutte à goutte, sous argon et à 0°C, 0,7 mL d'une solution de *n*-BuLi 1M dans l'hexane. Après 20 min d'agitation à 0°C, la 2,2,6-triméthyl-1,3-dioxin-4-one (210 mg, 1,49 mmol) est additionnée lentement à -78°C au milieu réactionnel. Après 45 min d'agitation à cette température, l'aldéhyde **1** (200 mg, 0,71 mmol) est ajouté au milieu. Après 40 min d'agitation à -78°C, on laisse le milieu réactionnel revenir à température ambiante, puis la réaction est quenchée par ajout d'une solution aqueuse saturée de NH₄Cl (jusqu'à pH 7 environ). Après extraction à l'éther, les phases organiques sont séchées sur MgSO₄, filtrées et concentrées sous vide. La purification est effectuée sur gel de silice (cyclohexane/AcOEt 6:4) pour donner le composé attendu (240 mg, 0,57 mmol, 80 %) sous forme d'une laque blanche. 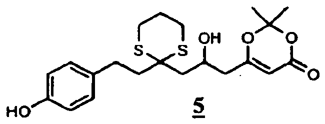 C₂₁H₂₈O₅S₂
M = 424,6 g.mol⁻¹
**RMN ¹H (400 MHz, CDCl₃):** δ = 1,69 (s, 3H, -C*H₃*) ; 1,70 (s, 3H, -C*H₃*); 1,90-2,06 (m, 3H) ; 2,11-2,20 (m, 1H) ; 2,25-2,46 (m, 4H) ; 2,65-2,82 (m, 6H) ; 4,28-4,33 (m, 1H) ; 5,35 (s, 1H); 6,79 (d, *J* = 8,4 Hz, 2H) ; 7,05 (d, *J* = 8,4 Hz, 2H).
**RMN ¹³C (100 MHz, CDCl₃) :** δ = 24,7 (2C) ; 25,4 ; 26,1 ; 26,3 ; 29,7 ; 41,8 ; 42,1 ; 44,6 ; 51,7 65,8 ; 5,3 ; 106,8 ; 115,5 ; 129,5 ; 133,1 ; 154,2 ; 161,4 ; 168,8.
**Masse (ESI+):** *m*/*z (*%*)* 463 [M+K]⁺ (6), 447 [M+Na]⁺ (56), 389 [M-C₃H₆O+Na]⁺ (100).
**SMHR (ESI+):** *m*/*z* calculée pour C₂₁H₂₈O₅NaS₂ 447,1276, trouvée 447,1270.

### Exemple 2: Préparation du (5R)- et (5S)-5-Hydroxy-6-{2-[2-(4-hydroxyphényl)-éthyl]-[1,3]dithian-2-yl}3-oxobexanoate de propyle (6a)

A une solution de dérivé phénolique **5** (500 mg, 1,18 mmol) dans 15 mL de toluène anhydre est ajouté le 1-propanol commercial (142 mg, 2,36 mmol). Le milieu réactionnel est porté 9 h à 110°C avant d'être concentré sous vide. La purification est effectuée sur gel de silice (cyclohexane/AcOEt 6:4) pour donner le mélange racémique du β-cétoester attendu (233 mg, 0,55 mmol, 46 %) sous forme d'une huile incolore. Ce produit est en fait un mélange de deux composés en équilibre : le β-cétoester et sa forme énolique correspondante dans un rapport 91:9. 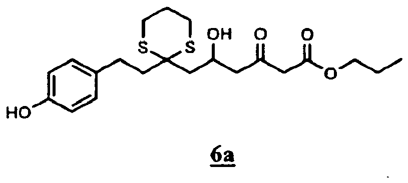 C₂₁H₃₀O₅S₂
M = 426,6 g.mol⁻¹
**RMN ¹H (400 MHz, CDCl₃) :** δ = 0,94 (t, *J* = 7,4 Hz, 3H); 1,61-1,72 (m, 2H) ; 1,86-2,05 (m, 3H) ; 2,13-2,30 (m, 2H) ; 2,34 (dd, *J* = 15,2, 8,8 Hz, 1H) ; 2,65 (dd, *J* = 16,8, 4,8 Hz, 1H) ; 2,69-2,98 (m, 7H) ; 3,51 (d, *J* = 1,6 Hz, 2H) ; 4,10 (t, *J* = 6,8 Hz, 2H) ; 4,46-4,54 (m, 1H) ; 6,75 (d, *J* = 8,4 Hz, 2H) ; 7,04 (d, *J* = 8,4 Hz, 2H).
**RMN ¹³C (100 MHz, CDCl₃) :** δ = 10,4 ; 21,9 ; 25,0 ; 26,1 ; 26,4 ; 29,6 ; 41,9 ; 43,9 ; 50,0 ; 50,3 ; 51,9 ; 65,2 ; 67,3 ; 115,5 ; 129,6 ; 133,3 ; 154,3 ; 167,5 ; 202,4.
**Masse (ESI+) :** *m*/*z (%)* 449 [M+Na]⁺ (25), 409 [M+H-H₂O]⁺ (8).
**SMHR (IE) :** *m*/*z* calculée pour C₂₁H₃₀O₅S₂ 426,1535, trouvée 426,1574.

### Exemple 3: Préparation du (3R,5S)- et (3S,5R)-3,5-Dihydroxy-6-{2-[2-(4hydroxyphényl)-éthyl]-[1,3]-dithian-2-yl}-hexanoate de propyle (8a)

A une solution de cétone **6a** (200 mg, 0,47 mmol) dans 2,6 mL de THF anhydre sont ajoutés, à température ambiante et sous argon, 1,0 mL d'une solution commerciale 1M de BEt₃ dans l'hexane puis 1,0 mL de MeOH anhydre. Après 1 h d'agitation à température ambiante, le milieu réactionnel est refroidi à -78°C puis NaBH₄ (37 mg, 0,98 mmol) est ajouté en une fois. Après 2 h d'agitation, le milieu réactionnel est quenché à -78°C par ajout d'un tampon méthanolique (pH = 7, AcONa/AcOH). Après une nuit d'agitation à température ambiante, le milieu réactionnel est concentré, repris par une solution aqueuse saturée de K₂CO₃ puis extrait à l'AcOEt. Les phases organiques sont alors rassemblées, séchées sur MgSO₄, filtrées et concentrées sous vide. Le résidu est alors repris par du MeOH et agité une nuit à température ambiante. Après évaporation du MeOH, la purification est effectuée sur gel de silice (cyclohexane/AcOEt 8:2) pour donner le mélange racémique du diol de configuration syn (135 mg, 0,31 mmol, 67 %) sous forme d'une huile incolore. 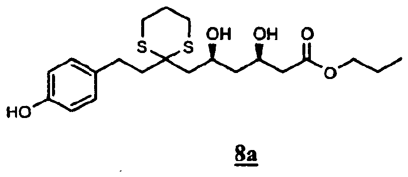 C₂₁H₃₂O₅S₂
M = 428,6 g.mol⁻¹
**RMN ¹H (400 MHz, CDCl₃) :** δ = 0,93 (t, *J* = 7,4 Hz, 3H) ; 1,47-1,54 (m, 1H) ; 1,60-1,77 (m, 3H) ; 1,87-2,06 (m, 3H) ; 2,10-2,20 (m, 1H) ; 2,21-2,30 (m, 1H) ; 2,35 (dd, *J* = 15,4, 9,0 Hz, 1H) ; 2,48 (dd, *J* = 16,0, 4,8 Hz, 1H) ; 2,55 (dd, *J =* 16,0, 7,6 Hz, 1H) ; 2,61-2,70 (m, 1H) ; 2,73-3,00 (m, 5H) ; 4,06 (t, *J* = 6,8 Hz, 2H) ; 4,24-4,36 (m, 2H) ; 6,76 (d, *J* = 8,4 Hz, 2H) ; 7,03 (d, *J* = 8,4 Hz, 2H).
**RMN ¹³C (100 MHz, CDCl₃) :** δ = 10,5 ; 22,0 ; 24,9 ; 26,1 26,4 ; 29,8 ; 41,9 ; 42,0 ; 43,1 ; 45,0 ; 51,9 ; 66,6 ; 68,4 ; 69,1 ; 115,6 ; 129,6 ; 133,1 ; 154,5 ; 172,6.
**Masse (ESI+) :** *m*/*z (*%*)* 451 [M+Na]⁺ (100), 429 [M+H]⁺ (1), 197 (20).
**SMHR (IE) :** *m*/*z* calculée pour C₂₁H₃₂O₅S₂ 428,1691, trouvée 428,1713.

### Exemple 4: Préparation du (2S,4R,6R)- et (2R,4S,6S)-(4-Hydroxy-11-oxo-1,7-dioxadispiro[5.1.5.2]pentadéca-9,12-dièn-2-yl)-acétoate de propyle (10a) et (2S,4R,6S)- et (2R,4S,6R)-(4-Hydroxy-11-oxo-1,7-dioxadispiro[5.1.5.2]pentadéca-9,12-dièn-2-yl)-acétoate de propyle (11a)

A une solution de diol syn **8a** (120 mg, 0,28 mmol) dans 10 mL d'un mélange acétone/eau (9:1) est ajouté, dans le noir et en une fois, le PIFA (385 mg, 0,90 mmol). Après 15 minutes d'agitation à température ambiante, le milieu réactionnel est quenché par ajout d'une solution saturée de NaHCO₃ avant d'être extrait à l'AcOEt. Les phases organiques sont rassemblées, séchées sur MgSO₄ et concentrées sous vide. La purification est effectuée sur gel de silice (cyclohexane/AcOEt 5:5) pour donner les produits attendus **10a** (16 mg, 48 µmol, 17 %) et **11a** (20 mg, 59 µmol, 21 %) sous forme d'huiles incolores. 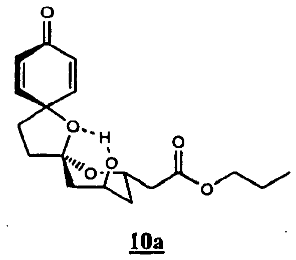 C₁₈H₂₄O₆
M = 336,4 g.mol⁻¹
**RMN ¹R (400 MHz, CDCl₃) :** δ = 0,96 (t, *J* = 7,4 Hz, 3H) ; 1,47-1,56 (m, 1H) ; 1,63-1,73 (m, 2H); 1,80-1,87 (m, 1H) ; 1,91-2,06 (m, 4H); 2,17-2,25 (m, 1H) ; 2,30-2,39 (m, 1H); 2,44-2,55 (m, 2H) ; 4,01-4,08 (m, 1H) ; 4,10-4,20 (m, 2H) ; 4,60-4,68 (m, 1H) ; 6,10 (dd, *J* = 10,1, 2,0 Hz, 1H) ; 6,19 (dd, *J* = 10,1, 2,0 Hz, 1H) ; 6,76 (dd, *J* = 10,1, 3,0 Hz, 1H) ; 7,16 (dd, *J* = 10,1, 3,0 Hz, 1H).
**RMN ¹³C (100 MHz, CDCl₃) :** δ = 10,6 ; 22,2 ; 34,2 ; 37,7 ; 38,8 ; 39,1 ; 41,0 ; 62,7 ; 64,7 ; 66,4 ; 80,3 ; 109,1 ; 127,3 ; 127,6 ; 148,7 ; 151,6 ; 171,4 ; 185,6.
**Masse (ESI+) :** *m*/*z (*%*)* 359 [M+Na]⁺ (100), 319 [M+H-H₂O]⁺ (3), 107 (41).
**SMHR (IE) :** *m*/*z* calculée pour C₁₈H₂₄O₆ 336,1573, trouvée 336,1565. 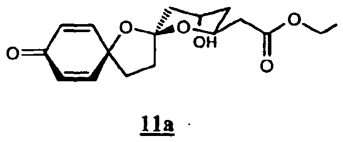 C₁₈H₂₄O₆
M = 336,4 g.mol⁻¹
**RMN ¹H (400 MHz, CDCl₃) :** δ = 0,96 (t, *J* = 7,4 Hz, 3H) ; 1,58-1,72 (m, 4H) ; 1,87-2,11 (m, 4H) ; 2,26-2,36 (m, 1H) ; 2,48 (dd, *J* = 15,5, 4,2 Hz, 1H) ; 2,64 (dd, *J* = 15,5, 9,2 Hz, 1H) ; 2,72-2,79 (m, 1H) ; 4,01-4,13 (m, 2H) ; 4,37-4,48 (m, 2H) ; 6,11 (dd, *J* = 10,2, 3,6 Hz, 1H) ; 6,11 (dd, *J* = 10,2, 3,6 Hz, 1H) ; 6,77 (dd, *J* = 10,2, 3,0 Hz, 1H) ; 6,91 (dd, *J* = 10,2, 3,0 Hz, 1H).
**RMN ¹³C (100 MHz, CDCl₃) :** δ = 10,6 ; 22,2 ; 35,3 ; 35,7 ; 37,3 ; 40,4 ; 41,1 ; 64,9 ; 66,5 ; 66,9 ; 78,1 ; 108,9 ; 127,2 ; 127,4 ; 149,3 ; 152,2 ; 171,6 ; 185,9.
**Masse (ESI+) :** *m*/*z (*%*)* 359 [M+Na]⁺ (100), 319 [M+H-H₂O]⁺ (3), 107 (39).
**SMHR (IE) :** *m*/*z* calculée pour C₁₈H₂₄O₆ 336,1573, trouvée 336,1567.

### Exemple 5: Préparation du (5R)- et (5S)-5-Hydroxy-6-{2-[2-(4-hydroxyphényl)-éthyl]-[1,3]-dithian-2-yl}-3-oxohexanoate de décyle (6b)

A une solution de dérivé phénolique **5** (500 mg, 1,18 mmol) dans 17 mL de toluène anhydre est ajouté le 1-décanol commercial (373 mg, 2,36 mmol). Le milieu réactionnel est porté 6 h à 110°C avant d'être concentré sous vide. La purification est effectuée sur gel de silice (cyclohexane/AcOEt 8:2 à 7:3) pour donner le mélange racémique du β-cétoester (387 mg, 0,74 mmol, 63 %) sous forme d'une huile incolore. Ce produit est en fait un mélange de deux composés en équilibre : le β-cétoester et sa forme énolique correspondante dans un rapport 93:7. 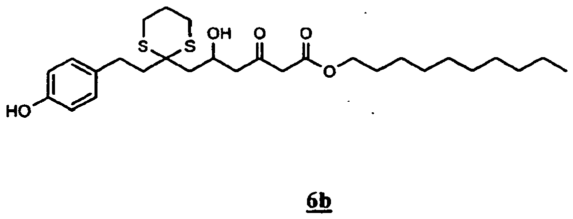 C₂₈H₄₄O₅S₂
M = 524,8 g.mol⁻¹
**RMN ¹H (400 MHz, CDCl₃) :** δ = 0,88 (t, *J* = 6,6 Hz, 3H) ; 1,20-1,38 (m, 14H) ; 1,59-1,68 (m, 2H) ; 1,87-2,06 (m, 3H) ; 2,13-2,28 (m, 2H) ; 2,34 (dd, *J* = 15,2, 8,8 Hz, 1H) ; 2,62 (dd, *J* = 17,2, 4,6 Hz, 1H) ; 2,67-2,98 (m, 7H) ; 3,50 (d, *J* = 1,6 Hz, 2H) ; 4,13 (t, *J* = 6,8 Hz, 2H) ; 4,45-4,52 (m, 1H) ; 6,75 (d, *J* = 8,0 Hz, 2H) ; 7,05 (d, *J* = 8,0 Hz, 2H).
**RMN ¹³C (100 MHz, CDCl₃) :** δ = 14,1 ; 22,6 ; 24,9 ; 25,8 ; 26,0 ; 26,3 ; 28,4 ; 29,2-29,5 ; 31,8 ; 41,7 ; 43,8 ; 49,9 ; 50,2 ; 51,8 ; 65,1 ; 65,8 ; 115,4 ; 129,6 133,3 ; 154,1 ; 167,4 ; 202,3.
**Masse (FAB+, NBA) :** *mlz (*%*)* 547 [M+Na]⁺ (25), 524 [M]⁺ (63), 507 (82), 239 (100). **SMHR (ESI+) :** *mlz* calculée pour C₂₈H₄₄O₅NaS₂ 547,2528, trouvée 547,2522.

### Exemple 6: Préparation du (3S,5R)- et (3R,5S)-3,5-Dihydroxy-6-{2-[2-(4-hydroxyphéoyl)-éthyl]-[1,3]-dithian-2-yl}-hexanoate de décyle (8b)

A une solution de cétone **6b** (360 mg, 0,69 mmol) dans 4,0 mL de THF anhydre sont ajoutés, à température ambiante et sous argon, 1,44 mL d'une solution commerciale 1M de BEt₃ dans l'hexane puis 1,7 mL de MeOH anhydre. Après 1 h d'agitation à température ambiante, le milieu réactionnel est refroidi à -78°C puis NaBH₄ (54 mg, 1,44 mmol) est ajouté en une fois. Après 2 h d'agitation, le milieu réactionnel est quenché à -78°C par ajout d'un tampon méthanolique (pH = 7, AcONa/AcOH). Après une nuit d'agitation à température ambiante, le milieu réactionnel est concentré, repris par une solution aqueuse saturée de K₂CO₃ puis extrait à l'AcOEt. Les phases organiques sont alors rassemblées, séchées sur MgSO₄, filtrées et concentrées sous vide. Le résidu est alors repris par du MeOH et agité 3 h à température ambiante. Après évaporation du MeOH, la purification est effectuée sur gel de silice (cyclohexane/AcOEt 8:2 à 7:3) pour donner le mélange racémique du diol de configuration syn (311 mg, 0,59 mmol, 86 %) sous forme d'une huile incolore. 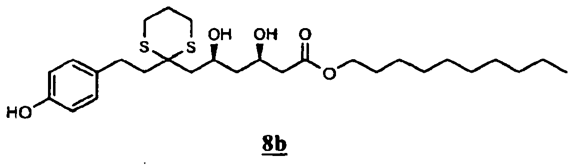 C₂₈H₄₆O₅S₂
M = 526,8 g.mol⁻¹
**RMN ¹H (400 MHz, CDCl₃) :** δ = 0,88 (t, *J* = 6,8 Hz, 3H) ; 1,23-1,35 (m, 14H); 1,49 (ddd, *J* = 14,4, 3,4, 2,8 Hz, 1H) ; 1,57-1,66 (m, 2H) ; 1,71 (ddd, *J* = 14,4, 9,6, 9,6 Hz, 1H) ; 1,89-2,06 (m, 3H) ; 2,11-2,31 (m, 2H) ; 2,36 (dd, *J* = 15,2, 9,2 Hz, 1H) ; 2,46 (dd, *J* = 16,0, 4,8 Hz, 1H) ; 2,53 (dd, *J* = 16,0, 8,0 Hz, 1H) ; 2,62-2,71 (m, 1H) ; 2,73-3,00 (m, 5H) ; 4,09 (t, *J* = 6,6 Hz, 2H) ; 4,24-4,34 (m, 2H) ; 6,76 (d, *J* = 8,4 Hz, 2H) ; 7,04 (d, *J* = 8,4 Hz, 2H).
**RMN ¹³C (100 MHz, CDCl₃) :** δ = 14,1 ; 22,6 ; 24,8 ; 25,8 ; 26,0 ; 26,3 ; 28,5 ; 29,2-29,7 ; 31,8 ; 41,8 ; 41,9 ; 43,0 ; 44,9 ; 51,8 ; 65,0 ; 68,2 ; 68,9 ; 115,5 ; 129,5 ; 133,1 ; 154,3 ; 172,5.
**Masse (FAB+, NBA) :** *m*/*z (*%*)* 549 [M+Na]⁺ (46), 526 [M]⁺ (50), 255 (100), 239 (94).
**SMHR (ESI+) :** *m*/*z* calculée pour C₂₈H₄₆O₅S₂K 565,2424, trouvée 565,2421.

### Exemple 7: Préparation du (2S,4R,6R)- et (2R,4S,6S)-(4-Hydroxy-11-oxo-1,7-dioxadispiro[5.1.5.2]pentadéca-9,12-dièn-2-yl)-acétoate de décyle (10b) et (2S,4R,6S)- et (2R,4S,6R)-(4-Hydroxy-11-oxo-1,7-dioxadispiro[5.1.5.2]pentadéca-9,12-dièn-2-yl)-acétoate de décyle (11b)

A une solution de diol *syn* **8b** (265 mg, 0,50 mmol) dans 27,8 mL d'un mélange acétone/eau (9:1) est ajouté, dans le noir et en une fois, le PIFA (630 mg, 1,46 mmol). Après 15 minutes d'agitation à température ambiante, le milieu réactionnel est quenché par ajout d'une solution saturée de NaHCO₃ avant d'être extrait à l'AcOEt. Les phases organiques sont rassemblées, séchées sur MgSO₄ et concentrées sous vide. La purification est effectuée sur gel de silice (cyclohexane/AcOEt 6:4) pour donner les produits attendus **10b** (38 mg, 87 µmol, 17 %) et **11b** (32 mg, 74 µmol, 15 %) sous forme d'huiles incolores. 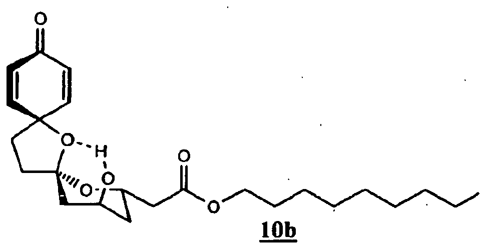 C₂₅H₃₈O₆
M = 434,6 g.mol⁻¹
**RMN ¹H (400 MHz, CDCl₃) :** δ = 0,88 (t, *J* = 7,0 Hz, 3H) ; 1,20-1,38 (m, 14H) ; 1,47-1,56 (m, 1H) ; 1,60-1,70 (m, 2H); 1,79-1,87 (m, 1H) ; 1,91-2,06 (m, 4H); 2,16-2,24 (m, 1H) ; 2,29-2,39 (m, 1H) ; 2,43-2,55 (m, 2H) ; 4,03-4,11 (m, 1H) ; 4,12-4,20 (m, 2H) ; 4,58-4,68 (m, 1H) ; 6,10 (dd, *J* = 10,0, 2,0 Hz, 1H) ; 6,19 (dd, *J* = 10,0, 2,0 Hz, 1H) ; 6,76 (dd, *J* = 10,0, 3,2 Hz, 1H) ; 7,16 (dd, *J* = 10,0, 3,2 Hz, 1H).
**RMN ¹³C (100 MHz, CDCl₃) :** δ = 14,1 ; 22,6 ; 25,9 ; 28,6 ; 29,3-29,5 ; 31,8 ; 34,0 ; 37,4 ; 38,6 ; 38,8 ; 40,8 ; 62,5 ; 64,5 ; 64,8 ; 80,0 ; 108,9 ; 127,0 ; 127,4 ; 148,4 ; 151,4 ; 171,2 ; 185,4.
**Masse (ESI+) :** *m*/*z (*%*)* 457 [M+Na]⁺ (100), 399 (12), 107 (11).
**SMHR (ESI+) :** *mlz* calculée pour C₂₅H₃₈O₆Na 457,2566, trouvée 457,2564. 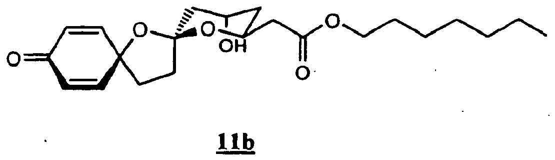 C₂₅H₃₈O₆
M = 434,6 g.mol⁻¹
**RMN ¹H (400 MHz, CDCl₃) :** δ = 0,88 (t, *J* = 6,8 Hz, 3H) ; 1,23-1,37 (m, 14H) ; 1,58-1,75 (m, 4H) ; 1,87-2,11 (m, 4H) ; 2,29 (ddd, *J* = 12,0, 7,8, 4,4 Hz, 1H) ; 2,47 (dd, *J* = 15,2, 4,2 Hz, 1H) ; 2,64 (dd, *J =* 15,2, 9,2 Hz, 1H) ; 2,75 (ddd, *J* = 13,0, 7,3, 1,6 Hz, 1H) ; 4,08 (t, *J* = 12,0 Hz, 1H); 4,10 (t, *J* = 12,0 Hz, 1H) ; 4,37-4,47 (m; 2H) ; 6,10 (dd, *J* = 10,4, 3,5 Hz, 1H) ; 6,11 (dd, *J* = 10,4, 3,5 Hz, 1H) ; 6,76 (dd, *J =* 10,4, 2,9 Hz, 1H) ; 6,91 (dd, *J* = 10,4, 2,9 Hz, 1H).
**RMN ¹³C (100 MHz, CDCl₃) :** δ = 14,3 ; 22,9 ; 25,2 ; 26,2 ; 28,9 ; 29,5-29,9 ; 32,1 ; 35,3 ; 35,7; 37,3 ; 40,4; 41,1 ; 64,9; 65,1 ; 66,9; 78,1 ; 108,8; 127,3; 127,4; 149,2; 152,2; 171,6; 185,8.
**Masse (ESI+) :** *m*/*z (*%*)* 457 [M+Na]⁺ (100), 399 (8), 107 (14).
**SMHR (ESI+) :** *m*/*z* calculée pour C₂₅H₃₈O₆Na 457,2566, trouvée 457,2561.

### Exemple 8: Préparation du (3S,5S)- et (3R,5R)-3,5-Dihydroxy-6-{2-[2-(4-hydroxyphényl)-éthyl]-[1,3]-dithian-2-yl}-hexanoate de décyle (9b)

A une suspension de Me₄NBH(OAc)₃ (752 mg, 2,86 mmol) dans 10 mL d'un mélange MeCN/AcOH (4:1) est ajoutée goutte à goutte, à 0°C, une solution de cétone **6b** (375 mg, 0,72 mmol) dans 6 mL d'un mélange MeCN/CH₂Cl₂ (2:1). Après 3 h d'agitation à 0°C, le milieu réactionnel est dilué dans l'AcOEt puis versé dans 20 mL d'une solution aqueuse saturée de tartrate mixte de sodium et potassium. Après filtration du précipité formé sur coton, les phases sont séparées et la phase aqueuse est de nouveau extraite à l'AcOEt. Les phases organiques sont alors rassemblées, séchées sur MgSO₄, filtrées et concentrées sous vide. La purification est effectuée sur colonne de silice (cyclohexane/AcOEt 8:2 à 7:3) pour donner le mélange racémique du diol de configuration anti (297 mg, 0,56 mmol, 79 %) sous forme d'une huile incolore.
Mélange *synlanti* 2:8. 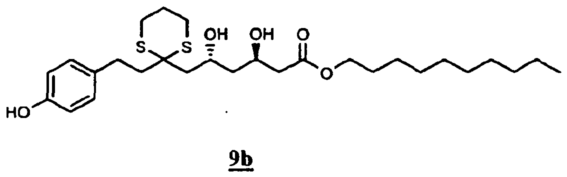 C₂₈H₄₆O₅S₂
M = 526,8 g.mol⁻¹
**RMN ¹H (400 MHz, CDCl₃) :** δ = 0,88 (t, *J* = 6,6 Hz, 3H) ; 1,23-1,35 (m, 14H); 1,57-1,70 (m, 4H) ; 1,87-2,07 (m, 3H) ; 2,09-2,19 (m, 1H) ; 2,20-2,30 (m, 1H) ; 2,32-2,47 (dd, *J* = 15,8, 9,3 Hz, 1H) ; 2,51-2,56 (m, 2H) ; 2,60-2,70 (m, 1H) ; 2,73-2,86 (m, 3H) ; 2,88-3,02 (m, 2H) ; 4,10 (t, *J* = 6,8 Hz, 2H) ; 4,33-4,42 (m, 2H) ; 6,74 (d, *J* = 7,8 Hz, 2H) ; 7,02 (d, *J* = 7,8 Hz, 2H).
**RMN ¹³C (100 MHz, CDCl₃) :** δ = 14,3 ; 22,8 ; 25,0 ; 26,0 ; 26,2 ; 26,5 ; 28,7 ; 29,4-29,9 ; 32,0 ; 41,7 ; 42,2 ; 43,3 ; 44,9 ; 52,1 ; 65,2 65,7 66,2 ; 115,6 ; 129,7 ; 133,3 ; 154,4 ; 173,0.
**Masse (ESI+) :** *m*/*z (*%*)* 565 [M+K]⁺ (8), 549 [M+Na]⁺ (100).
**SMHR (ESI+) :** *m*/*z* calculée pour C₂₈H₄₆O₅NaS₂ 549,2684, trouvée 549,2683.

### Exemple 9: Préparation du (2S,4S,6R)- et (2R,4R,6S)-(4-Hydroxy-11-oxo-1,7-dioxadispiro[5.1.5.2]pentadéca-9,12-dièn-2-yl)-acétoate de décyle (12b) et (2S,4S,6S)- et (2R,4R,6R)-(4-Hydroxy-11-oxo-1,7-dioxadispiro[5.1.5.2]pentadéca-9,12-dièn-2-yl)-acétoate de décyle (13b)

A une solution de diol *anti* **9b** (270 mg, 0,51 mmol) dans 10 mL d'un mélange acétone/eau (9:1) est ajouté, en une fois, le PIFA (705 mg, 1,64 mmol). Après 10 minutes d'agitation à température ambiante, le milieu réactionnel est quenché par ajout d'une solution saturée de NaHCO₃ avant d'être extrait à l'AcOEt. Les phases organiques sont rassemblées, séchées sur MgSO₄ et concentrées sous vide. La purification est effectuée sur gel de silice (cyclohexane/AcOEt 7:3 à 5:5) pour donner les produits attendus **12b** (36 mg, 84 µmol, 16 %) et **13b** (38 mg, 88 µmol, 17 %) sous forme d'huiles incolores. 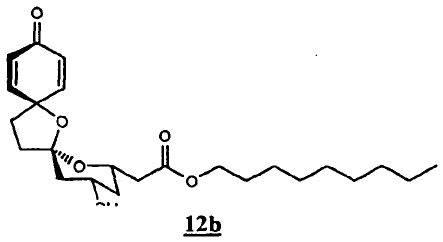 C₂₅H₃₈O₆
M = 434,6 g.mol⁻¹
**RMN ¹H (400 MHz, CDCl₃) :** δ = 0,88 (t, *J* = 6,6 Hz, 3H) ; 1,20-1,39 (m, 15H) ; 1,58-1,70 (m, 3H) ; 1,94-2,12 (m, 4H) ; 2,18-2,25 (m, 1H) ; 2,30-2,37 (m, 1H) ; 2,44-2,57 (m, 2H) ; 4,02-4,10 (m, 1H) ; 4,12-4,21 (m, 2H) ; 4,28-4,36 (m, 1H) ; 6,10 (dd, *J* = 10,0, 1,0 Hz, 1H) ; 6,16 (dd, *J =* 10,0, 1,0 Hz, 1H) ; 6,77 (dd, *J =* 10,0, 3,0 Hz, 1H) ; 7,08 (dd, *J* = 10,0, 3,0 Hz, 1H).
**RMN ¹³C (100 MHz, CDCl₃) :** δ = 14,3 ; 22,9 ; 26,1 ; 28,8 ; 29,5-29,7 ; 32,1 ; 34,7 ; 38,8 ; 40,2 ; 41,1 ; 42,7 ; 65,1 ; 66,0 ; 79,4 ; 109,0 ; 127,1 ; 127,4 ; 149,3 ; 152,2 ; 171,4 ; 185,8.
**Masse (ESI+) :** *mlz (*%*)* 473 [M+K]⁺ (8), 457 [M+Na]⁺ (100).
**SMHR (ESI+) :** *m*/*z* calculée pour C₂₅H₃₈O₆Na457,2566, trouvée 457,2559. 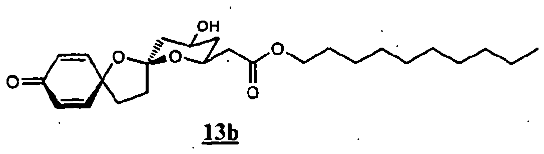 C₂₅H₃₈O₆
M = 434,6 g.mol⁻¹
**RMN¹H (400 MHz, CDCl₃) :** δ = 0,88 (t, *J* = 6,8 Hz, 3H) ; 1,23-1,38 (m, 15H); 1,59-1,70 (m, 2H) ; 1,77-1,90 (m, 2H) ; 1,98-2,10 (m, 2H) ; 2,14 (ddd, *J* = 12,4, 4,4 Hz, 1,2 Hz, 1H) ; 2,24-2,34 (m, 1H) ; 2,45-2,54 (m, 2H) ; 2,67 (dd, *J* = 15,6, 8,8 Hz, 1H) ; 3,87-3,96 (m, 2H) ; 4,04-4,15 (m, 2H) ; 6,11 (dd, *J* = 9,4, 2,0 Hz, 1H) ; 6,13 (dd, *J* = 10,4, 2,0 Hz, 1H) ; 6,77 (dd, *J* = 9,4, 2,9 Hz, 1H) ; 6,90 (dd, *J* = 10,4, 2,9 Hz, 1H).
**RMN ¹³C (100 MHz, CDCl₃) :** δ = 14,3 ; 22,9 ; 26,1 ; 28,9 ; 29,5-29,7 ; 32,1 ; 33,6 ; 35,0 ; 40,0 ; 41,0 ; 43,3 ; 65,2 ; 66,7 ; 68,5 ; 78,5 ; 109,4 ; 127,4 ; 127,7 ; 148,8 ; 151,5 ; 171,4 ; 185,7.
**Masse (ESI+) :** *m*/*z (*%*)* 473 [M+K]⁺ (7), 457 [M+Na]⁺ (100).
**SMHR (ESI+) :** *m*/*z* calculée pour C₂₅H₃₈O₆Na 457,2566, trouvée 457,2565.

### Exemple 10: Préparation du (2S,4S,6S)- et (2R,4R,6R)-(4-Acétoxy-11-oxo-1,7-dioxadispiro[5.1.5.2]pentadéca-9,12-dièn-2-yl)-acétoate de décyle (14b)

A une solution d'alcool **13b** (15,0 mg, 0,03 mmol) dans 1 mL de CH₂Cl₂ sont ajoutés, à température ambiante et sous argon, la DMAP (5,1 mg, 0,04 mmol) et l'anhydride acétique (4,2 mg, 0,04 mmol). Après une nuit d'agitation à température ambiante, le milieu réactionnel est quenché par ajout d'une solution aqueuse 1N de HCl avant d'être extrait au CH₂Cl₂. Les phases organiques sont rassemblées, séchées sur MgSO₄ et concentrées sous vide. La purification est effectuée sur gel de silice (cyclohexanc/AcOEt 8:2) pour donner le produit attendu (9,8 mg, 0,02 mmol, 61 %) sous forme d'huile incolore. 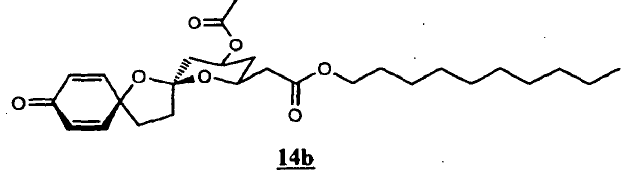 C₂₇H₄₀O₇
M = 476,
**RMN ¹H (400 MHz, CDCl₃) :** δ = 0,88 (t, *J* = 6,8 Hz, 3H) ; 1,23-1,45 (m, 15H); 1,59-1,67 (m, 2H) ; 1,84-1,96 (m, 2H) ; 2,00-2,11 (m, 5H) ; 2,1 (ddd, *J* = 12,4, 4,4 Hz, 1,2 Hz, 1H) ; 2,25-2,34 (m, 1H) ; 2,46-2,58 (m, 2H) ; 2,65 (dd, *J* = 16,0, 9,0 Hz, 1H) ; 3,93-4,01 (m, 1H) ; 4,04-4,15 (m, 2H) ; 4,90-4,99 (m, 1 H) ; 6,11 (dd, *J* = 10,4, 2,0 Hz, 1H) ; 6,13 (dd, *J* = 10,0, 2,0 Hz, 1H) ; 6,74 (dd, *J* = 10,4, 3,0 Hz, 1H) ; 6,89 (dd, *J* = 10,0, 3,0 Hz, 1H).
**RMN ¹³C (100 MHz, CDCl₃) :** δ = 14,3 ; 21,4 ; 22,9 ; 26,1 ; 28,9 ; 29,5-29,7 ; 32,1 ; 33,5 ; 35,0 ; 36,2 ; 39,7 ; 40,9 ; 65,2 ; 68,3 ; 68,6 ; 78,7 ; 109,1 ; 127,5; 127,8; 148,6; 151,2; 170,6 ; 171,0 ; 185,5.
**SMHR (ESI+) :** *m*/*z* calculée pour C₂₇H₄₀O₇Na 499,2672, trouvée 499,2670.

### Exemple 11: Préparation du Heptanoate de 2-décyloxycarbonylméthyl-11-oxo-1,7-dioxadispiro[5.1.5.2]pentadéca-9,12-dièn-4-yle (15b)

A une solution d'alcool **12b** (14,8 mg, 0,03 mmol) dans 1 mL de CH₂Cl₂ anhydre, sont ajoutés, à 0°C et sous argon, la DMAP (4,2 mg, 0,03 mmol), le chlorure d'heptanoyle (6,2 mg, 0,04 mmol) puis la triéthylamine (4,2 mg, 0,04 mmol). Après 0,5 h d'agitation à 0°C et 4 h à température ambiante, le milieu réactionnel est quenché par ajout d'une solution aqueuse 1N de HCl. Après extraction au CH₂Cl₂, les phases organiques sont rassemblées, séchées sur MgSO₄, filtrées et concentrées sous vide. La purification est effectuée sur gel de silice (cyclohexane/AcOEt 85:15) pour donner l'ester attendu (14,0 mg, 0,03 mmol, 75 %) sous forme d'huile incolore. 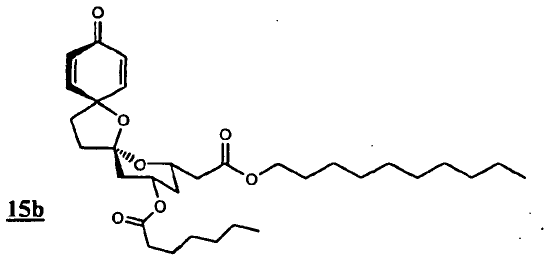 C₃₂H₅₀O₇
M = 546,7 g.mol⁻¹
**RMN ¹H (400 MHz, CDCl₃) :** δ = 0,88 (m, 6H) ; 1,23-1,38 (m, 21H) ; 1,55-1,70 (m, 5H) ; 1,94-2,16 (m, 4H) ; 2,17-2,39 (m, 4H) ; 2,43-2,56 (m, 2H) ; 4,02-4,10 (m, 1H) ; 4,12-4,19 (m, 2H) ; 4,36-4,44 (m, 1H) ; 5,15-5,25 (m, 1H) ; 6,09 (dd, *J* = 10,0, 2,0 Hz, 1H) ; 6,16 (dd, *J* = 10,0, 2,0 Hz, 1H) ; 6,76 (dd, *J* = 10,0, 3,0 Hz, 1H) ; 7,08 (dd, *J* = 10,0, 3,0 Hz, 1H).
**RMN ¹³C (100 MHz, CDCl₃) :** δ = 14,2; 14,3 ; 22,7 ; 22,9 ; 25,1 ; 26,1 ; 28,9 ; 29,0 ; 29,5-29,7 ; 31,6 ; 32,1 ; 34,7 ; 36,6 ; 38,8 ; 39,2 ; 41,0 ; 65,1 ; 65,7 ; 67,6 ; 79,6 ; 108,8 ; 127,1 ; 127,5 ; 149,2 ; 152,0 ; 171,4 ; 173,2 ; 185,8.
**Masse (ESI+) :** *m*/*z (*%*)* 585 [M+K]⁺ (7), 569 [M+Na]⁺ (100).
**SMHR (ESI+) :** *m*/*z* calculée pour C₃₂H₅₀O₇Na 569,3454, trouvée 569,3449.

### Exemple 12: Préparation du (5R)- et (5S)-5-Hydroxy-6-{2-[2-(4-hydroxy-3,5-diméthoxyphényl)-éthyl]-[1,3]-dithian-2-yl}-3-oxohexanoate de décyle (6'b)

### 1) préparation du composé de départ 5' : 4-Benzyloxy-3,5-diméthoxybenzaldéhyde (5'-1)

A une solution de KOH (3,69 g, 65,8 mmol) dans 40 mL d'eau est ajoutée, en plusieurs fractions, une solution d'aldéhyde commercial (6,0 g, 32,9 mmol) et de bromure de benzyle (5,9 mL, 49,4 mmol) dans 70 mL de THF. Une quantité catalytique de Bu₄NHSO₄ (0,56 g, 1,6 mmol) est alors ajoutée puis le milieu réactionnel est agité une nuit à température ambiante. Après extraction au CH₂Cl₂, les phases organiques sont rassemblées, séchées sur MgSO₄, filtrées puis concentrées sous vide. La purification est effectuée sur colonne de silice (cyclohexane/EtOAc 85:15) pour donner l'aldéhyde **5'-1** (8,43 g, 31,0 mmol, 94 %) sous forme d'un solide blanc. 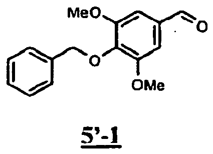 C₁₆H₁₆O₄
M = 272,3 g.mol⁻¹
Analyses : en accord avec la littérature (Bennett C. J., Caldwell S. T., McPhail D. B., Morrice P. C., Duthie G. G., Hartley R. C., Bioorg. & Med. Chem. 2004, 12, 2079-2098)

### 3-oxo-4-(triphénylphosphoranylidène)butanoate d'éthyle (5'-2)

A une solution de 4-chloro-3-oxobutanoate d'éthyle commercial (18,81 g, 114,3 mmol) dans 200 mL de THF anhydre est ajouté, à température ambiante, la triphénylphosphine (30,0 g, 114,3 mmol). Le milieu réactionnel est porté 18 h à reflux avant d'être concentré sous vide. L'huile obtenue est reprise par de l'eau distillée puis lavée par de l'éther. La phase aqueuse est alors basifiée par une solution aqueuse saturée de NaHCO₃ (jusque pH 8). Après extraction à l'AcOEt, les phases organiques sont rassemblées, séchées sur MgSO₄, filtrées puis concentrées sous vide. La purification est effectuée par recristallisation dans l'éther pour donner, après filtration et séchage, le composé **5'-2** (14,96 g, 38,0 mmol, 34 %) sous forme d'un solide légèrement jaune. 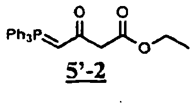 C₂₄H₂₃O₃P
M = 390,4 g.mol⁻¹
Analyses : en accord avec la littérature (Sun K. M., Dawe R. D., Carbohydrate Research 1987, 171, 35-47).

### 5-(4-Benzyloxy-3,5-diméthoxyphényl)-3-oxopentanoate d'éthyle (5'3)

A une solution de phosphoranylidène **5'-2** (6,09 g, 15,61 mmol) dans 25 mL de DMPU et 30 mL de THF anhydre, est ajouté, sous argon et à température ambiante, le NaH (1,32 g, 33,0 mmol, 60 % dans l'huile). Après 20 min d'agitation à température ambiante, l'aldéhyde **5'-1** (2,50 g, 9,18 mmol) en solution dans 10 mL de THF est ajouté lentement au milieu réactionnel. Après 50 min d'agitation à température ambiante, le milieu réactionnel est porté 1,5 h à 40°C. Après une nuit d'agitation à température ambiante, le milieu réactionnel est quenché par ajout d'une solution aqueuse saturée de NH₄Cl. Après extraction à l'Et₂O, les phases organiques sont séchées sur MgSO₄, filtrées et concentrées sous vide. La purification est effectuée sur gel de silice (cyclohexane/AcOEt 9:1) pour donner le composé **5'-3** (2,33 g, 6,06 mmol, 66 %) sous forme d'une huile légèrement jaune. Il s'agit en fait d'un mélange d'isomères *Z* et *E* en équilibre avec les formes énoliques correspondantes. 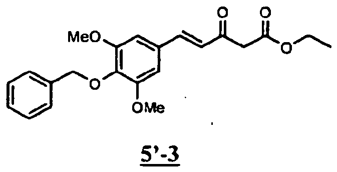 C₂₂H₂₄O₆
M = 384,4 g.mol⁻¹
**Masse (FAB+, NBA) :** *m*/*z (*%*)* 385 [M+H]⁺ (46), 293 (100).

### 5-(4-Hydroxy-3,5-diméthoxyphényl)-3-oxopentanoate d'éthyle (5'-4)

A une solution de composé **5'-3** (578 mg, 1,50 mmol) dans 12 mL de AcOEt est ajouté, à température ambiante, 80 mg de Pd/C 5 %. Le milieu réactionnel est agité une nuit sous atmosphère de H₂ puis il est filtré sur Célite^{®}, lavé à l'AcOEt. Le filtrat est ensuite concentré sous vide pour fournir le produit attendu **5'-4** (423 mg, 1,42 mmol, 95 %) sous forme d'huile incolore. Lé produit brut est utilisé dans l'étape suivante sans autre purification. 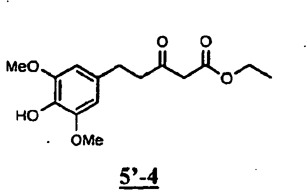 C₁₅H₂₀O₆
M = 296,3 g.mol⁻¹
**RMN ¹H (400 MHz, CDCl₃) :** δ = 1,25 (t, *J* = 7,1 Hz, 3H) ; 2,81-2,86 (m, 4H) ; 3,43 (s, 2H) ; 3,84 (s, 6H) ; 4,18 (q, *J* = 7,1 Hz, 2H) ; 6,41 (s, 2H).
**RMN ¹³C (100 MHz, CDCl₃) :** δ = 14,0 ; 29,5 ; 44,7 ; 49,3 ; 56,1 ; 61,2 ; 104,9 ; 131,5 ; 133,0 ; 147,0 ; 167,1 ; 202,1.
**SMHR (IE) :** *m*/*z* calculée pour C₁₅H₂₀O₆ 296,1260, trouvée 296,1253.

### {2-[2-(4-Hydroxy-3,5-diméthoxyphényl)-éthyl]-[1,3]-dithian-2-yl}-acétate d'éthyle (5'-5)

A une solution de β-cétoester **5'-4** (550 mg, 1,9 mmol) et de 1,3-propanedithiol (0,19 mL, 1,9 mmol) dans 3 mL de CH₂Cl₂ anhydre, est ajouté, goutte à goutte, sous argon et à température ambiante, 0,26 mL de BF₃.OEt₂. Après une nuit d'agitation, le milieu réactionnel est quenché par ajout de 3 mL d'une solution aqueuse de NaOH 1N. Après extraction au CH₂Cl₂, les phases organiques sont séchées sur MgSO₄, filtrées et concentrées sous vide. La purification est effectuée sur gel de silice (cyclohexane/AcOEt 7:3) pour donner le composé **5'-5** (576 mg, 1,5 mol, 80 %) sous forme d'une laque blanche. 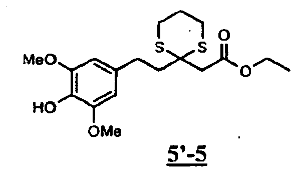 C₁₈H₂₆O₅S₂
M = 386,5 g.mol⁻¹
**RMN ¹H (400 MHz, CDCl₃) :** δ = 1,29 (t, *J* = 7,0 Hz, 3H) ; 1,85-1,98 (m, 1H) ; 2,08-2,16 (m, 1H) ; 2,32-2,39 (m, 2H) ; 2,74-2,86 (m, 4H) ; 3,06 (ddd, *J* = 14,8, 10,8, 2,8 Hz, 2H) ; 3,11 (s, 2H) ; 3,88 (s, 6H) ; 4,18 (q, *J* = 7,0 Hz, 2H) ; 6,46 (s, 2H).
**RMN ¹³C (100 MHz, CDCl₃) :** δ = 14,4 ; 25,1 ; 26,6 ; 30,7 ; 41,9 ; 42,9 ; 50,2 ; 56,4 ; 60,8 ; 105,3 ; 132,8; 133,1 ; 147,1 ; 169,9.
**Masse (ESI+) :** *m*/*z (*%*)* 425 [M+K]⁺ (21), 409 [M+Na]⁺ (100).
**SMHR (ESI+) :** *mlz* calculée pour C₁₈H₂₆O₅NaS₂ 409,1119, trouvée 409,1112.

### {2-[2-(4-Hydroxy-3,5-diméthoxyphényl)-éthyl]-[1,3]-dithian-2-yl}-acéthaldéhyde (1')

A une solution d'ester **5'-5** (1,42 g, 3,7 mmol) dans 35 mL de toluène anhydre, est ajoutée, goutte à goutte, sous argon et à -78°C, 4,6 mL d'une solution commerciale de DIBAL-H 1,7M dans le toluène. Après 15 min d'agitation à cette température, le milieu réactionnel est quenché par ajout de 5 mL de MeOH. Après retour à température ambiante (1h), une solution aqueuse de NaOH 1N est ajoutée. Après extraction à l'AcOEt, les phases organiques sont séchées sur MgSO₄, filtrées et concentrées sous vide. La purification est effectuée sur gel de silice (cyclohexane/AcOEt 8:2) pour donner l'aldéhyde **1'** (853 mg, 2,5 mmol, 68 %) sous forme d'une huile jaune. 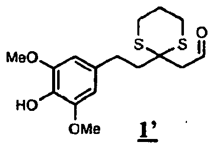 C₁₆H₂₂O₄S₂
M = 342,5 g.mol⁻¹
**RMN ¹H (400 MHz, CDCl₃) :** δ = 1,91-2,08 (m, 2H) ; 2,25-2,30 (m, 2H) ; 2,74-2,78 (m, 2H) ; 2,83-2,94 (m, 4H) ; 2,96 (d, *J* = 2,8 Hz, 2H) ; 3,87 (s, 6H) ; 6,40 (s, 2H) ; 9,82 (t, *J* = 2,8 Hz, 1H).
**RMN ¹³C (100 MHz, CDCl₃) :** δ = 24,6 ; 26,2 ; 30,8 ; 42,5 ; 49,1 ; 50,2 ; 56,3 ; 105,0 ; 132,1 ; 133,1 ; 147,1 ; 199,7.
**Masse (ESI+) :** *m*/*z (*%*)* 397 [M+MeOH+Na]⁺ (100), 365 [M+Na]⁺ (34).

### 6-(2-Hydroxy-3-{2-[2-(4-hydroxy-3,5-diméthoxyphényl)-éthyl]-[1,3]-dithian-2-yl}-propyl)-2,2-diméthyl-[1,3]-dioxin-4-one (5')

A une solution de diisopropylamine (0,87 mL, 6,23 mmol) dans 18 mL de THF anhydre sont ajoutés goutte à goutte, sous argon et à 0°C, 2,5 mL d'une solution de *n*-BuLi 2,5M dans l'hexane. Après 20 min d'agitation à 0°C, la 2,2,6-triméthyl-1,3-dioxin-4-one (744 mg, 5,23 mmol) est additionnée lentement à -78°C au milieu réactionnel. Après 45 min d'agitation à cette température, l'aldéhyde **1'** (853 mg, 2,49 mmol) est ajouté au milieu. Après 40 min d'agitation à -78°C, on laisse le milieu réactionnel revenir à température ambiante, puis la réaction est quenchée par ajout d'une solution aqueuse saturée de NH₄Cl (jusqu'à pH 7 environ). Après extraction à l'AcOEt, les phases organiques sont séchées sur MgSO₄, filtrées et concentrées sous vide. La purification est effectuée sur gel de silice (cyclohexane/AcOEt 6:4) pour donner le composé **5'** (384 mg, 0,79 mmol, 32 % non optimisé) sous forme d'une laque légèrement jaune. 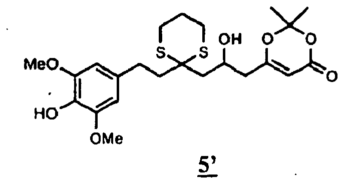 C₂₃H₃₂O₇S₂
M = 484,6 g.mol⁻¹
**RMN ¹H (400 MHz, CDCl₃ :** δ = 1,68 (s, 3H) ; 1,69 (s, 3H) ; 1,90-2,07 (m, 3H) ; 2,12-2,22 (m, 1H) ; 2,25-2,40 (m, 3H) ; 2,45 (dd, *J* = 14,4, 8,0 Hz, 1H) ; 2,65-3,02 (m, 6H) ; 3,87 (s, 6H) ; 4,29-4,36 (m, 1H) ; 5,36 (s, 1H) ; 6,42 (s, 2H).
**RMN ¹³C (100 MHz, CDCl₃) :** δ = 24,6 ; 24,7 ; 25,4 ; 26,1 ; 26,3 ; 30,8 ; 41,9 ; 42,0 ; 44,6 ; 51,7 ; 56,3 ; 65,7 ; 95,2 ; 105,1 ; 106,6 ; 132,4 ; 133,0 ; 147,1 ; 161,1 ; 168,7.

### 2) préparation du composé de départ 6'b :

A une solution de dérivé phénolique **5'** 370 mg, 0,76 mmol) dans 14 mL de toluène anhydre est ajouté le 1-décanol commercial (363 mg, 2,29 mmol). Le milieu réactionnel est porté 6 h à 110°C avant d'être concentré sous vide. La purification est effectuée sur gel de silice (cyclohexane/AcOEt 8:2 à 7:3) pour donner le mélange racémique du β-cétoester (273 mg, 0,47 mmol, 61 %) sous forme d'une huile incolore. Ce produit est en fait un mélange de deux composés en équilibre : le β-cétoester et sa forme énolique correspondante dans un rapport 94:6. 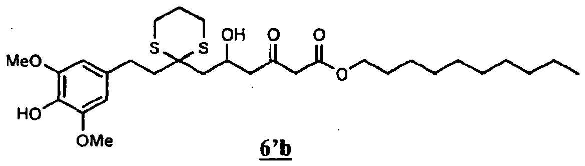 C₃₀H₄₈O₇S₂
M = 584,8 g.mol⁻¹
**RMN ¹H (400 MHz, CDCl₃) :** δ = 0,88 (t, *J* = 6,6 Hz, 3H) ; 1,22-1,38 (m, 14H) ; 1,59-1,68 (m, 2H) ; 1,87-2,09 (m, 3H) ; 2,15-2,30 (m, 2H) ; 2,36 (dd, *J* = 15,6, 9,2 Hz, 1H) ; 2,67-3,03 (m, 8H) ; 3,51 (s, 2H) ; 3,87 (s, 6H) ; 4,12 (t, *J* = 6,8 Hz, 2H) ; 4,47-4,54 (m, 1H) ; 6,45 (s, 2H).
**RMN ¹³C (100 MHz, CDCl₃) :** δ = 14,2 ; 22,8 ; 25,0 ; 26,0 ; 26,2 ; 26,4 ; 28,6 ; 29,3-29,6 ; 32,0 ; 42,0 ; 43,9 ; 50,1 ; 50,4 ; 51,8 ; 56,4 ; 65,1 ; 65,8 ; 105,2 ; 132,8 ; 133,0 ; 147,1 ; 167,2 ; 202,3.
**Masse (ESI+)** : *m*/*z* (%) 623 [M+K]⁺ (6), 607 [M+Na]⁺ (100).
**SMHR (ESI+)** : *m*/*z* calculée pour C₃₀H₄₈O₇NaS₂ 607,2739, trouvée 607,2737.

### Exemple 13: Préparation du (3S,5R)- et (3R,5S)-3,5-Dihydroxy-6-{2-[2-(4-hydroxy-3,5-diméthoxyphényl)-éthyl]-[1,3]-dithian-2-yl}-hexanoate de décyle (8'b)

A une solution de cétone **6'b** (260 mg, 0,44 mmol) dans 2,6 mL de THF anhydre sont ajoutés, à température ambiante et sous argon, 0,99 mL d'une solution commerciale 1M de BEt₃ dans l'hexane puis 1 mL de MeOH anhydre. Après 1 h d'agitation à température ambiante, le milieu réactionnel est refroidi à -78°C puis NaBH₄ (35 mg, 0,93 mmol) est ajouté en une fois. Après 2 h d'agitation, le milieu réactionnel est quenché à -78°C par ajout d'un tampon méthanolique (pH = 7, AcONa/AcOH). Après une nuit d'agitation à température ambiante, le milieu réactionnel est concentré, repris par une solution aqueuse saturée de K₂CO₃ puis extrait à l'AcOEt. Les phases organiques sont alors rassemblées, séchées sur MgSO₄, filtrées et concentrées sous vide. Le résidu est alors repris par du MeOH et agité 3 h à température ambiante. Après évaporation du MeOH, la purification est effectuée sur gel de silice (cyclohexane/AcOEt 6:4) pour donner le mélange racémique du diol de configuration syn (227 mg, 0,39 mmol, 87 %) sous forme d'une huile incolore. 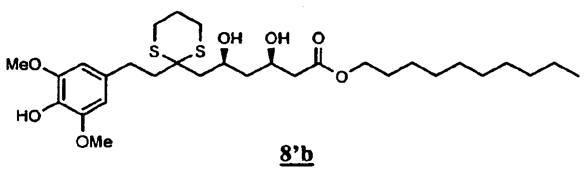 C₃₀H₅₀O₅S₂
M = 586,8 g.mol⁻¹
**RMN ¹H (400 MHz, CDCl₃) :** δ = 0,88 (t, *J* = 7,2 Hz, 3H) ; 1,23-1,38 (m, 14H) ; 1,52-1,79 (m, 4H) ; 1,91-2,09 (m, 3H) ; 2,13-2,32 (m, 2H) ; 2,36 (dd, *J* = 15,2, 9,2 Hz, 1H) ; 2,44-2,57 (m, 2H) ; 2,63-2,73 (m, 1H) ; 2,75-3,03 (m, 5H) ; 3,88 (s, 6H) ; 4,09 (t, *J* = 6,4 Hz, 2H) ; 4,26-4,36 (m, 2H) ; 6,43 (s, 2H).
**RMN ¹³C (100 MHz, CDCl₃) :** δ = 14,3 ; 22,8 ; 25,1 ; 26,0 ; 26,3 ; 26,5 ; 28,7 ; 29,4-29,7 ; 31,1 ; 32,0 ; 41,9 ; 42,2 ; 43,4 ; 45,3 ; 52,0 ; 56,5 ; 65,1 ; 68,3 ; 68,9 ; 105,2 ; 132,8 ; 133,2 ; 147,2 ; 172,5.
**Masse (ESI+) :** *m*/*z* (%) 625 [M+K]⁺ (4), 609 [M+Na]⁺ (100).
**SMHR (ESI+)** : *m*/*z* calculée pour C₃₀H₅₀O₇NaS₂ 609,2896, trouvée 609,2892.

### Exemple 14: Préparation du (2S,4R,6R)- et (2R,4S,6S)-(4-Hydroxy-3,5-diméthoxy-11-oxo-1,7-dioxadispiro[5.1.5.2]pentadéca-9,12-dièn-2-yl)-acétoate de décyle (10'b) et (2S,4R,6S) et (2R,4S,6R)-(4-Hydroxy-3,5-diméthoxy-11-oxo-1,7-dioxadispiro[5.1.5.2]pentadéca-9,12-dièn-2-yl)-acétoate de décyle (11'b)

A une solution de diol syn **8'b** (180 mg, 0,30 mmol) dans 6,6 mL d'un mélange acétone/eau (9:1) est ajouté, dans le noir et en une fois, le PIFA (422 mg, 0,98 mmol). Après 15 minutes d'agitation à température ambiante, le milieu réactionnel est quenché par ajout d'une solution saturée de NaHCO₃ avant d'être extrait à l'AcOEt. Les phases organiques sont rassemblées, séchées sur MgSO₄ et concentrées sous vide. La purification est effectuée sur gel de silice (cyclohexane/AcOEt 4:6 à 3:7) pour donner les produits attendus **10'b** (39 mg, 80 µmol, 26 %) et **11'b** (17 mg, 34 µmol, 11 %) sous forme d'huiles incolores. 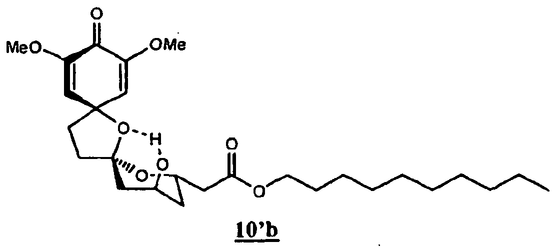 C₂₇H₄₂O₈
M = 494,6 g.mol⁻¹
**RMN ¹H (400 MHz, CDCl₃)** : δ = 0,88 (t, *J* = 6,4 Hz, 3H) ; 1,20-1,38 (m, 14H) ; 1,47-1,56 (m, 1H) ; 1,58-1,69 (m, 2H) ; 1,78-1,85 (m, 1H) ; 1,91-2,06 (m, 4H) ; 2,20 (dd, *J* **=** 11,2, 7,2 Hz, 1H) ; 2,36-2,54 (m, 3H) ; 3,67 (s, 3H) ; 3,81 (s, 3H) ; 4,02-4,13 (m, 2H) ; 4,14-4,18 (m, 1H) ; 4,63-4,72 (m, 1H) ; 5,66 (d, *J* = 2,2 Hz, 1H) ; 6,32 (d, *J* = 2,2 Hz, 1H).
**RMN ¹³C (100 MHz, CDCl₃)** : δ = 14,3 ; 22,9 ; 26,2 ; 28,9 ; 29,5-29,9 ; 32,1 ; 36,0 ; 37,8 ; . 39,0 ; 39,2 ; 40,9 ; 56,2 ; 55,5 ; 62,7 ; 64,8 ; 64,9 ; 82,0 ; 108,2 ; 116,5 ; 120,0 ; 148,9 ; 149,4 ; 171,5 ; 176,9.
**SMHR (ESI+)** : *m*/*z* calculée pour C₂₇H₄₂O₈Na 517,2777, trouvée 517,2767. 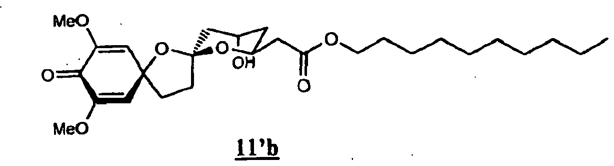 C₂₇H₄₂O₈
M = 494,6 g.mol⁻¹
**RMN ¹H (400 MHz, CDCl₃) :** δ = 0,88 (t, *J* = 6,8 Hz, 3H, 3xH10") ; 1,21-1,38 (m, 14H, 2xH3", 2xH4", 2xH5", 2xH6", 2xH7", 2xH8", 2xH9") ; 1,56-1,72 (m, 4H, 2xH2", H3a, H3b) ; 1,91 (dd, *J* = 14,0, 3,6 Hz, 1H, H5a) ; 1,99-2,14 (m, 3H, H15a, H5b, H14a) ; 2,32-2,43 (m, 1H, H14b) ; 2,50 (dd, *J*= 15,4, 4,0 Hz, 1H, H2'a) ; 2,65 (dd, *J* = 15,4, 9,2 Hz, 1H, H2'b) ; 2,74-2,81 (m, 1H, H15b); 3,67 (s, 3H, -OC*H*₃) ; 3,68 (s, 3H, -OC*H*₃) ; 4,02-4,14 (m, 2H, 2xH1") ; 4,38-4,49 (m, 2H, H4, H2) ; 5,68 (d, *J* = 2,4, 1H, H9) ; 5,94 (d, *J* = 2,4 Hz, 1H, H13).
**RMN ¹³C (100 MHz, CDCl₃) :** δ = 14,3 ; 22,9 ; 26,2 ; 28,9 ; 29,5-29,7 ; 32,1 ; 35,9 ; 37,4 ; 40,7; 41,2; 55,4; 65,0; 65,1; 66,6; 79,7 ; 108,2; 116,8 ; 119,7; 149,0; 149,3; 171,4; 176,2.
**SMHR (ESI+)** : *m*/*z* calculée pour C₂₇H₄₂O₈Na 517,2777, trouvée 517,2775.

### Exemple 15: Préparation du (5R)- et (5S)-5-Hydroxy-6-{2-[2-(4-hydroxyphényl)-éthyl]-[1,3]-dithian-2-yl)-3-oxohexanoate de octadécyle (6c)

A une solution de dérivé phénolique 5 (500 mg, 1,18 mmol) dans 16 mL de toluène anhydre est ajouté le 1-octadécanol commercial (956 mg, 3,53 mmol). Le milieu réactionnel est porté 6 h à 110°C avant d'être concentré sous vide. La purification est effectuée sur colonne de silice (cyclohexane/AcOEt 8:2 à 7:3) pour donner le mélange racémique du β-cétoester (480 mg, 0,75 mmol, 64 %) sous forme d'une huile incolore. Ce produit est en fait un mélange de deux composés en équilibre : le β-cétoester et sa forme énolique correspondante dans un rapport 9:1. 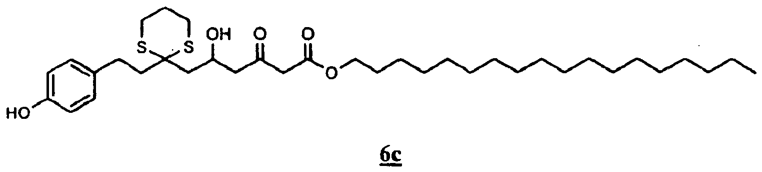 C₃₆H₆₀O₅S₂
M = 637,0 g.mol⁻¹
**RMN ¹H (400 MHz, CDCl₃)** : δ = 0,88 (t, *J* = 6,6 Hz, 3H) ; 1,23-1,33 (m, 30H) ; 1,59-1,68 (m, 2H) ; 1,88-2,07 (m, 3H) ; 2,14-2,27 (m, 2H) ; 2,34 (dd, *J* = 15,0, 8,8 Hz, 1H) ; 2,61 (dd, *J* = 17,2, 4,6 Hz, 1 H) ; 2,69-2,99 (m, 7H) ; 3,50 (d, *J* = 2,0 Hz, 2H) ; 4,13 (t, *J* = 6,8 Hz, 2H) ; 4,44-4,52 (m, 1H) ; 6,75 (d, *J* = 8,6 Hz, 2H) ; 7,06 (d, *J* = 8,6 Hz, 2H).
**RMN ¹³C (100 MHz, CDCl₃)** : δ = 14,1 ; 22,7 ; 24,9 ; 25,8 ; 26,0 ; 26,3 ; 28,4 ; 29,2-29,7 ; 31,9 ; 41,7 ; 43,8 ; 49,9 ; 50,2 ; 51,8 ; 65,0 ; 65,8 ; 115,4 ; 129,6 ; 133,4 ; 154,0 ; 167,4 ; 202,2.
**Masse (FAB+, NBA)** : *m*/*z* (%) 659 [M+Na]⁺ (6), 636 [M]⁺ (4), 511 (74), 239 (100).
**SMHR (IE)** : *m*/*z* calculée pour C₃₆H₆₀O₅S₂ 636,3882, trouvée 636,3845.

### Exemple 16: Préparation du (3S,5R)- et (3R,5S)-3,5-Dihydroxy-6-{2-[2-(4-hydroxyphényl)-éthyl]-[1,31-dithian-2-yl}-hexanoate de octadécyle (8c)

A une solution de cétone **6c** (450 mg, 0,71 mmol) dans 4,0 mL de THF anhydre sont ajoutés, à température ambiante et sous argon, 1,48 mL d'une solution commerciale 1M de BEt₃ dans l'hexane puis 1,7 mL de MeOH anhydre. Après 1 h d'agitation à température ambiante, le milieu réactionnel est refroidi à -78°C puis NaBH₄ (56 mg, 1,48 mmol) est ajouté en une fois. Après 2 h d'agitation, le milieu réactionnel est quenché à -78°C par ajout d'un tampon méthanolique (pH = 7, AcONa/AcOH). Après une nuit d'agitation à température ambiante, le milieu réactionnel est concentré, repris par une solution aqueuse saturée de K₂CO₃ puis extrait à l'AcOEt. Les phases organiques sont alors rassemblées, séchées sur MgSO₄, filtrées et concentrées sous vide. Le résidu est alors repris par du MeOH et agité 3 jours à température ambiante. Après évaporation du MeOH, la purification est effectuée sur gel de silice (cyclohexane/AcOEt 9:1 à 7:3) pour donner le mélange racémique du diol de configuration syn (257 mg, 0,40 mmol, 57 %) sous forme d'une huile incolore. 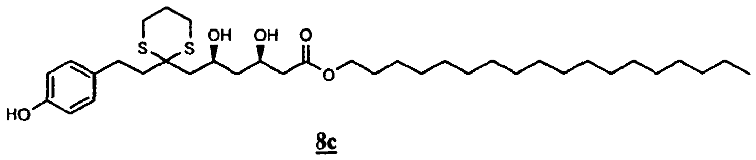 C₃₆H₆₂O₅S₂
M = 639,0 g.mol⁻¹
**RMN ¹H (400 MHz, CDCl₃)** : δ = 0,88 (t, *J* = 6,6 Hz, 3H) ; 1,22-1,35 (m, 30H) ; 1,45-1,51 (m, 1H) ; 1,57-1,66 (m, 2H) ; 1,68-1,75 (m, 1H) ; 1,90-2,06 (m, 3H) ; 2,11-2,31 (m, 2H) ; 2,36 (dd, *J* = 15,2, 8,8 Hz, 1H) ; 2,47 (dd, *J* = 16,0, 4,8 Hz, 1H); 2,53 (dd, *J* = 16,0, 7,8 Hz, 1H) ; 2,62-2,71 (m, 1H) ; 2,73-3,01 (m, 5H) ; 4,09 (t, *J* = 7,0 Hz, 2H) ; 4,23-4,40 (m, 2H) ; 6,77 *(d, J=* 8,6 Hz, 2H) ; 7,04 (d, *J* = 8,6 Hz, 2H).
**RMN ¹³C (100 MHz, CDCl₃)** : δ = 14,1 ; 22,7 ; 24,8 ; 25,9 ; 26,0 ; 26,3 ; 28,5 ; 29,2-29,7 ; 31,9 ; 41,8 ; 41,9 ; 43,0 ; 44,9 ; 51,8 ; 65,0 ; 68,2 ; 68,9 ; 115,5 ; 129,5 ; 133,1 ; 154,3 ; 172,5. **Masse (ESI+)** : *m*/*z* (%) 677 [M+K]⁺ (7), 661 [M+Na]⁺ (100).
**SMHR (ESI+)** : *m*/*z* calculée pour C₃₆H₆₂O₅NaS₂ 661,3936, trouvée 661,3941.

### Exemple 17: Préparation du (2S,4R,6R)- et (2R,4S,6S)-(4-Hydroxy-11-oxo-1,7-dioxadispiro[5.1.5.2]pentadéca-9,12-dién-2-yl)-acétoate de octadécyle (10c) et (2S,4R,6S)- et (2R,4S,6R)-(4-Hydroxy-11-oxo-1,7-dioxadispiro(5.1.5.2]pentadéca-9,12 dièn-2-yl)-acétoate de octadécyle (11c)

A une solution de diol syn **8c** (180 mg, 0,28 mmol) dans 10 mL d'un mélange acétone/eau (9:1) est ajouté, en une fois, le PIFA (388 mg, 0,90 mmol). Après 15 minutes d'agitation à température ambiante, le milieu réactionnel est quenché par ajout d'une solution saturée de NaHCO₃ avant d'être extrait à l'AcOEt. Les phases organiques sont rassemblées, séchées sur MgSO₄ et concentrées sous vide. La purification est effectuée sur gel de silice (cyclohexane/AcOEt 8:2) pour donner les produits attendus **10c** (30 mg, 55 µmol, 19 %) et **11c** (22 mg, 40 µmol, 14 %) sous forme d'huiles incolores. 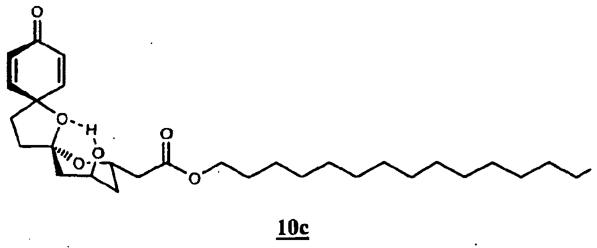 C₃₃H₅₄O6
M = 546,8 g.mol⁻¹
**RMN ¹H (400 MHz, CDCl₃)** : δ = 0,88 (t, *J* = 6,8 Hz, 3H) ; 1,22-1,39 (m, 30H) ; 1,51 (ddd, *J* = 13,6, 12,0, 2,8 Hz, 1H) ; 1,60-1,68 (m, 2H) ; 1,80-1,86 (m, 1H) ; 1,91-2,05 (m, 4H) ; 2,16-2,24 (m, 1H); 2,29-2,38 (m, 1H); 2,42-2,55 (m, 2H); 4,03-4,10 (m, 1H) ; 4,11-4,20 (m, 2H) ; 4,60-4,67 (m, 1H) ; 6,10 (dd, *J* = 10,0, 2,0 Hz, 1H) ; 6,19 (dd, *J* = 10,0, 2,0 Hz, 1H) ; 6,76 (dd, *J* = 10,0, 3,2 Hz, 1H) ; 7,16 (dd, *J* = 10,0, 3,2 Hz, 1H).
**RMN ¹³C (100 MHz, CDCl₃)** : δ = 14,3 ; 22,9 ; 26,1 ; 28,9 ; 29,5 ; 29,6-29,9 ; 32,1 ; 34,2 ; 37,7 ; 38,8 ; 39,1 ; 41,0 ; 62,7 ; 64,7 ; 65,1 ; 80,3 ; 109,0 ; 127,3 ; 127,7 ; 148,6 ; 151,6 ; 171,4 ; 185,5.
**Masse (ESI+)** : *m*/*z* (%) 569 [M+Na]⁺ (100), 529 [M+H-H₂O]⁺ (4), 511 (20).
**SMHR (IE)** : *m*/*z* calculée pour C₃₃H₅₄O₆ 546,3920, trouvée 546,3901. 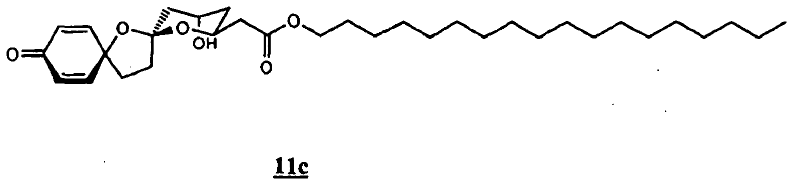 C₃₃H₅₄O₆
M = 546,8 g.mol⁻¹
**RMN ¹H (400 MHz, CDCl₃)** : δ = 0,88 (t, *J* = 7,0 Hz, 3H) ; 1,22-1,40 (m, 30H) ; 1,58-1,72 (m, 4H) ; 1,87-2,13 (m, 4H) ; 2,19-2,37 (m, 1H) ; 2,44-2,57 (m, 1H) ; 2,64 (dd, *J* = 15,2, 9,2 Hz, 1H) ; 2,72-2,79 (m, 1H) ; 4,03-4,18 (m, 2H) ; 4,37-4,47 (m, 2H) ; 6,11 (dd, *J =* 10,0, 3,0 Hz, 1H) ; 6,11 (dd, *J* = 10,0, 3,0 Hz, 1H) ; 6,76 (dd, *J* = 10,0, 3,0 Hz, 1H) ; 6,90 (dd, *J* = 10,0, 3,0 Hz, 1H).
**RMN ¹³C (100 MHz, CDCl₃)** : δ = 14,3 ; 22,9 ; 26,2 ; 28,9 ; 29,5 ; 29,6-29,9 ; 32,1 ; 35,4 ; 35,7 ; 37,4 ; 40,5 ; 41,1 ; 65,0 ; 65,1 ; 67,0 ; 78,1 ; 108,8 ; 127,5 ; 149,2 ; 152,1 ; 171,2 ; 185,8.
**Masse (ESI+)** *: m*/*z* (%) 569 [M+Na]⁺ (100), 529 [M+H-H₂O]⁺ (20), 511 (38).
**SMHR (IE)** : *m*/*z* calculée pour C₁₃H₅₄O₆ 546,3920, trouvée 546,3922.

### Exemple 18: Préparation du (3S,5S)- et (3R,5R)-3,5-Dibydroxy-6-(2-12-(4-hydroxyphényl)-éthyl]-[1,3]-dithian-2-yl}-bexanoate de octadécyle (9c)

A une suspension de Me₄NBH(OAc)₃ (330 mg, 1,26 mmol) dans 3,75 mL d'un mélange CH₃CN/AcOH (4:1) est ajoutée goutte à goutte, à 0°C, une solution de cétone **6c** (200 mg, 0,31 mmol) dans 2,25 mL d'un mélange CH₃CN/CH₂Cl₂ (2:1). Après 3 h d'agitation à 0°C, le milieu réactionnel est dilué dans l'AcOEt puis versé dans 20 mL d'une solution aqueuse saturée de tartrate mixte de sodium et potassium. Après filtration du précipité formé sur coton, les phases sont séparées et la phase aqueuse est de nouveau extraite à l'AcOEt. Les phases organiques sont alors rassemblées, séchées sur MgSO₄, filtrées et concentrées sous vide. La purification est effectuée sur gel de silice (cyclohexane/AcOEt 8:2) pour donner le mélange racémique du diol de configuration anti (169 mg, 0,26 mmol, 84 %) sous forme d'une huile incolore.
Mélange syn/anti 2:8. 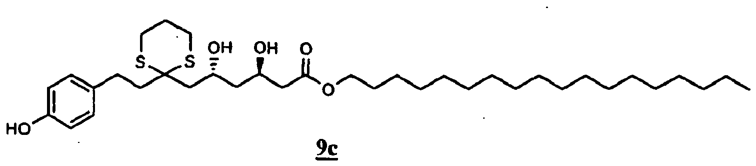 C₃₆H₆₂O₅S₂
M = 639,0 g.mol⁻¹
**RMN ¹H (400 MHz, CDCl₃)** : δ = 0,88 (t, *J* = 6,8 Hz, 3H) ; 1,23-1,35 (m, 30H) ; 1,58-1,74 (m, 4H) ; 1,86-2,10 (m, 3H) ; 2,09-2,30 (m, 2H) ; 2,32-2,55 (m, 3H); 2,56-3,01 (m, 6H) ; 4,09 (t, *J* = 6,8 Hz, 2H) ; 4,33-4,43 (m, 2H) ; 6,74 (d, *J* = 8,6 Hz, 2H) ; 7,04 (d, *J* = 8,6 Hz, 2H).
**RMN ¹³C (100 MHz, CDCl₃)** : δ = 14,3 ; 22,8 ; 25,0 ; 26,0 ; 26,2 ; 26,4 ; 28,7 ; 29,4-29,8 ; 32,1 ; 41,7; 42,1 43,2 ; 44,8 ; 52,1 ; 65,2 ; 65,7 ; 66,1 ; 115,6 ; 129,6 ; 133,1 ; 154,5 ; 173,0.
**Masse (ESI+)** : *mlz* (%) 661 [M+Na]⁺ (88), 639 [M+H]⁺ (3), 107 (100).
**SMHR (ESI+)** : *m*/*z* calculée pour C₃₆H₆₂O₅NaS₂ 661,3936, trouvée 661,3931.

### Exemple 19: Préparation du (2S,4S,6R)- et (2R,4R,6S)-(4-Hydroxy-11-oxo-1,7-dioxadispiro(5.1.5.2]pentadéca-9,12-dièn-2-yl)-acétoate de octadécyle (12c) et (2S,4S,6S)- et (2R,4R,6R)-(4-Hydroxy-11-oxo-1,7-dioxadispiro[5.1.5.2]pentadéca-9,12-dièn-2-yl)-acétoate de octadécyle (13c)

A une solution de diol anti **9c** (150 mg, 0,23 mmol) dans 10 mL d'un mélange acétone/eau (9:1) est ajouté, en une fois, le PIFA (323 mg, 0,75 mmol). Après 10 minutes d'agitation à température ambiante, le milieu réactionnel est quenché par ajout d'une solution saturée de NaHCO₃ avant d'être extrait à l'AcOEt. Les phases organiques sont rassemblées, séchées sur MgSO₄ et concentrées sous vide. La purification est effectuée sur gel de silice (cyclohexane/AcOEt 8:2) pour donner les produits attendus **12c** (20 mg, 37 µmol, 16 %) et **13c** (20 mg, 37 µmol, 16 %) sous forme d'huiles incolores. 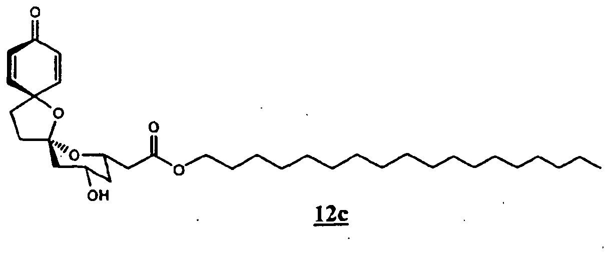 C₃₃H₅₄O₆
M = 546,8 g.mol⁻¹
**RMN ¹H (400 MHz, CDCl₃)** : δ = 0,89 (t, *J* = 6,8 Hz, 3H) ; 1,23-1,39 (m, 31H) ; 1,52-1,80 (m, 3H) ; 1,95-2,13 (m, 4H) ; 2,17-2,26 (m, 1H) ; 2,29-2,38 (m, 1H) ; 2,43-2,58 (m, 2H) ; 4,04-4,21 (m, 3H) ; 4,29-4,36 (m, 1 H) ; 6,11 (dd, *J* = 10,0, 2,0 Hz, 1 H) ; 6,17 (dd, *J* = 10,0, 2,0 Hz, 1H) ; 6,77 (dd, *J* = 10,0, 3,0 Hz, 1H) ; 7,09 (dd, *J* = 10,0, 3,0 Hz, 1H).
**RMN ¹³C (100 MHz, CDCl₃)** : δ = 14,4 ; 22,9 ; 26,1 ; 28,9 ; 29,5-29,9 ; 32,1 ; 34,7 ; 38,8 ; 40,2 ; 41,1 ; 42,7 ; 65,1 ; 66,0 ; 79,4 ; 109,0 ; 127,1 ; 127,4 ; 149,2 ; 152,2 ; 171,4 ; 185,8. **SMHR (IE)** : *m*/*z* calculée pour C₃₃H₅₄O₆ 546,3920, trouvée 546,3912. 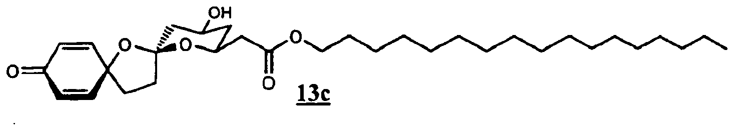 C₃₃H₅₄O₆
M = 546,8 g.mol⁻¹
**RMN ¹H (400 MHz, CDCl₃)** : δ = 0,89 (t, *J* = 6,8 Hz, 3H) ; 1,23-1,38 (m, 31H) ; 1,58-1,73 (m, 2H) ; 1,78-1,91 (m, 2H) ; 1,99-2,10 (m, 2H) ; 2,15 (ddd, *J* = 12,4, 4,4 Hz, 1,2 Hz, 1H) ; 2,25-2,35 (m, 1H) ; 2,46-2,55 (m, 2H) ; 2,67 (dd, *J* = 15,6, 8,8 Hz, 1H) ; 3,88-3,97 (m, 2H) ; 4,05-4,16 (m, 2H) ; 6,11 (dd, *J* = 10,0, 2,0 Hz, 1 H) ; 6,14 (dd, *J* = 10,0, 2,0 Hz, 1H) ; 6,78 (dd, *J* = 10,0, 3,0 Hz, 1H) ; 6,91 (dd, *J* = 10,0, 3,0 Hz, 1H).
**RMN ¹³C (100 MHz, CDCl₃)** : δ = 14,4 ; 22,9 ; 26,2 ; 28,9 ; 29,5 ; 29,6-29,9 ; 32,2 ; 33,6 ; 35,0 ; 40,0 ; 41,0 ; 43,3 ; 65,2 ; 66,8 ; 68,5 ; 78,5 ; 109,4 ; 127,4 ; 127,7 ; 148,8 ; 151,5; 171,4 ; 185,6.
**SMHR (ESI+)** : *m*/*z* calculée pour C₃₃H₅₄O₆Na 569,3818; trouvée 569,3799.

### Exemple 20: Préparation du (5R)- et (5S)-5-Hydroxy-6-{2-[2-(4-hydroxyphényl)-éthyl]-[1,3]-dithian-2-yl)-3-oxohexanoate de 1-décylundécyle (6d)

A une solution de dérivé phénolique 5 (500 mg, 1,18 mmol) dans 15 mL de toluène anhydre est ajouté le 11-hénéicosanol commercial (736 mg, 2,36 mmol). Le milieu réactionnel est porté 9 h à 110°C avant d'être concentré sous vide. La purification est effectuée sur gel de silice (cyclohexane/AcOEt 8:2) pour donner le mélange racémique du β-cétoester (279 mg, 0,41 mmol, 35 %) sous forme d'une huile incolore. Ce produit est en fait un mélange de deux composés en équilibre : le β-cétoester et sa forme énolique correspondante dans un rapport 88:12. 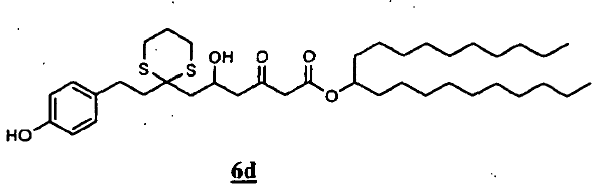 C₃₉H₆₆O₅S₂
M = 679,1 g.mol⁻¹
**RMN ¹H (400 MHz, CDCl₃)** : δ = 0,88 (t, *J* = 7,0 Hz, 6H) ; 1,20-1,34 (m, 32H) ; 1,50-1,58 (m, 4H) ; 1,88-2,08 (m, 3H) ; 2,15-2,31 (m, 2H) ; 2,33 (dd, *J* = 15,2, 8,8 Hz, 1H) ; 2,59 (dd, *J* = 17,2, 4,4 Hz, 1H) ; 2,68-2,99 (m, 7H) ; 3,48 (d, *J =* 4,4 Hz, 2H) ; 4,44-4,52 (m, 1H) ; 4,92 (quint., *J* = 6,2 Hz, 1H) ; 6,75(d, *J* = 8,4Hz, 2H) ; 7,06(d, *J* = 8,4Hz, 2H).
**RMN ¹³C (100 MHz, CDCl₃)** : δ = 14,3 ; 22,9 ; 25,1 ; 25,5 ; 26,2 ; 26,5 ; 29,5-29,8 ; 32,1 ; 34,1 ; 41,9 ; 44,0 ; 50,4 ; 50,5 ; 52,1 ; 65,2 ; 76,4; 115,6 ; 129,8 ; 133,7 ; 154,2 ; 167,3 ; 202,5.
**Masse (ESI+)** : *m*/*z* (%) 701 [M+Na]⁺ (100), 679 [M+H]⁺ (11).
**SMHR (IE)** : *m*/*z* calculée pour C₃₉H₆₆O₅S₂ 678,4352, trouvée 678,4365.

### Exemple 21: Préparation du (3S,5R)- et (3R,5S)-3,5-Dihydroxy-6-{2-[2-(4-hydroxyphényl)-éthyl]-[1,3]-dithian-2-yl}-hexanoate de 1-décylundécyle (8d)

A une solution de cétone **6d** (250 mg, 0,37 mmol) dans 2,6 mL de THF anhydre sont ajoutés, à température ambiante et sous argon, 0,77 mL d'une solution commerciale 1M de BEt₃ dans l'hexane puis 1,0 mL de MeOH anhydre. Après 1 h d'agitation à température ambiante, le milieu réactionnel est refroidi à -78°C puis NaBH₄ (37 mg, 0,98 mmol) est ajouté en une fois. Après 2 h d'agitation, le milieu rédactionnel est quenché à -78°C par ajout d'un tampon méthanolique (pH = 7, AcONa/AcOH). Après une nuit d'agitation à température ambiante, le milieu réactionnel est concentré, repris par une solution aqueuse saturée de K₂CO₃ puis extrait à l'AcOEt. Les phases organiques sont alors rassemblées, séchées sur MgSO₄, filtrées et concentrées sous vide. Le résidu est alors repris par du MeOH et agité une nuit à température ambiante. Après évaporation du MeOH, la purification est effectuée sur gel de silice (cyclohexane/AcOEt 8:2) pour donner le mélange racémique du diol de configuration syn (249 mg, 0,37 mmol, 99 %) sous forme d'une huile incolore. 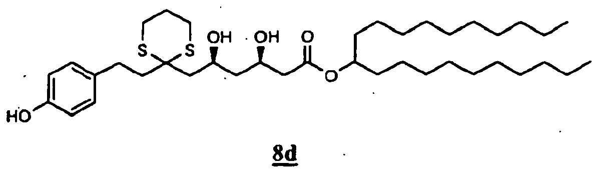 C₃₉H₆₈O₅S₂
M = 681,1 g.mol⁻¹
**RMN ¹H (400 MHz, CDCl₃)** : δ = 0,88 (t, *J* = 7,0 Hz, 6H) ; 1,19-1,34 (m, 32H) ; 1,47-1,56 (m, 5H) ; 1,67-1,76 (m, 1H) ; 1,89-2,08 (m, 3H) ; 2,12-2,31 (m, 2H) ; 2,36 (dd, *J* = 15,2, 8,8 Hz, 1H) ; 2,46 (dd, *J* = 15,8, 4,8 Hz, 1H) ; 2,53 (dd, *J* = 15,8, 7,6 Hz, 1H) ; 2,62-2,71 (m, 1H) ; 2,74-2,99 (m, 5H) ; 4,25-4,34 (m, 2H) ; 4,91 (quint., *J* = 6,0 Hz, 1H) ; 6,76 (d, *J* = 8,4 Hz, 2H) ; 7,04 (d, *J* = 8,4 Hz, 2H).
**RMN ¹³C (100 MHz, CDCl₃)** : δ = 14,3 ; 22,9 ; 25,1 ; 25,5 ; 26,2 ; 26,5 ; 29,5-29,9 ; 32,0 ; 34,2 ; 42,1 ; 43,2 ; 45,1 ; 52,1 ; 68,5 ; 69,1 ; 75,4 ; 115,7 ; 129,7 ; 133,4 ; 154,4 ; 172,5.
**Masse (ESI+)** : *m*/*z* (%) 703 [M+Na]⁺ (100), 681 [M+H]⁺ (6).
**SMHR (IE)** : *m*/*z* calculée pour C₃₉H₆₈O₅S₂ 680,4508, trouvée 680,4531.

### Exemple 22: Préparation du (2S,4R,6R)- et (2R,4S,6S)-(4-Hydroxy-11-oxo-1,7-dioxadispiro[5.1.5.2]pentadéca-9,12-dièn-2-yl)-acétoate de 1-décylundécyle (10d) et (2S,4R,6S)- et (2R,4S,6R)-(4-Hydroxy-11-oxo-1,7-dioxadispiro[5.1.5.2]pentadéca-9,12-dièn-2-yl)-acétoate de 1-décylundécyle (11d)

A une solution de diol syn **8d** (230 mg, 0,34 mmol) dans 12,2 mL d'un mélange acétone/eau (9:1) est ajouté, dans le noir et en une fois, le PIFA (465 mg, 1,08 mmol). Après 15 minutes d'agitation à température ambiante, le milieu réactionnel est quenché par ajout d'une solution saturée de NaHCO₃ avant d'être extrait à l'AcOEt. Les phases organiques sont rassemblées, séchées sur MgSO₄ et concentrées sous vide. La purification est effectuée sur gel de silice (cyclohexane/AcOEt 9:1) pour donner les produits attendus **10d** (24 mg, 41 µmol, 12 %) et **11d** (22 mg, 37 µmol, 11 %) sous forme d'huiles incolores. 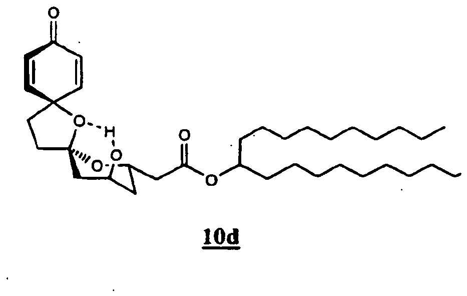 C₃₆H₆₀O₆
M = 588,9 g.mol⁻¹
**RMN ¹H (400 MHz, CDCl₃)** : δ = 0,87 (t, *J* = 7,2 Hz, 3H) ; 0,88 (t, *J* = 6,8 Hz, 3H) ; 1,20-1,36 (m, 32H) ; 1,48-1,56 (m, 5H) ; 1,79-1,86 (m, 1H) ; 1,91-2,05 (m, 4H) ; 2,14-2,22 (m, 1H) ; 2,28-2,36 (m, 1H) ; 2,45-2,49 (m, 2H) ; 4,12-4,19 (m, 1H) ; 4,61-4,69 (m, 1H) ; 4,93 (quint., 1H) ; 6,10 (dd, *J* = 10,2, 2,0 Hz, 1H) ; 6,20 (dd, *J* = 10,0, 2,0 Hz, 1H) ;6,75 (dd, *J* = 10,2, 2,8 Hz, 1H) ; 7,16 (dd, *J* = 10,0, 2,8 Hz, 1H).
**RMN ¹³C (100 MHz, CDCl₃)** : δ = 14,3 ; 22,9 ; 25,6 ; 29,5 ; 29,8 ; 32,1 ; 34,2 ; 34,5 ; 37,6 ; 38,9 ; 39,1 ; 41,2 ; 62,8 ; 64,8 ; 75,0 ; 80,3 ; 109,1 ; 127,3 ; 127,7 ; 148,6 ; 151,7 ; 171,2 ; 185,6.
**Masse (ESI+)** : *m*/*z* (%) 611 [M+Na]⁺ (100), 571 [M+H-H₂O]⁺ (2).
**SMHR (IE)** : *m*/*z* calculée pour C₃₆H₆O₆ 588,4390, trouvée 588,4380. 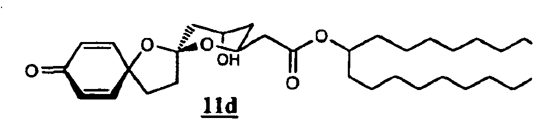 C₃₆H₆₀O₆
M = 588,9 g.mol⁻¹
**RMN ¹H (400 MHz, CDCl₃)** : δ = 0,88 (t, *J* = 6,8 Hz, 6H) ; 1,20-1,34 (m, 32H) ; 1,48-1,57 (m, 4H) ; 1,60-1,67 (m, 2H) ; 1,87-2,1 (m, 4H) ; 2,31-2,40 (m, 1H) ; 2,45 (dd, *J* = 15,8, 4,2 Hz, 1H) ; 2,63 (dd, *J* = 15,8, 9,0 Hz, 1H) ; 2,73-2,80 (m, 1H) ; 4,36-4,48 (m, 2H) ; 4,89 (quint., *J* = 6,4 Hz, 1H) ; 6,09 (dd, *J* = 10,2, 2,0 Hz, 1H) ; 6,11 (dd, *J* = 10,2, 2,0 Hz, 1H) ; 6,76 (dd, *J* = 10,2, 3,1 Hz, 1H) ; 6,92 (dd, *J* = 10,2, 3,1 Hz, 1H).
**RMN ¹³C (100 MHz, CDCl3)** : δ = 14,3 ; 22,9 ; 25,6 ; 29,3 ; 29,7-29,8 ; 32,1 ; 34,3 ; 35,4 ; 35,7 ; 37,3 ; 40,5 ; 41,2 ; 64,9 ; 66,7 ; 75,1 ; 78,1 ; 108,8 ; 127,3 ; 127,4 ; 149,2 ; 152,2 ; 171,2 ; 185,8.
**Masse (ESI+)** : *m*/*z* (%) 611 [M+Na]⁺ (100), 571 [M+H-H₂O]⁺ (3).
**SMHR (ESI+)** : *m*/*z* calculée pour C₃₆H₆₀O₆Na 611,4288, trouvée 611,4297.

### Exemple 23: Préparation de l'Acide 5-Hydroxy-6-{2-[2-(4-hydroxyphényl)-éthyl]-[1,3]-dithian-2-yl}-3-oxohexanoïque dihexylamide (6e)

A une solution de dérivé phénolique 5 (500 mg, 1,18 mmol) dans 15 mL de toluène anhydre est ajoutée la di-n-hexylamine (655 mg, 3,53 mmol), puis le milieu réactionnel est porté à 110°C. Après 6 h d'agitation à cette température, le milieu réactionnel est concentré sous vide. La purification est effectuée sur colonne de silice (cyclohexane/AcOEt95:5 jusque 85:15) pour donner l'amide attendue (293 mg, 0,53 mmol, 45 %) sous forme d'une huile incolore. Ce composé est en fait un mélange de deux composés en équilibre, le composé β-cétoamide et sa forme énolique correspondante dans un rapport 8:2. 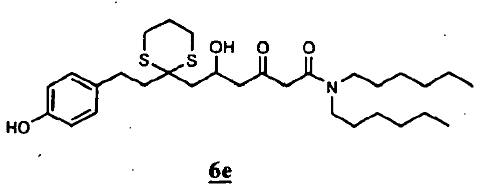 C₃₀H₄₉NO₄S₂
M = 551,9 g.mol⁻¹
**RMN ¹H (400 MHz, CDCl₃)** : δ = 0,84 (2t, J = 6,8 Hz, 3H, 3xH6') ; 0,93 (2t, J = 6,8 Hz, 3H, 3xH6') ; 1,23-1,35 (m, 12H, 4xH3', 4xH4', 4xH5') ; 1,48-1,60 (m, 4H, 4xH2') ; 1,85-2,07 (m, 3H, -SCH₂C*H*₂-, H6a) ; 2,10-2,50 (m, 4H, 2xH1", H6b, H4a) ; 2,64-3,01 (m, 7H, 2x H2", H4b, 2x-SC*H*₂-) ; 3,12-3,22 (m, 2H, 2xH1') ; 3,23-3,42 (m, 2H, 2xH1') ; 3,54 (s, 2H, 2xH2) ; 4,36-4,46 (m, 1 H, H5) ; 6,76 (d, *J* = 8,2 Hz, 2H, 2xH3"') ; 7,02 (d, *J* = 8,2 Hz, 2H, H2"').
**RMN ¹³C (100 MHz, CDCl₃)** : δ = 14,1 ; 22,7 ; 25,1 ; 26,2 ; 26,5 ; 26,6-29,7 ; 31,6 ; 31,7 ; 41,4; 43,8 ; 46,4; 48,9; 49,4; 50,6; 52,2; 65,3; 115,9; 129,7; 133,1 ; 154,8; 166,9; 203,7.
**Masse (ESI+)** : *m*/*z* (%) 590 [M+K]⁺ (5), 574 [M+Na]⁺ (100).
**SMHR (ESI+)** : *m*/*z* calculée pour C₃₀H₄₉NO₄NaS₂ 574,3001, trouvée 574,2991.

### Exemple 24. Préparation de l'acide 3,5-dihydroxy-6-{2-[2-(4-hydroxyphényl)-éthyl]-[1,3]-dithian-2-yl}-hexanoïque dihexylamide (7e)

A une suspension de NaBH₄ (50 mg, 1,32 mmol) dans 9 mL de MeOH est ajoutée à 0°C une solution de cétone **6e** (242 mg, 0,44 mmol) dans 6 mL de CH₂Cl₂. Après 3 h d'agitation à 0°C, le milieu réactionnel est quenché par ajout d'une solution aqueuse saturée de NH₄Cl. Après dilution du milieu réactionnel dans un mélange CH₂Cl₂ et H₂O, celui-ci est extrait au CH₂Cl₂. Les phases organiques sont alors rassemblées, séchées sur MgSO₄, filtrées et concentrées sous vide. La purification est effectuée sur colonne de silice (cyclohexane/AcOEt 8:2) pour donner le mélange de 4 diastéréoisomères non séparables de l'alcool attendu (173 mg, 0,03 mmol, 76 %) sous forme d'une huile jaune. 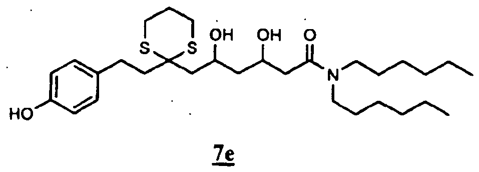 C₃₀H₅₁NO₄S₂
M = 553,9 g.mol⁻¹
**Masse (DCI, NH₃ + isobutane)** : *m*/*z* (%) 576 [M+Na]⁺ (18), 554 [M+H]⁺ (21), 428 (100).

### Exemple 25: Préparation du (2S,4R,6R)- et (2R,4S,6S)-N,N-dihexyl-2-(4-hydroxy-11-oxo-1,7-dioxadispiro[5.1.5.2]pentadéca-9,12-dièn-2-yl)acétamide (10e)

A une solution de diol **7e** (120 mg, 0,22 mmol) dans 5,5 mL d'un mélange acétone/eau (10:1) est ajouté, dans le noir et en une fois, le PIFA (298 mg, 0,69 mmol). Après 15 minutes d'agitation à température ambiante, le milieu réactionnel est quenché par ajout d'une solution saturée de NaHCO₃ avant d'être extrait à l'AcOEt. Les phases organiques sont rassemblées, séchées sur MgSO₄ et concentrées sous vide. La purification est effectuée sur gel de silice (CH₂Cl_{2/}MeOH 99:1) pour donner le produit majoritaire attendu (11 mg, 24 µmol, 11 %) sous forme d'huile incolore (les autres composés formés n'ont pas été isolés). 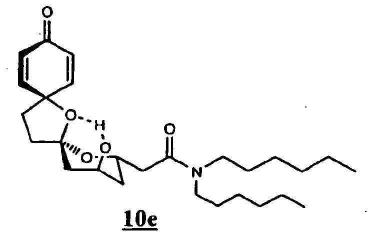 C₂₇H₄₃O₅N
M = 461,6 g.mol⁻¹
**RMN ¹H (400 MHz, CDCl₃)** : δ = 0,87 (t, *J* = 6,8 Hz, 3H) ; 0,90 (t, *J* = 6,8 Hz, 3H) ; 1,24-1,35 (m, 12H); 1,47-1,65 (m, 6H); 1,85-2,05 (m, 4H); 2,08-2,17 (m, 1H) ; 2,24-2,33 (m, 2H) ; 2,62 (dd, *J* = 14,8, 9,4 Hz, 1H) ; 3,13-3,23 (m, 2H) ; 3,30-3,38 (m, 1H) ; 3,43-3,51 (m, 1H) ; 4,13-4,18(m, 1H) ; 4,69-4,78 (m, 1H) ; 6,10(dd, *J* = 10,0, 1,8Hz, 1H) ; 6,19(dd, *J =* 10,0, 1,8 Hz, 1H) ; 6,75 (dd, *J* = 10,0, 3,0 Hz, 1H) ; 7,22 (dd, *J* = 10,0, 3,0 Hz, 1H).
**RMN ¹³C (100 MHz, CDCl₃)** : δ = 14,2 ; 22,8 ; 26,7 ; 26,9 ; 28,2 ; 29,5 ; 31,8 ; 34,2 ; 38,0 ; 38,8 ; 39,1 ; 39,2 ; 46,7; 48,6; 63,7; 64,9 ; 80,2; 109,0 ; 127,2; 127,5 ; 148,8 ; 152,2; 170,1 ; 185,7.
**Masse (ESI+)** : *m*/*z* (%) 500 [M+K]⁺ (6), 484 [M+Na]⁺ (100).
**SMHR (ESI+)** : *m*/*z* calculée pour C₂₇H₄₃NO₅Na 484,3039, trouvée 484,3040.

### Exemple 26: Préparation du (5R)- et (5S)-5-Hydroxy-1-(4-hydroxyphényl)-6-{2-[2-(4-hydroxyphényl)-éthyl]-[1,3]-dithian-2-yl}-hexan-3-one (3)

A une solution d'aldéhyde **1** (200 mg, 0,71 mmol) et d'énolate de triméthylsilyle **2** (546 mg, 1,77 mmol) dans 10 mL de CH₂Cl₂ anhydre, sont ajoutés, goutte à goutte, sous argon et à -78°C, 0,22 mL de BF₃.OEt₂. Après 2 h d'agitation à cette température, 2 x 0,5 éq. supplémentaires de silylénol éther sont ajoutés. Après 1h d'agitation, le milieu réactionnel est quenché par ajout de 10 mL d'une solution aqueuse de NaOH 1N à température ambiante. Après extraction à l'AcOEt, les phases organiques sont séchées sur MgSO₄, filtrées et concentrées sous vide. Le résidu est alors mis en solution dans CH₂Cl₂ puis 2 éq. de TBAF sont ajoutés à 0°C. Après 1 h d'agitation, une solution aqueuse saturée de NH₄Cl est ajoutée. Après extraction à l'AcOEt, les phases organiques sont séchées sur MgSO₄, filtrées et concentrées sous vide. La purification est effectuée sur colonne de silice (cyclohexane/AcOEt 7:3) pour donner le composé attendu (266 mg, 0,60 mmol, 84 %) sous forme d'une huile incolore. 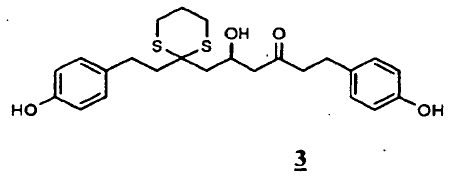 C₂₄H₃₀O₄S₂
M = 446,6 g.mol⁻¹
**RMN ¹H (400 MHz, MeOD)** : δ = 1,80-1,95 (m, 2H) ; 2,01-2,23 (m, 4H) ; 2,50-2,87 (m, 8H) ; 4,42 (s, 1H) ; 6,62-6,72 (m, 4H) ; 6,92-7,03 (m, 4H).
**RMN ¹³C (100 MHz, MeOD)** : δ = 26,4 ; 26,8 ; 27,0 ; 29,7 ; 30,7 ; 43,1 ; 45,4 ; 46,3 ; 52,3 ; 53,2 ; 66,4 ; 116,1 ; 130,2 ; 130,3 ; 133,1 ; 134,2 ; 156,3 ; 156,5 ; 211,4.
**Masse (IE)** : *m*/*z* (%) 446 [M]⁺ (1), 428 (4), 239 (100), 107 (70).
**SMHR (ESI+)** : *m*/*z* calculée pour C₂₄H₃₀O₄NaS₂ 469,1483, trouvée 469,1489.

### Exemple 27: Préparation du (8R,10S,21S)- et (8S,10R,21R)-21-Hydroxy-7,9,11-trioxatétraspiro[5.1.1.1.5¹².2¹⁰.3⁸.2⁶]tétracosa-1,4,13,16-dièn-3,15-one (4a) et (8R,10R,21S)-21-Hydroxy-7,9,11-trioxatétraspiro[5.1.1.1.5¹².2¹⁰.3⁸.2⁶]tétracosa-1,4,13,16-dièn-3,15-one (4b)

A une solution de dérivé phénolique **3** (217 mg, 0,49 mmol) dans 18 mL d'un mélange acétone/H₂O (9:1), est ajouté le PIFA (1,25 g, 2,92 mmol). Après une nuit d'agitation, le milieu réactionnel est quenché par ajout d'une solution aqueuse saturée de NaHCO₃. Après extraction à l'AcOEt, les phases organiques sont rassemblées, séchées sur MgSO₄, filtrées et concentrées sous vide. La purification est effectuée sur colonne de silice (CH₂Cl₂/MeOH 98:2) pour donner les diastéréoisomères séparables **4a** (35 mg, 0,09 mmol, 19 %) et **4b** (20 mg, 0,06 mmol, 11 %) sous forme d'huiles incolores. 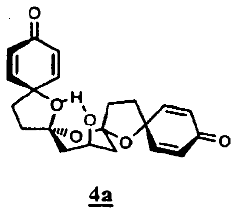 C₂₁H₂₂O₆
M = 370,4 g.mol⁻¹
**RMN ¹H (400 MHz, CDCl₃)** : δ = 1,91-2,42 (m, 10H) ; 2,49-2,56 (ddd, *J* = 12,4, 6,8, 2,0 Hz, 1H); 2,60-2,67 (m, 1H) ; 4,21-4,29 (m, 1H) ; 6,12-6,21 (m, 4H) ; 6,74-6,80 (m, 2H) ; 6,85 (dd, *J* = 10,0, 3,0 Hz, 1H) ; 6,91 (dd, *J* = 10,0, 3,0 Hz, 1H).
**RMN ¹³C (100 MHz, CDCl₃)** : δ = 35,0 ; 35,2 ; 38,6 ; 39,6 ; 41,8 ; 42,6 ; 63,6 ; 78,9 ; 109,0 ; 110,2; 127,3 ; 127,4 ; 127,7 ; 127,8 ; 148,4 ; 148,6 ; 150,3 ; 150,6 ; 185,2.
**Masse (DCI, NH3 + isobutane)** : *m*/*z* (%) 388 [M+NH₄]⁺ (7), 371 [M+H]⁺ (12), 353 (30), 316 (39), 288 (100).
**SMHR (IE)** : *m*/*z* calculée pour C₂₁H₂₂O₆ 370,1416, trouvée 370,1421. 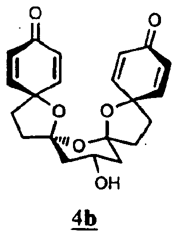 C₂₁H₂₂O₆
M = 370,4 g.mol⁻¹
**RMN ¹H (400 MHz, CDCl₃) :** δ = 1,77 (dd, *J* = 12,8, 10,4 Hz, 2H) ; 2,03-2,10 (m, 2H) ; 2,16-2,23 (m, 2H) ; 2,25-2,37 (m, 6H); 4,38-4,47 (m, 1H); 6,09-6,16 (m, 4H) ; 6,75 (dd, *J* = 10,2, 2,8 Hz, 2H, H1) ; 7,02 (dd, *J* = 10,2, 2,8 Hz, 2H).
**RMN ¹³C (100 MHz, CDCl₃)** : δ = 35,3 ; 40,4 ; 44,2 ; 62,0 ; 79,8 ; 110,1 ; 127,5 ; 127,7 ; 149,3 ; 150,6 ; 185,5.
**Masse (DCI, NH3 + isobutane)** : *m*/*z* (%) 388 [M+NH₄]⁺ (56), 371 [M+H]⁺ (37), 353 (99), 316 (43), 288 (89), 107 (100).
**SMHR (IE)** : *m*/*z* calculée pour C₂₁H₂₂O₆ 370,1416, trouvée 370,1423.

### Exemple 28: Préparation du (8R,10S,21S)- et (8S,10R,21R)-Heptanoate de 7,9,11-trioxatétraspiro[5.1.1.1.5¹².2¹⁰.3⁸.2⁶]tétracosa-1,4,13,16-dièn-3,15-on-21-yle (16a)

A une solution d'alcool **4a** (29,7 mg, 0,08 mmol) dans 2 mL de CH₂Cl₂ anhydre, sont ajoutés, à 0°C et sous argon, 1a DMAP (9,8 mg, 0,08 mmol), le chlorure d'heptanoyle (14,3 mg, 0,10 mmol) puis la triéthylamine (9,7 mg, 0,10 mmol). Après 0,5 h d'agitation à 0°C et 4 h à température ambiante, le milieu réactionnel est quenché par ajout d'une solution aqueuse 1N de HCl. Après extraction au CH₂Cl₂, les phases organiques sont rassemblées, séchées sur MgSO₄, filtrées et concentrées sous vide. La purification est effectuée sur gel de silice (cyclohexane/AcOEt 7:3) pour donner l'ester attendu (21,6 mg, 0,04 mmol, 56 %) sous forme d'huile incolore. 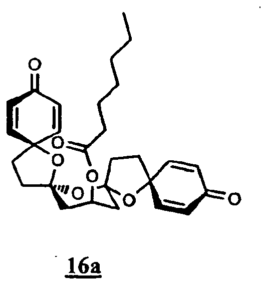 C₂₈H₃₄O₇
M = 482,6 g.mol⁻¹
**RMN ¹H (400 MHz, CDCl₃) :** δ = 0,88 (t, *J* = 6,2 Hz, 3H) ; 1,22-1,36 (m, 6H) ; 1,55- 1,65 (m, 2H) ; 1,94-2,38 (m, 12H) ; 2,52 (ddd, *J* = 12,8, 7,2, 3,6 Hz, 1H) 2,76-2,83 (m, 1H) ; 5,18-5,26 (m, 1H) ; 6,11-6,21 (m, 4H) ; 6,72-6,78 (m, 2H) ; 6,85 (dd, *J* = 10,0, 2,8 Hz, 1H) ; 6,92 (dd, *J* = 10,0, 2,8 Hz, 1H).
**RMN ¹³C (100 MHz, CDCl₃) :** δ = 14,2 ; 22,7 ; 25,1 ; 29,0 ; 31,6 ; 34,7 ; 35,5 ; 38,4 ; 39,8 ; 39,9 ; 66,1 ; 78,9 ; 79,6 ; 109,2 ; 110,1 ; 127,6 ; 127,8 ; 127,9 ; 128,2 148,8 ; 149,9 ; 151,0 ; 173,4 ; 185,3 ; 185,5.

### Exemple 29: Préparation du (8R,10R,21S)-Heptanoate de 7,9,11-trioxatétraspiro[5.1.1.1.5¹².2¹⁰.3⁸.2⁶]tétracosa-1,4,13,16-dièn-3,15-on-21-yle (16b)

A une solution d'alcool **4b** (17 mg, 0,05 mmol) dans 1 mL de CH₂Cl₂ anhydre, sont ajoutés, à 0°C et sous argon, la DMAP (6 mg, 0,05 mmol), le chlorure d'heptanoyle (8 mg, 0,06 mmol) puis la triéthylamine (6 mg, 0,06 mmol). Après 0,5 h d'agitation à 0°C et 4 h à température ambiante, le milieu réactionnel est quenché par ajout d'une solution aqueuse 1N de HCl. Après extraction au CH₂Cl₂, les phases organiques sont rassemblées, séchées sur MgSO₄, filtrées et concentrées sous vide. La purification est effectuée sur gel de silice (cyclohexane/AcOEt 7:3) pour donner l'ester attendu (7 mg, 14 µmol, 30 %) sous forme d'huile incolore. 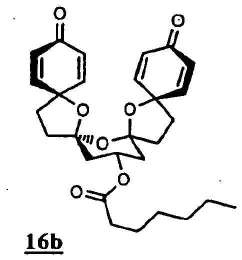 C₂₈H₃₄O₇
M = 482,6 g.mol⁻¹
**RMN ¹H (400 MHz, CDCl₃) :** δ = 0,88 (t, *J*= 6,8 Hz, 3H) ; 1,22-1,37 (m, 6H) ; 1,55- 1,65 (m, 2H) ; 1,87 (dd, *J =* 13,5, 8,0 Hz, 2H) ; 2,04-2,19 (m, 4H) ; 2,26-2,38 (m, 6H) ; 2,49 (dd, *J* = 13,5, 5,4 Hz, 2H) ; 5,32-5,40 (m, 1H) ; 6,09-6,17 (m, 4H) ; 6,75 (dd, *J* = 10,0, 2,8 Hz, 2H, H1) ; 7,03 (dd, *J*= 10,2, 2,8 Hz, 2H).
**RMN ¹³C (100 MHz, CDCl₃) :** δ = 14,2 ; 22,7 ; 25,1 ; 29,0 ; 31,6 ; 34,8 ; 35,4 ; 39,6 ; 40,2 ; 65,4 ; 79,6 ; 109,3 ; 127,6 ; 127,7 ; 149,2 ; 150,7 ; 173,1 ; 185,4.
**Masse (ESI+)** : *m*/*z* (%) 521 [M+K]⁺ (14), 505 [M+Na]⁺ (100).
**SMHR (ESI+)** : *m*/*z* calculée pour C₂₈H₃₄O₇Na 505,2202, trouvée 505,2204.

### Example 30: Préparation du (8R,10S,21S)- et (8S,10R,21R)-Tetradécanoate de 7,9,11-trioxatétraspiro[5.1.1.1.5¹².2¹⁰.3⁸.2⁶]tétracosa-1,4,13,16-dièn-3,15-on-21-yle (16c)

A une solution d'alcool **4a** (31,0 mg, 0,08 mmol) dans 3 mL de CH₂Cl₂ anhydre, sont ajoutés, à 0°C et sous argon, la DMAP (10,0 mg, 0,08 mmol), le chlorure de tétradécanoyle (25,0 mg, 0,10 mmol) puis la triéthylamine (10,1 mg, 0,10 mmol). Après 0,5 h d'agitation à 0°C et 4 h à température ambiante, le milieu réactionnel est quenché par ajout d'une solution aqueuse 1N de HCl. Après extraction au CH₂Cl₂, les phases organiques sont rassemblées, séchées sur MgSO₄, filtrées et concentrées sous vide. La purification est effectuée sur gel de silice (cyclohexane/AcOEt 7:3) pour donner l'ester attendu (26,7 mg, 0,05 mmol, 55 %) sous forme d'huile incolore. 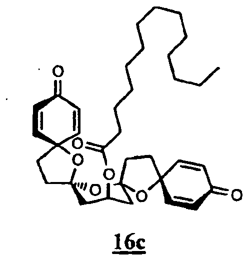 C₃₅H₄₈O₇
M = 580,8 g.mol⁻¹
**RMN ¹H (400 MHz, CDCl₃) :** δ = 0,88 (t, *J* = 6,8 Hz, 3H) ; 1,23-1,34 (m, 20H) ; 1,55- 1,65 (m, 2H) ; 1,94-2,38 (m, 12H) ; 2,52 (ddd, *J* = 12,8, 7,6, 3,6 Hz, 1H) ; 2,80 (ddd, *J*= 12,8, 7,2, 2,0 Hz, 1H) ; 5,18-5,27 (m, 1H) ; 6,11-6,20 (m, 4H) ; 6,72-6,77 (m, 2H) ; 6,85 (dd, *J* = 10,0, 3,0 Hz, 1H); 6,92 (dd, *J* = 10,0, 3,0 Hz, 1H).
**RMN ¹³C (100 MHz, CDCl₃) :** δ = 14,3 ; 22,9 ; 25,1 ; 29,3-29,9 ; 32,1; 34,6 ; 35,4 ; 38,3 ; 39,8 ; 39,9 ; 66,1 ; 78,9 ; 79,6 ; 109,2 ; 110,1 ; 127,6 ; 127,7 ; 127,9 ; 128,2 ; 148,7 ; 148,8 ; 149,9 ; 151,0; 173,4 ; 185,3 ; 185,4.
**Masse (ESI+) :** *m*/*z* (%) 619 [M+K]⁺ (15), 603 [M+Na]⁺ (100).
**SMHR (ESI+)** : *m*/*z* calculée pour C₃₅H₄₈O₇Na 603,3298, trouvée 603,3295.

### Exemple 31: Préparation du (8R,10R,21S)-Tetradécanoate de 7,9,11-trioxatétraspiro[5.1.1.1.5¹².2¹⁰.3⁸.2⁶]tétracosa-1,4,13,16-dièn-3,15-on-21-yle (16d)

A une solution d'alcool **4b** (12,1 mg, 0,03 mmol) dans 1 mL de CH₂Cl₂ anhydre, sont ajoutés, à 0°C et sous argon, la DMAP (3,9 mg, 0,03 mmol), le chlorure de tétradécanoyle (10 mg, 0,04 mmol) puis la triéthylamine (4,0 mg, 0,04 mmol). Après 0,5 h d'agitation à 0°C et 4 h à température ambiante, le milieu réactionnel est quenché par ajout d'une solution aqueuse 1N de HCl. Après extraction au CH₂Cl₂, les phases organiques sont rassemblées, séchées sur MgSO₄, filtrées et concentrées sous vide. La purification est effectuée sur gel de silice (cyclohexane/AcOEt 7:3) pour donner l'ester attendu (13,7 mg, 0,02 mmol, 72 %) sous forme d'huile incolore. 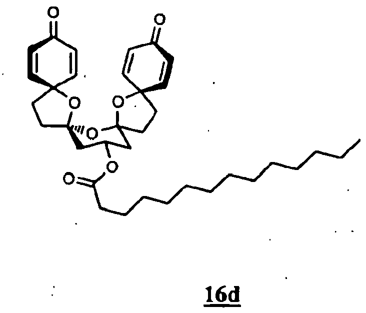 C₃₅H₄₈O₇
M = 580,8 g.mol⁻¹
**RMN ¹H (400 MHz, CDCl₃) :** δ = 0,88 (t, *J* = 6,8 Hz, 3H) ; 1,22-1,35 (m, 20H) ; 1,55-1,65 (m, 2H) ; 1,87 (dd, *J* = 13,5, 8,2 Hz, 2H) ; 2,03-2,18 (m, 4H) ; 2,26-2,36 (m, 6H); 2,49 (dd, *J* = 13,5, 5,2 Hz, 2H) ; 5,33-5,40 (m, 1H) ; 6,09-6,16 (m, 4H) ; 6,75 (dd, *J* = 10,0, 2,8 Hz, 2H) ; 7,03 (dd, *J* = 10,0, 2,8 Hz, 2H).
**RMN ¹³C (100 MHz, CDCl₃) :** δ = 14,3 ; 22,9 ; 25,1 ; 29,0-29,9 ; 32,1 ; 34,7 ; 35,4 ; 39,6 ; 40,2 ; 65,4 ; 79,6 ; 109,3 ; 127,6 ; 127,7 ; 149,2 ; 150,7 ; 173,1 ; 185,5.
**Masse (ESI+) :** *m*/*z* (%) 619 [M+K]⁺ (14), 603 [M+Na]⁺ (100).
**SMHR (ESI+) :** *m*/*z* calculée pour C₃₅H₄₈O₇Na 603,3298, trouvée 603,3299.

### Exemple 32: Préparation du (8R,10S,21S)- et (8S,10R,21R)-3-Oxohexadécanoate de 7,9,11-trioxatétraspiro[5.1.1.1.5¹².2¹⁰.3⁸.2⁶]tétracosa-1,4,13,16-dièn-3,15-on-21-yle (16e)

### 1) Préparation du composé intermédiaire 17:

### 5-(1-Hydroxytridécylidène)-2,2-diméthyl-[1,3]dioxane-4,6-dione (17)

A une solution d'acide de Meldrum (10,60 g, 73,5 mmol) dans 70 mL de CH₂Cl₂ anhydre, est ajoutée à 0°C, 14,3 mL de pyridine. Après 15 min d'agitation à cette température, le chlorure de tétradécanoyle commercial (18,16 g, 73,5 mmol) est ajouté goutte à goutte, tel que la température reste inférieure à 10°C. Après 2 h d'agitation à 0°C et 1 h à température ambiante, le milieu réactionnel est lavé par 50 mL d'une solution de HCl 2N puis 2x50 mL d'eau. Après un dernier lavage par une solution aqueuse saturée en NaCl, la phase organique est séchée sur MgSO₄, filtrée et concentrée sous vide. L'huile obtenue est cristallisée par ajout d'éthanol absolu puis laissée une nuit au frigo. Le solide est alors filtré puis rincé par de l'éthanol absolu froid avant d'être séché pour donner le composé **17** (21,25 g, 59,9 mmol, 81 %) sous forme d'un solide blanc. 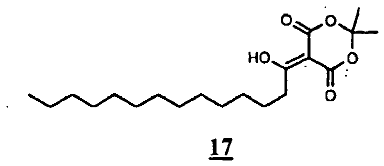 C₂₀H₃₄O₅
M = 354,5 g.mol⁻¹
**RMN ¹H (400 MHz, CDCl₃) :** δ = 0,88 (t, *J* = 7,2 Hz, 3H, 3xH14') ; 1,24-1,43 (m, 20H, 2xH4', 2xH5', 2xH6', 2xH7', 2xH8', 2xH9', 2xH10', 2xH11', 2xH12', 2xH13') ; 1,65-1,72 (m, 2H, 2xH3') ; 1,74 (s, 6H, 2x-C*H₃*); 3,07 (t, *J* = 7,6 Hz, 2H, 2xH2').
**RMN ¹³C (100 MHz, CDCl₃) :** δ *=* 14,1 (C14'); 22,7 (C13') ; 26,1 (C3') ; 26,8 (2x-CH₃) ; 29,2-29,6 (C4', C5', C6', C7', C8', C9', C10', C11') ; 31,9 (C12') ; 35,7 (C2') ; 91,2 (C5) ; 104,7 (C2) ; 160,2 ; 170,6 (C4 et C6) ; 198,3 (C1').
**Masse (FAB+, NBA) :** *m*/*z (%)* 355 [M+H]⁺ (5), 297 (100).

### 2) Préparation du composé 16e

Une solution d'alcool **4a** (50 mg, 0,14 mmol) et de dérivé **17** (48 mg, 0,14 mmol) dans 2 mL de THF anhydre est portée à reflux 6 h. Le milieu réactionnel est concentré sous vide. La purification est effectuée sur gel de silice (cyclohexanc/AcOEt 8:2) pour donner l'ester attendu **16e** (30,4 mg, 0,05 mmol, 36 %) sous forme d'huile incolore. 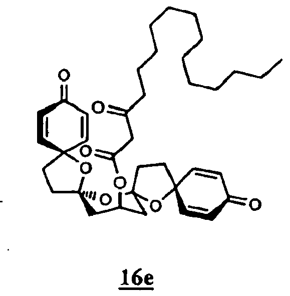 C₃₇H₅₀O₈
M = 622,8 g.mol⁻¹
**RMN ¹H (400 MHz, CDCl₃) :** δ = 0,88 (t, *J* = 6,8 Hz, 3H) ; 1,22-1,32 (m, 20H) ; 1,53-1,62 (m, 2H) ; 1,97-2,27 (m, 10H) ; 2,45-2,54 (m, 3H) ; 2,75-2,82 (m, 1H) ; 3,44 (s, 2H) ; 5,25-5,34 (m, 1H) ; 6,11-6,21 (m, 4H) ; 6,72-6,78 (m, 2H) ; 6,84 (dd, *J* = 9,6, 3,0 Hz, 1H) ; 6,92 (dd, *J* = 10,0, 3,0 Hz, 1 H).
**RMN ¹³C (100 MHz, CDCl₃) :** δ = 14,3 ; 22,9 ; 23,7 ; 29,2-29,8 ; 32,1 ; 35,4 ; 38,4 ; 39,6 ; 39,9 ; 43,5 ; 49,4 ; 67,3 ; 78,9 ; 79,7 ; 109,1 ; 109,9 ; 127,6 ; 127,8 ; 127,9 ; 128,2 ; 148,7 ; 148,8 ; 149,8 ; 150,9 ; 166,8 ; 185,2 ; 185,4 ; 202,9.

### Exemple 33: Préparation du (8R,10S,21S)- et (8S,10R,21R)- 1-(7,9,11-trioxatétraspiro[5.1.1.1.5¹².2¹⁰.3⁸.2⁶]tétracosa-1,4,13,16-tétraèn-3,5-dion-21-yl)-pentadécan-2-one 23c et (8R,10R,21R)-1-(7,9,11-trioxatétraspiro[5.1.1.1.5¹².2¹⁰.3⁸.2⁶]tétracosa-1,4,13,16-tétraèn-3,5-dion-21-yl)-pentadécan-2-one (méso-23d)

### 1) Préparation du composé :

### 3-[4-(Tétrahydro-pyran-2-yloxy)-phényl]-propionic acide éthyl ester (18)

A une solution d'ester **4'-1** (3,0 g ; 15,5 mmol) dans CH₂Cl₂ (25 mL), sont ajoutés le 3,4-dihydro-2H-pyrane (1,95 g ; 23,2 mmol) et le catalyseur PPTS (774 mg ; 3,02 mmol). La réaction est laissée sous agitation tout en la suivant par plaque CCM avec un système d'éluant (AcOEt/cyclohexane 1:9). Après 3 h d'agitation à température ambiante, on passe au lavage avec NaCl et à l'extraction avec AcOEt. La phase organique est séchée sur MgSO₄, filtrée et concentrée sous vide. Après une colonne chromatographique (AcOEt/cyclohexane 1,5:8,5), l'ester protégé **18** (3,99 g ; 14,4 mmol ; 93%) est isolé sous forme d'huile jaune. 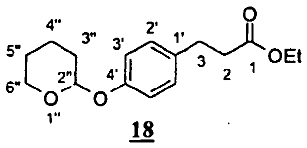 C₁₆H₂₂O₄
M = 278 g.mol⁻¹
R_{f}=0.29 (1 :9 ethyl acetate/cyclohexane)
**UV :** 280, 273, 222, 202 nm.
I**R vₘₐₓ (film, cm⁻¹)**: 2943, 1735, 1510, 1235, 1202, 1038, 969, 921.
**¹H NMR (400 MHz, CDCl₃) :** δ = 1.23 (t, *J* = 7.1 Hz, 3H, -OCH₂*CH₃*), 1.55-1.69 (m, 3H, H5a", H4a", H4b"), 1.81-1.88 (m, 2H, H5b", H3a"), 1.99 (m, 1H, H3b"), 2.57 (t, *J*= 7.9 Hz, 2H, 2xH3), 2.88 (t, *J* = 7.9 Hz, 2H, 2xH2), 3.60 (m, 1H, H6a"), 3.90 (m, 1H, H6b"), 4.12 (q, *J* = 7.1 Hz, 2H, -O*CH₂*CH₃), 5.37 (brs, 1H, H2"), 6.97 (d, *J* = 8.5 Hz, 2H, 2xH2'), 7.10 (d, *J*= 8.5 Hz, 2H, 2xH3').
**¹³C NMR (100 MHz, CDCl₃)** : δ = 14.3 (-OCH₂*CH₃*), 19.0 (C₅"), 25.4 (C4"), 30.3 (C3"), 30.5 (C3), 36.3 (C2), 60.5 (-O*CH₂*CH₃), 62.2 (C6"), 96.6 (C2"), 116.6 (2xC2'), 129.3 (2xC3'), 133.8 (C1'), 155.7 (C4'), 173.1 (C1).
**LRMS (ESI+):** *m*/*z* (%) 301 (100) [*M*+Na]⁺, 195 (43).

### 2) Préparation du composé :

### {2-Oxo-4-[4-hydroxy-phénylyran-2-yloxy]-butyl}-phosphonic acide diéthyl ester (19)

A une solution de diéthylméthylphosphonate (4,79 g ; 37,8 mmol) dans du THF anhydre (20 mL) est ajouté le *n*-BuLi à 2,5 M dans l'hexane (15 mL, 36,5 mmol) à -78°C. Pendant cette addition un trouble blanc est observé. Après 45 min d'agitation, on ajoute une solution d'ester **18** (3,5 g ; 12,6 mmol) préparée dans du THF (15 mL). Après 2 h d'agitation la réaction est neutralisée par l'ajout de 30 mL de NH₄Cl, suivie d'une extraction avec AcOEt (3x50 mL). La phase organique récupérée est ensuite séchée sur MgSO₄, filtrée et concentrée sous vide. La purification est effectuée sur gel de silice (AcOEt/cyclohexane 1:1) pour donner l'ester **19** (3,33g ; 8,67 mmol; 69%) sous forme d'huile jaune. 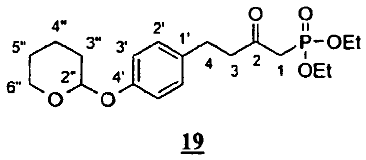 C₁₉H₂₉O₆P
M = 384g.mol⁻¹
R_{f}=0.16(1 :1 ethylacetatelcyclohexane)
**UV** : 280, 274, 222, 202 nm.
**IR νₘₐₓ (film, cm⁻¹):** 2942, 2872, 1715, 1510, 1392, 1236, 1023, 968.
**¹H NMR (400 MHz, CDCl₃) :** δ = 1.32 (t, *J* = 7.1 Hz, 6H, 2x-OCH₂*CH₃*), 1.56-1.72 (m, 3H, HH₅ₐ", H4a", H4b"), 1.83-1.88 (m, 2H, H3a", H5b"), 2.00 (m, 1H, H3b"), 2.85 (m, 2H, 2xH3), 2.93 (m, 2H, 2xH4), 3.06 (d, *J* = 22.8 Hz, 2H, 2xH1), 3.59 (m, 1H, H6a"), 3.91 (m, 1H, H6b"), 4.12 (q, *J* = 7.1, 4H, 2x-O*CH₂*CH₃), 5.37 (brs, 1H, H2"), 6.96 (d, *J* = 8.6 Hz, 2H, 2xH2'), 7.09 (d, *J* = 8.6 Hz, 2H, 2xH3').
**¹³C NMR (100 MHz, CDCl₃) :** δ = 16.1 (2x-OCH₂*CH₃*), 18.6 (C5"), 25.0 (C4"), 28.4 (C4), 30.1 (C3"), 42.2 (d, *J* = 126 Hz, C1), 45.4 (C3), 61.7 (2x-O*CH₂*CH₃), 62.2 (C6"), 96.2 (C2"), 116.3 (2xC2'), 129.0 (2xC3'), 133.4 (C1'), 155.2 (C4'), 200.9 (d, *J* = 6 Hz, C2).
**LRMS (ESI+):** *m*/*z* (%) 407 (100) [*M+*Na]⁺, 301 (72).

### 3) Préparation du composé:

### (2-{2-[4-(Tetrahydro-pyran-2-yloxy)-phényl]-éthyl]-[1,3] dithian-2-yl)-acétaldéhyde (20)

A une solution d'aldéhyde **1** (1,80 g ; 6,38 mmol) dans CH₂Cl₂ anhydre (40 mL), on ajoute le 3,4-dihydro-2H-pyrane (1,17 mL ; 12,8 mmol) et le PPTS (318 mg ; 1,28 mmol). On laisse la réaction se faire à température ambiante, tout en suivant son avancée par plaque CCM avec un système d'éluant (AcOEt/cyclohexane 2:8). Après 90 min d'agitation, on passe au lavage avec 30 mL de NaHCO₃ et à l'extraction avec AcOEt. La phase organique est séchée sur MgSO₄, filtrée et concentrée sous vide. Après une colonne chromatographique (AcOEt/cyclohexane 8:92), l'aldéhyde protégé **20** (1,98 g ; 5,41 mmol ; 85%) est isolé sous forme d'huile jaune. 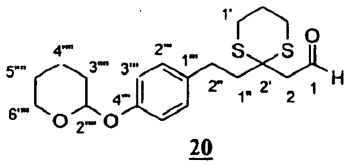 C₁₉H₂₆O₃S₂
M = 366g mol⁻¹
R_{f} = 0.24 (1:9 ethyl acetate/cyclohexane)
**UV** : 280, 222, 218, 201 nm.
**IR νₘₐₓ (film, cm⁻¹) :** 2943, 2851, 1718, 1510, 1440, 1234, 1110, 1037, 968, 921.
**¹H NMR (400 MHz, CDCl₃):** δ = 1.56-1.72 (m, 3H, H4a"", H5a"", H4b""), 1.82-1.88 (m, 2H, H3a"", H3b""), 1.94-2.08 (m, 3H, -SCH₂C*H*H-, -SCH₂C*H*H-, H5b""), 2.24-2.31 (m, 2H, 2xH1"), 2.74-2.81 (m, 2H, 2xH2"), 2.86-2.95 (m, 46H, 2x-SC*H*H-, 2x-SCH*H*-, 2xH6""), 3.59 (m, 1H, H6a""), 3.91 (m, 1H, H6b""), 5.39 (brs, 1H, H2""), 6.98 (d, *J* = 8.5 Hz, 2H, 2xH3"'), 7.10 (d, *J* = 8.5 Hz, 2H, 2xH2'"), 9.82 (t, *J* = 2.8Hz, 1H, H1).
**¹³C NMR (100 MHz, CDCl₃):** δ = 19.0 (C5""), 24.8 (-SCH₂CH₂-), 25.4 (C4""), 26.4 (2x-SCH₂-), 30.1 (C2"), 30.6 (C3""), 42.6 (C1"), 49.3 (C2'), 50.6 (C2), 62.2 (C6""), 96.7 (C2""), 116.8 (2xC2"'), 129.5 (2xC3"'), 134.2 (C1"'), 155.7 (C4"'), 199.8 (C1).
**LRMS (ESI+):** m/z (%) 389 (100) [M+Na]⁺, 107 (28).

### 4) Préparation du composé :

### 1-[4-(Tetrahydro-pyran-2-yloxy)-phenyl]-6-(2-{2-[4-(tetrahydro-pyran-2-yloxy)-phenyl]-ethyl}-[1,3]dithian-2-yl)-hex-4-en-3-one (21)

Dans un ballon de 50 mL est introduit le réactif de Wittig-Horner **19** (964 mg ; 2,51 mmol) dans du CH₃CN anhydre (10 mL). Ensuite, LiCl (106 mg; 2,51 mmol) et le diisopropyle amine (3,24 g ; 25,1 mmol) sont ajoutés. Après 30 min d'agitation à température ambiante, l'aldéhyde 20 (600 mg ; 1,64 mmol), dans du CH₃CN anhydre (10 mL), est injecté. La réaction est laissée sous agitation une nuit. Puis le solvant est évaporé et le résidu est dilué dans 30 mL d'AcOEt, qu'on lave ensuite avec une solution saturée en NH₄Cl et qu'on extrait avec AcOEt. La solution est séchée sur MgSO₄ et concentrée sous vide, puis purifiée sur colonne chromatographique (AcOEt/cyclohexane 1:9) pour conduire à l'énone **21** (795 mg ; 1,33 mmol ; 81%). 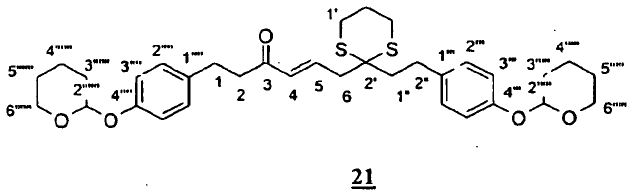 C₃₄H₄₄O₅S₂
M = 596 g.mol⁻¹
R_{f}= 0.23 (15:85 ethyl acetate/cyclohexane)
**UV:** 274, 222, 200 nm.
**IR νₘₐₓ (film, cm⁻¹):** 2942, 1671, 1627, 1509, 1234, 1202, 1110, 1037, 1022, 968, 921, 830.
**¹H NMR (400 MHz, CDCl₃):** δ = 1.56-1.68 (m, 6H, H5a""', H5a""", 2xH4""', 2xH4"""), 1.81-1.91 (m, 4H, H3a""', H3b""", H5b""', H5b"""), 1.93-2.02 (m, 4H, - SCH₂C*H*₂, H3b""', H3b"""), 2.08 (m, 4H, 2xH1", 2xH1), 2.72 (m, 2H, 2xH2"), 2.69-2.92 (m, 8H, 2x-SC*H*₂CH₂, 2xH6, 2xH2), 3.57 (m, 2H, H6a""', H6a"""), 3.89 (m, 2H, H6b""', H6b"""), 5.36 (m, 2H, H2'"", H2"""), 6.16 (d, *J* = 16.0 Hz, 1H, H4), 6.88 (td, *J* = 16.0, 7.2 Hz, 1H, H5), 6.94-7.17 (m, 8H, 2xH2"', 2xH2"", 2xH3"', 2xH3"").
**¹³C NMR (100 MHz, CDCl₃):** δ = 18.9 (C5""', C5"""), 25.0 (-SCH₂*C*H₂), 25.3 (C4""', C4"""), 26.2 (2x-S*C*H₂*C*H₂), 29.4 (C2"), 29.9 (C2), 30.5 (C3""', C3""''), 41.5 (C1"), 41.6 (C1), 41.9 (C6), 52.1 (C2'), 62.1 (C6""', C6"""), 96.5 (C2""', C2"""), 116.6 (2xC2"', 2xC2""), 129.3 (2xC3"', 2xC3""), 133.1 (C4), 134.3 (C1"', C1 ""), 141.8 (C5), 155.5 (C4"', C4""), 199.3 (C3).
**LRMS (ESI+):** *m*/*z* (%) 619 (100) [*M*+Na]⁺, 413 (57), 282 (52).

### 5) Préparation du composé:

### 5-((2-(4-(tétrahydro-2H-pyran-2-yloxy)phénéthyl)-1,3-dithian-2-yl)méthyl)-1-(4-(tétrahydro-2H-pyran-2-yloxy)phényl)icosane-3,7-dione 22

Le triméthyl-(1-méthylène-tétradécyloxy)-silane (176 mg, 0.6 mmol) et l'énone **21** (176 mg, 0.3 mmol) sont dissous dans 2 mL de THF anhydre et la solution est refroidit à -78°C. Deux gouttes de fluorure de tétrabutylammonium (1 M dans le THF) sont injectées. Le mélange est agité pendant 1 h à cette température, puis la solution est laissée remonter à température ambiante sous agitation. Après addition d'eau et extraction à l'acétate d éthyle, le produit est séché et le solvant évaporé. Le résidu est purifié par flash chromatographie pour conduire au composé **22**.

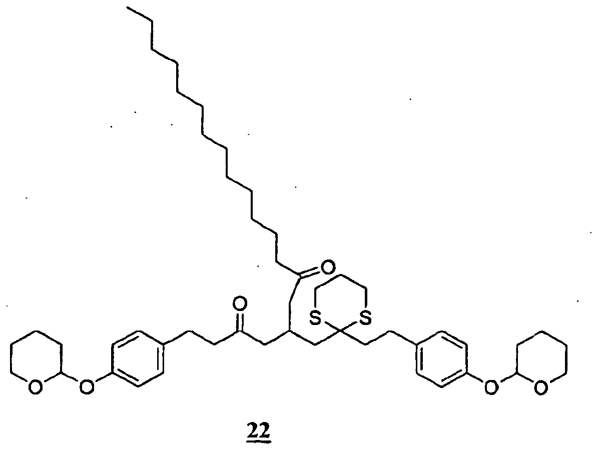

### 6) Préparation des composés :

### (±)-(8S,10R,21R)-1-(7,9,11-trioxatétraspiro[5.1.1.1.5¹².2¹⁰.3⁸.2⁶]tétracosa-1,4,13,16-tétraèn-3,5-dion-21-yl)-pentadécan-2-one 23c et (8R,10R,21R)-1-(7,9,11-trioxatétraspiro[5.1.1.1.5¹².2¹⁰.3⁸.2⁶]tétracosa-1,4,13,16-tétraèn-3,5-dion-21-yl)-pentadécan-2-one (méso-23d)

Le composé **22** dans un mélange acétone/H₂O (10 :1, 0.04 M) est ajouté dans le noir et à température ambiante le PIFA (6.2 équiv). Après une nuit d'agitation, le milieu réactionnel est traité par une solution saturée de NaHCO₃, avant d'être extrait à l'AcOEt. Après séchage (MgSO₄), le solvant est évaporé. Les deux produits **23c** et **23d** sont isolés par flash chromatographie.

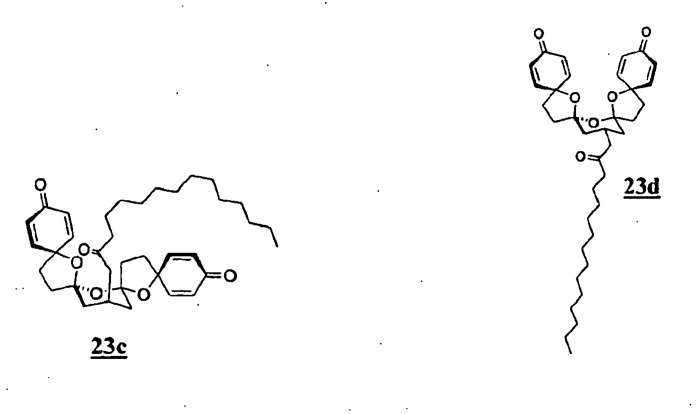

### II) ACTIVITE DES COMPOSES

### II-1) Activité autitoxoplasmique in vitro

Le test mis en oeuvre ici est un test de microtitrage colorimétrique. Une monocouche de cellules HFF (Human Foreskin fibroblast) est infectée, de manière synchrone, par le parasite (10⁴ parasites par puits, souche RH-β1). Après lavage, des doses croissantes (de 50 nM à 100 µM) du composé sont ajoutées, excepté dans le puits de contrôle (ajout ou non de DMSO). Après 48 h d'incubation, l'activité β-galactosidase est mesurée par ajout d'un substrat de l'enzyme, le rouge de chlorophénol β-D-galactopyranoside (CPRG). Les plaques sont de nouveau incubées (de 30 min à 16 h à 37°C) avant d'être lue à 570 et 630 nm.
Les résultats sont présentés dans le tableau I ci-dessous.

### II-2) Activité antipaludique in vitro

Avant expérimentation, des parasites de souche 3D7 (chloroquino-sensible, Delemarre-Van de Waal H. A. et F. C. de Waal, Ned. Tijdschr. Geneeskd 1981, 125, 375-377 ; numéro ATCC : MRA-102) de *Plasmodium falciparum* sont maintenus en culture selon la méthode Trager et Jansen (Trager et Jansen, 1976).
L'activité antipaludique est mesurée *in vitro* sur culture asynchrone de globules rouges infectés par *Plasmodium falciparum* selon une méthode modifiée (Ancelin et al., 2003) d'après le microtest isotopique de Desjardins (Desjardins *et al.,* 1979). Les suspensions de globules rouges infectés (1,5 % d'hématocrite finale, 0,6 % de parasitémie) ont été cultivées en milieu complet (RPMI 1640 supplémenté avec 0,5 % d'Albumax de type I) soit en absence de composé à tester (contrôle) soit en contact avec des concentrations variables (de 1,5.10⁻⁵ à 4,6.10⁻⁹ M) établies par dilutions sériées en milieu complet à partir d'une solution hautement concentrée (10 mM en DMSO) du composé à tester. A l'issue de 48 h d'incubation, 0,5 µCi [³H]-hypoxanthine (un précurseur nécessaire à la synthèse des acides nucléiques du parasite, radiomarqué) sont ajoutés à chaque puits de la plaque de microtitrage. Après 18 h d'incubation, la réaction d'incorporation d'hypoxanthine est stoppée et les cellules lysées par congélation directe à -80°C. Après décongélation, les macromolécules parasitaires, incluant les acides nucléiques radiomarqués, sont recueillies sur filtre au moyen d'un collecteur de cellules. Un cocktail de scintillation est ajouté au filtre et la radioactivité incorporée est alors mesurée au compteur à scintillation.
Le bruit de fond radioactif est mesuré à partir de globules rouges non infectés et soustrait de chaque mesure du filtre. La viabilité des parasites traités par le composé à tester est mesurée par leur capacité de synthèse d'acides nucléiques à partir d'un précurseur radiomarqué ([H³]-hypoxanthine) en comparaison aux parasites contrôles cultivés en absence du composé et est exprimée en pourcentage du contrôle. Les mesures sont analysées au moyen du logiciel Graphpad Prism et la CI₅₀ (concentration en composé capable d'inhiber in vitro 50 % de la croissance des parasites) est déterminée graphiquement. Les résultats obtenus apparaissent sous forme de CI₅₀ dans le tableau et sont le résultat de deux expériences réalisées indépendamment (différentes cultures parasitaires, différentes dilutions) en duplicat.

Les résultats sont présentés dans le tableau I ci-dessous :

**TABEAU I**

| **Nom ou numéro des Produits** | *Toxoplasma* Test prolifération (CI 50) | *Plasmodium* Test prolifération souche 3D7 (CI 50) |
|---|---|---|
| **Chloroquine** | *non testé* | 8-9 nM |
| **Aculéatine A naturelle** | 0,309 µM | 0,288 µM |
| **Aculéatine B naturelle** | 0,365 µM | 0,454 µM |
| **Aculéléatine 6-*épi*-D** | 0,377 µM | 0,451 µM |
| **10a** | 0,500 µM | 1,842 µM |
| **10b** | 0,341 µM | 0,654 µM |
| **11b** | 1,636 µM | 1,364 µM |
| **10c** | 2,4 µM | 0,770 µM |
| **12c** | 3,54 µM | 1,031 µM |
| **13c** | 3,525 µM | 1,133 µM |
| **12d** | 4,45 µM | 1,151 µM |
| **4b** | 1,5 µM | 0,584 µM |
| **4a** | 0,173 µM | 0,414 µM |
| **16c** | 0,346 µM | 0,0815 µM |
| **16d** | 0,636 µM | 0,0917 µM |
| **10e** | *non testé* | 0,899 µM |
| **16a** | *non testé* | 0,343 µM |
| **16b** | *non testé* | 0,331 µM |
| **16e** | *non testé* | 0,122 µM |

Les composés de l'invention possèdent une forte activité aussi bien sur le genre *Plasmodium* que sur le genre *Toxoplasma.*

### II-3) Cycle biologique de Plasmodium spp

La figure 10 présente les différents cycles biologiques de *Plasmodium* Spp. (Robert V & Boudin C. Bull Soc Path Exo 2003; 96 : 6-20))

### Cycle parasitaire chez l'homme :

Le terme « phase exoérythrocytaire » désigne le sporozoïte et le stade hépatique. Ces stades ne semblent avoir aucun effet pathologique pour l'homme.

Le cycle parasitaire chez l'homme commence lorsqu'un anophèle femelle infectieux se gorge sur le sang d'un individu. Les sporozoïtes, formes parasitaires mobiles d'environ 10 µm de long sur 1 µm de large qui se concentrent dans les glandes salivaires, sont émis au site de piqûre lorsque le moustique envoie sa salive avant et pendant le repas sanguin.

Une fois injectés à l'homme, les sporozoïtes atteignent le foie, où ils pénètrent dans les hépatocytes. Rapidement le sporozoïte se transformera en trophozoïte, entouré d'une membrane plasmique (le plasmalemme) au sein d'une vacuole parasitophore. Commence alors une période de réplication intense : la schizogonie hépatique. Pendant cette période de l'ordre de 5 à 6 jours pour *P. falciparum* et 15 jours pour *P. malariae,* il y aura formation de plusieurs milliers de mérozoïtes hépatiques. La cellule hépatique distendue, gonflée va éclater déchargeant ainsi des mérozoïtes dans la circulation sanguine. Ces mérozoïtes ne peuvent cependant pas envahir les hépatocytes. Dans les infections à *P. vivax* ou à *P. ovale* certaines formes intrahépatocytaires ne se divisent pas immédiatement mais restent inactives pendant des mois avant que cette multiplication ne commence. Ces formes endormies ou hypnozoïtes seraient responsables des rechutes qui caractérisent l'infection par ces deux parasites.

### Stade sanguin :

Le cycle érythrocytaire est seul responsable de la maladie.

Après leur libération dans la circulation, les mérozoïtes hépatiques vont rapidement envahir les érythrocytes et initier le stade sanguin. Une fois entré, le mérozoïte va se transformer en anneau, caractérisé par un cytoplasme très fin entourant la vacuole parasitophore. Ensuite le cytoplasme s'épaissit et le parasite augmente de taille. A ce stade, appelé trophozoïte, apparaissent des grains de pigment dans le cytoplasme, qui résultent de la dégradation de l'hémoglobine en hémozoïne. Ce trophozoïte entame une série de mitoses jusqu'à formation d'un schizonte mature (rosace) qui éclate en rompant la membrane du globule rouge pour libérer, selon l'espèce, de 8 à 32 mérozoites. Ces derniers peuvent ensuite envahir d'autres érythrocytes. Ce cycle érythrocytaire est de 48 heures pour *P. falciparum, P.* vivax et *P. ovale* et de 72 heures pour *P. malariae.* Quelques parasites vont avoir un développement différent (gamétocytogenèse) aboutissant aux gamétocytes, formes sexuées du parasite chez l'homme.

### II-4) Détermination du temps de contact minimal nécessaire à la cytotoxicité en fonction du stade parasitaire (time-course).

Une culture de parasites *Plasmodiumfalciparum* est synchronisée au VarioMACS (qui retient les formes matures, qui synthétisent un pigment : l'hémozoïne). Après 6 h de passage de la culture sur colonne VarioMACS, la plage de synchronisation des parasites est restreinte par une seconde synchronisation au sorbitol 5 % (qui ne retient que le stade jeune, dit en anneau) au temps t₀, c'est-à-dire peu de temps après l'invasion de globules rouges sains par les mérozoïtes issus des schizontes obtenus par synchronisation au VarioMACS. Les parasites sont alors mis en contact lors de chaque stade (anneau, puis trophozoïte puis schizonte) avec une concentration fixe de composé (20xCI₅₀ préalablement définie par le protocole ci-dessus) et pendant des temps variables (30 min, 1 h 30 min, 3 h, 5 h, 7 h et 9 h pour les parasites traités en stade anneau ; 30 min, 1 h 30 min, 3 h, 5 h et 7 h pour les parasites traités en stade trophozoîte et schizonte). A l'issue du temps de contact défini, le tapis globulaire est lavé deux fois en milieu complet. A t₀₊₄₄ₕ, (c'est-à-dire au début du cycle parasitaire suivant), les tapis globulaires sont resuspendus et redistribués en plaque 96 puits (en sextuplats), 0,5 µCi d'[³H]-hypoxanthine tritiée sont alors ajoutés au milieu de culture. Dès lors le traitement est le même que celui de l'expérience sus-mentionnée avec une différence : les résultats obtenus apparaissent sous forme d'un graphique qui représente la viabilité des parasites exprimée en pourcentage de contrôle en fonction du temps de contact de la culture avec le composé à tester.

La figure 11 présente les résultats obtenus avec les composés **16c** et **16d** et montrent que ces molécules sont actives sur les différents stades sanguins (anneaux, trophozoïtes et schizontes). L'action de ces molécules est extrêmement rapide et n'est pas réversible permettant ainsi une activité prolongée.

## Revendications

1. Composé polyspirannique de formule (I) suivante : 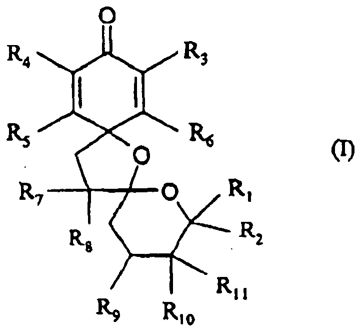 dans laquelle :
- R₁ représente l'hydrogène et R₂ représente un groupe -CH₂COX-R', dans lequel X= N, O, CH₂ ;
lorsque X=O, R' représente un alkyle saturé ou non saturé, linéaire ou ramifié de 1 à 20 atomes de carbone ou un cycloalkyle de 3 à 8 atomes de carbone;
lorsque X= CH₂, R' représente l'hydrogène, un alkyle saturé ou non saturé, linéaire ou ramifié de 1 à 20 atomes de carbone ou un cycloalkyle de 3 à 8 atomes de carbone; lorsque X=N, N peut être monosubstitué par R' ou disubstitué par R' et R" qui représentent, indépendamment l'un de l'autre, l'hydrogéne ou un alkyle saturé ou non saturé, linéaire ou ramifié de 1 à 20 atomes de carbone ou un cycloalkyle de 3 à 8 atomes de carbone;
R₉ représente :
• un groupe OH,
• un groupe O-allyle, un groupe O-benzyle, un groupe O-CO-alkyle, un groupe O-COCH₂CO-alkyle, ledit résidu alkyle étant saturé ou non saturé, linéaire ou ramifié de 1 à 20 atomes de carbone, ou un groupe OCO-cycloalkyle de 3 à 8 atomes de carbone,
ou,
- R₁ et R₂ représentent ensemble un groupe G-1: 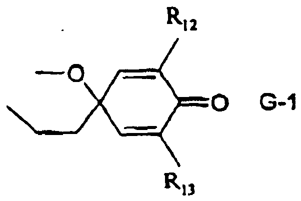 R₁₂ et R₁₃ représentent indépendamment l'un de l'autre l'hydrogène, un groupe alkyle, saturé ou non saturé, linéaire ou ramifié de 1 à 6 atomes de carbone, un groupe cycloalkyle de 3 à 8 atomes de carbone, un groupe O-alkyle, saturé ou non saturé, linéaire ou ramifié de 1 à 6 atomes de carbone, un groupe O-cycloalkyle de 3 à 8 atomes de carbone, un atome d'halogène;
R₉ représente :
• un groupe OH,
• un groupe alkyle saturé ou non saturé, linéaire ou ramifié de 1 à 20 atomes de carbone, un groupe cycloalkyle de 3 à 8 atomes de carbone,
• un groupe S(O)-R" dans lequel R" est un alkyle saturé ou non saturé, linéaire ou ramifié de 1 à 20 atomes de carbone ou un groupe cycloalkyle de 3 à 8 atomes de carbone,
• un groupe O-alkyle, un groupe O-CO-alkyle, un groupe O-COCH₂CO-alkyle, ledit résidu alkyle étant saturé ou non saturé, linéaire ou ramifié de 1 à 20 atomes de carbone, un groupe O-cycloalkyle de 3 à 8 atomes de carbone, un groupe O-CH₂-CH₂-N(CH₃)₂; un groupe O-CH₂-CCH,
• un groupe 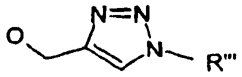 dans lequel R"' est un alkyle saturé ou non saturé, linéaire ou ramifié de 1 à 20 atomes de carbone,
• un groupe N(R")(R"'), R" et R"' étant tels que définis ci-dessus,
et quels que soient R₁ et R₂ :
- R₃, R₄, R₅, R₆ représentent indépendamment l'un de l'autre l'hydrogène, un groupe alkyle, saturé ou non saturé, linéaire ou ramifié de 1 à 6 atomes de carbone, un groupe cycloalkyle de 3 à 8 atomes de carbone, un groupe O-alkyle, saturé ou non saturé, linéaire ou ramifié de 1 à 6 atomes de carbone, un groupe O-cycloalkyle de 3 à 8 atomes de carbone, un atome d'halogène;
- R₇ et R₈ représentent indépendamment l'un de l'autre l'hydrogène, un groupe alkyle, saturé ou non saturé, linéaire ou ramifié de 1 à 6 atomes de carbone, un groupe cycloalkyle de 3 à 8 atomes de carbones, un groupe O-alkyle, saturé ou non saturé, linéaire ou ramifié de 1 à 6 atomes de carbone, un groupe O-cycloalkyle de 3 à 8 atomes de carbone, un groupe NH-alkyle saturé ou non saturé, linéaire ou ramifié de 1 à 6 atomes de carbone, un groupe NH-cycloalkyle de 3 à 8 atomes de carbone, un groupe NHCO-R, dans lequel R peut être un groupe alkyle, saturé ou non saturé, linéaire ou ramifié de 1 à 6 atomes de carbone, un groupe cycloalkyle de 3 à 8 atomes de carbones,
- R₁₀ et R₁₁ représentent indépendamment l'un de l'autre l'hydrogène, un groupe hydroxyle, un groupe O-alkyle, le résidu alkyle étant saturé ou non saturé, linéaire ou ramifié de 1 à 8 atomes de carbone, un groupe O-cycloalkyle de 3 à 8 atomes de carbone, une amine tertiaire.

2. Composé polyspirannique selon la revendication 1, de formule générale (II) suivante : 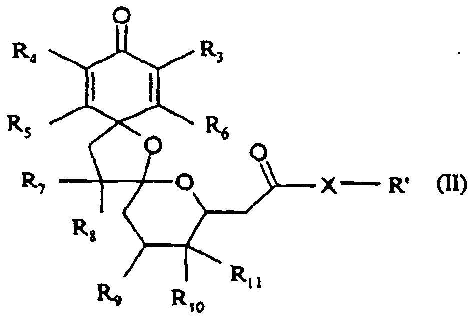 dans laquelle R₃ à R₈, R₁₀ à R₁₁, X et R' sont tels que définis dans la revendication 1 et, R₉ représente un groupe OH, un groupe O-allyle, un groupe O-benzyle, un groupe O-CO-alkyle, ledit alkyle étant saturé ou non saturé, linéaire ou ramifié de 1 à 20 atomes de carbone, ou un groupe O-CO-cycloalkyle comportant 3 à 8 atomes de carbones.

3. Composé polyspirannique selon la revendication 2, de formule générale (III) suivante : 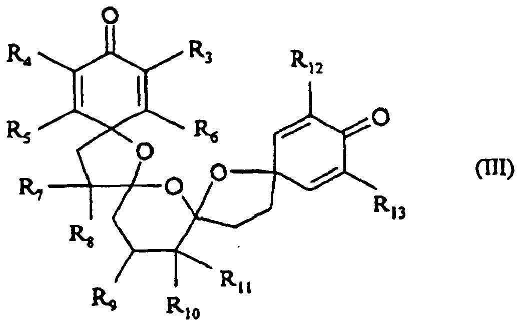 dans laquelle R₃ à R₈ et R₁₀ à R₁₃ sont tels que définis dans la revendication 1 lorsque R₁ et R₂ forment ensemble un groupe G-1, et
R₉ représente :
• un groupe OH,
• un groupe alkyle saturé ou non saturé, linéaire ou ramifié de 1 à 20 atomes de carbone, un groupe cycloalkyle de 3 à 8 atomes de carbone,
• un groupe S(O)-R" dans lequel R" est un alkyle saturé ou non saturé, linéaire ou ramifié de 1 à 20 atomes de carbone ou un groupe cycloalkyle de 3 à 8 atomes de carbone,
• un groupe O-alkyle, un groupe O-CO-alkyle, un groupe O-COCH₂CO-alkyle, ledit résidu alkyle étant saturé ou non saturé, linéaire ou ramifié de 1 à 20 atomes de carbone, un groupe O-cycloalkyle de 3 à 8 atomes de carbone, un groupe O-CH₂-CH₂-N(CH₃)₂ un groupe O-CH₂-CCH,
• un groupe 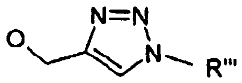 dans lequel R"' est un alkyle saturé ou non saturé, linéaire ou ramifié de 1 à 20 atomes de carbone,
un groupe N(R")(R"'), R" et R"' étant tels que définis ci-dessus.

4. Composé polyspirannique de formule générale (II) selon la revendication 2, dans laquelle R₃ à R₈ et R₁₀ à R₁₁ représentent l'hydrogène, R₉ représente OH, X= O et R' est un alkyle saturé, linéaire à 3 atomes de carbone, à 10 atomes de carbone ou à 18 atomes de carbone.

5. Composé polyspirannique de formule générale (III) selon la revendication 3, dans laquelle :
R₃ à R₈ et R₁₀ à R₁₃ représentent l'hydrogène,
R₉ est un groupe OH, ou O-CO-alkyle dans lequel l'alkyle est linéaire et possède 7 atomes de carbone, 14 atomes de carbone ou 16 atomes de carbone.

6. Composé polyspirannique de formule générale (III) selon la revendication 5, constitué d'un mélange d'énantiomères (8R, 10S, 21S) et (8S, 10R, 21R).

7. Composé polyspirannique de formule générale (III) selon la revendication 5, constitué de l'énantiomère (8R, 10R, 21S), ou de l'énantiomère (8*R* 10*S*, 21*S*) ou encore de l'énantiomère (8*S*, 10*R*, 21*R*).

8. Utilisation d'au moins un composé polyspirannique, ou des sels de celui-ci physiologiquement acceptables, de formule (I) telle que définie dans la revendication I, pour la préparation d'un médicament destiné à la prévention ou au traitement de pathologies impliquant les parasites appartenant au phylum des apicomplexes.

9. Utilisation d'au moins un composé polyspirannique selon la revendication 8, de formule générale (11), telle que définie dans la revendication 2_{.}

10. Utilisation d'au moins un composé polyspirannique selon la revendication 8, de formule générale (III), telle que définie dans la revendication 3.

11. Utilisation d'au moins un composé polyspirannique selon l'une des revendications 8 à 10, dans laquelle lesdits parasites sont de l'espèce des *Plasmodium.*

12. Utilisation d'au moins un composé polyspirannique selon l'une des revendications 8 à 10, dans laquelle lesdits parasites sont du genre *Toxoplasma.*

13. Utilisation d'au moins un composé de formule polyspirannique selon la revendication 12, dans laquelle lesdits troubles sont le paludisme ou la toxoplasmose.

14. Composition pharmaceutique comprenant comme substance active au moins un composé polyspirannique de formule générale I telle que définie dans la 1, en association avec un véhicule pharmaceutiquement acceptable.

15. Composition pharmaceutique selon la revendication 14, se présentant sous forme administrable par voie orale à raison de 0,1 mg/kg/j à 100 mg/kg/j de substance active.

16. Procédé de préparation des composés de formule 1 :
comprenant une étape d'oxydation phénolique avec un oxydant choisi parmi le PIFA ou le PIDA, d'un composé de formule (I-Z) suivante : 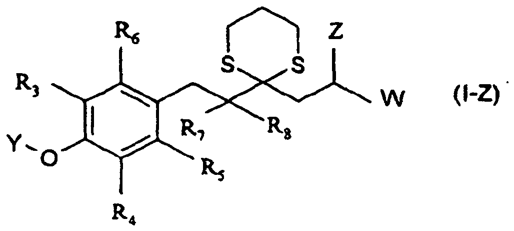 dans laquelle:
- soit W représente un groupe de formule (II-W) suivante: 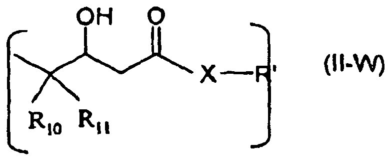 et X = O ou N.
Y représente l'hydrogène,
et Z représente :
• un groupe OH,
R₃ à R₈, R₁₀, R₁₁, X, R' et R"' étant tels que définis dans la revendication 1,
pour obtenir un composé de formule générale II dans laquelle X = O ou N et R9 représente un groupe OH,
et comprenant ensuite optionnellement une étape d'acylation ou d'alkylation pour obtenir un composé de formule générale II dans lequel R₉ est un groupe O-CO-alkyle, un groupe O-allyle, un groupe O-benzyle, un groupe O-COCH₂CO-alkyle, ledit résidu alkyle étant saturé ou non saturé, linéaire ou ramifié de 1 à 20 atomes de carbone, ou un groupe O-CO-cycloalkyle comportant 3 à 8 atomes de carbone,
- soit W représente un groupe de formule II'-W suivante : 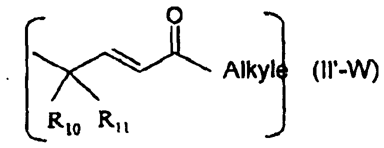 ledit alkyle étant saturé ou non saturé, linéaire ou ramifié de 1 à 17 atomes de carbone ou un cycloalkyle de 3 à 8 atomes de carbone, et X = CH₂ ;
Y représente un groupe (Pr₃)Si-,
et Z représente un groupe (Pr₃)Si-O-,
pour obtenir un composé de formule générale II dans laquelle X = CH₂ et R9 représente un groupe OH,
et comprenant ensuite optionnellement une étape d'acylation ou d'alkylation pour obtenir un composé de formule générale II dans lequel R₉ est un groupe O-CO-alkyle, un groupe O-allyle, un groupe O-benzyle, un groupe O-COCH₂CO-alkyle, ledit résidu alkyle étant saturé ou non saturé, linéaire ou ramifié de 1 à 20 atomes de carbone, ou un groupe O-CO-cycloalkyle comportant 3 à 8 atomes de carbone,
soit W représente un groupe III-W : 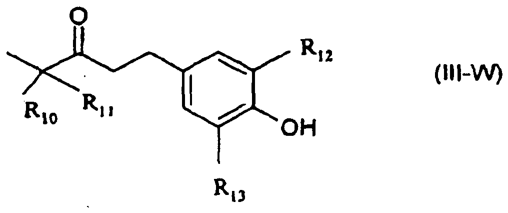 et Z représente :
• un groupe OH,
• un groupe alkyle saturé ou non saturé, linéaire ou ramifié de 1 à 20 atomes de carbone, un groupe cycloalkyle de 3 à 8 atomes de carbone,
• un groupe S-R" dans lequel R" est un alkyle saturé ou non saturé, linéaire ou ramifié de 1 à 20 atomes de carbone ou un groupe cycloalkyle de 3 à 8 atomes de carbone,
• un groupe O-alkyle, l'alkyle étant saturé ou non saturé, linéaire ou ramifié de 1 à 20 atomes de carbone, un groupe O-cycloalkyle de 3 à 8 atomes de carbone, un groupe O-CH₂CH₂-N(Ch₃)₂ ; un groupe O-CH₂-CCH,
• un groupe N(R")(R"'), R" et R'" étant tels que définis ci-dessus,
R₃ à R₈ et R₁₀ à R₁₃ étant tels que définis dans la revendication 1,
pour obtenir un composé de formule générale III dans lequel R9 représente :
• un groupe OH,
• un groupe alkyle saturé ou non saturé, linéaire ou ramifié de 1 à 20 atomes de carbone, un groupe cycloalkyle de 3 à 8 atomes de carbone,
• un groupe S(O)-R" dans lequel R" est un alkyle saturé ou non saturé, linéaire ou ramifié de 1 à 20 atomes de carbone ou un groupe cycloalkyle de 3 à 8 atomes de carbone,
• un groupe O-alkyle, l'alkyle étant saturé ou non saturé, linéaire ou ramifié de 1 à 20 atomes de carbone, un groupe O-cycloalkyle de 3 à 8 atomes de carbone, un groupe O-CH₂-CH₂-N(CH₃)₂ ; un groupe O-CH₂-CCH.
• un groupe N(R")(R"'), R" et R"' étant tels que définis ci-dessus,
et, lorsque Z est un groupe OH (composé III-1), comprenant optionnellement une étape d'acylation ou d'alkylation pour obtenir un composé de formule générale III dans lequel R₉ est un groupe O-alkyle, un groupe O-cycloalkyle, un groupe O-CO-alkyle, un groupe O-COCH₂CO-alkyle, un groupe O-CH₂-CH₂-N(CH₃)₂ ; un groupe O-CH₂-CCH,
et/ou, lorsque Z représente un groupe -O-CH₂-CCH, comprenant optionnellement une réaction d'addition avec N₃-R"' pour obtenir un composé de formule générale III
dans lequel R₉ est un groupe 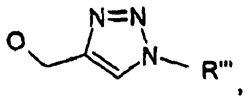 R"' étant tel que défini dans la revendication 1.

17. Procédé de préparation des composés de formule II selon la revendication 16, dans lequel X = O, N et comprenant les étapes suivantes :
• Réaction d'un aldéhyde de formule (IV) suivante : 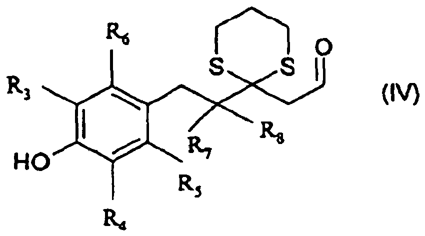 R₃ à R₈ étant tels que définis dans la revendication 1,
avec un composé de formule VII suivante : 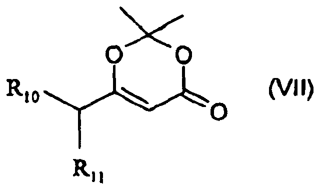 pour obtenir le composé de formule (V) suivante : 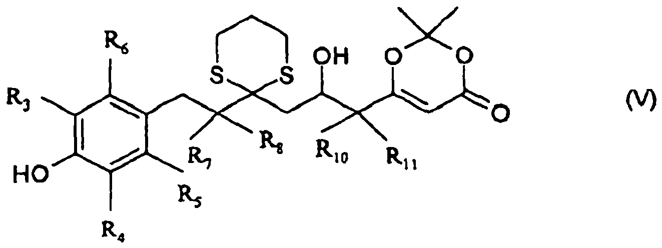 R₃ à R₈ et R₁₀ à R₁₁ étant tels que définis dans la revendication 1,
• réaction du composé (V) obtenu avec un composé R'-X-H, (X= O) ou (R')(R")-X-H (X= N), R' et R" étant tels que définis dans la revendication 1, puis réduction avec un réducteur, choisi parmi NaBH₄ ou NaBH₄ et Et₃B, ou Me₄NBH(OAc)₃, pour obtenir un composé de formule (I-Z) dans lequel W représente le groupe II-W;
• oxydation phénolique du composé (I-Z) obtenu avec un oxydant choisi parmi le PIFA et le PIDA, afin d'obtenir un composé II dans lequel R₉ est OH;
• éventuellement, réaction d'acylation ou d'alkylation du composé II dans lequel R₉ est OH, pour obtenir un composé de formule générale II dans lequel R9 représente un groupe O-allyle, un groupe O-benzyle, un groupe O-CO-alkyle, ou un groupe O-CO-cycloalkyle tels que définis dans la revendication 1.

18. Procédé de préparation des composés de formule II selon la revendication 16, dans lequel X = CH₂ et comprenant les étapes suivantes :
• réaction d'un ester de formule (VIII) suivante : 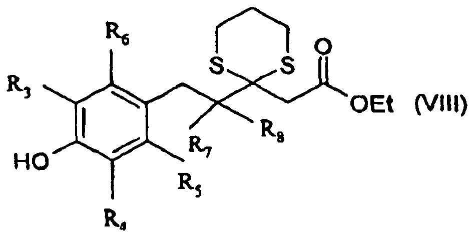 R₃ à R₈ étant tels que définis dans la revendication 1,
avec un acétate de tert-butyl 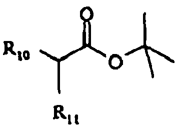 en présence d'une base telle que le LDA, pour obtenir le composé de formule (IX) suivante : 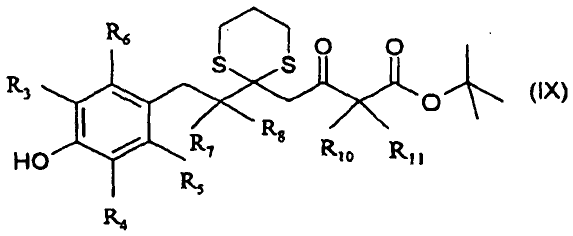 R₃ à R₈ étant tels que définis dans la revendication 1,
• réduction du composé (IX) obtenu puis réaction avec le chlorure de tripropyle silyle pour conduire au composé de formule X suivante : 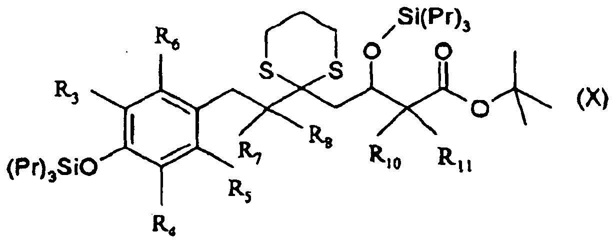
• réduction du composé (X) obtenu avec un réducteur pour conduire au composé de formule (XI) suivante : 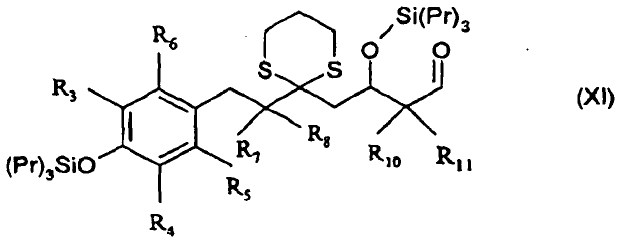
• réaction de Wittig sur le composé XI obtenu pour obtenir un composé de formule (I-Z) dans lequel W représente le groupe II'-W;
• oxydation phénolique du composé (I-Z) obtenu avec un oxydant choisi parmi le PIFA et le PIDA, afin d'obtenir un composé II dans lequel R₉ est OH et X =CH₂;
• éventuellement, réaction d'acylation ou d'alkylation du composé II dans lequel R₉ est OH et X=CH₂, pour obtenir un composé de formule générale II dans lequel X=CH₂ et R₉ représente un groupe O-allyle, un groupe O-benzyle, un groupe O-CO-alkyle, ou un groupe O-CO-cycloalkyle tels que définis dans la revendication I.

19. Procédé de préparation des composés de formule III selon la revendication 16, dans lequel le composé I-Z, est obtenu par addition d'un nucléophile par addition de type Michael sur un composé de formule III-3 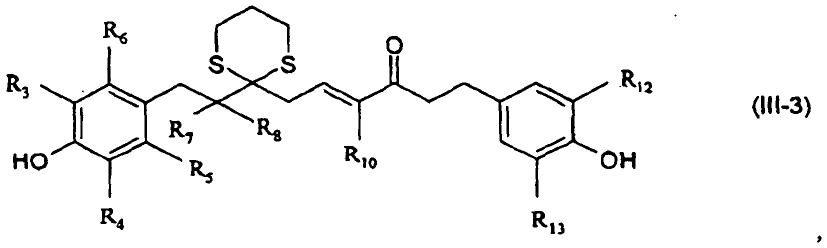 W représentant le groupe III-W et Z représentant:
• un groupe alkyle saturé ou non saturé, linéaire ou ramifié de 1 à 20 atomes de carbone, un groupe cycloalkyle de 3 à 8 atomes de carbone,
• un groupe S-R" dans lequel R" est un alkyle saturé ou non saturé, linéaire ou ramifié de 1 à 20 atomes de carbone ou un groupe cycloalkyle de 3 à 8 atomes de carbone,
• un groupe O-alkyle tel que défini dans la revendication 1, un groupe O-cycloalkyle de 3 à 8 atomes de carbone, un groupe O-CH₂-CH₂-N(CH₃)₂ ; un groupe O-CH₂-CCH,
• un groupe N(R")(R"'), R" et R"' étant tels que définis ci-dessus,
ledit composé III-3 étant obtenu par déshydratation d'un composé I-Z dans lequel Z= OH de formule (III-1) suivante: 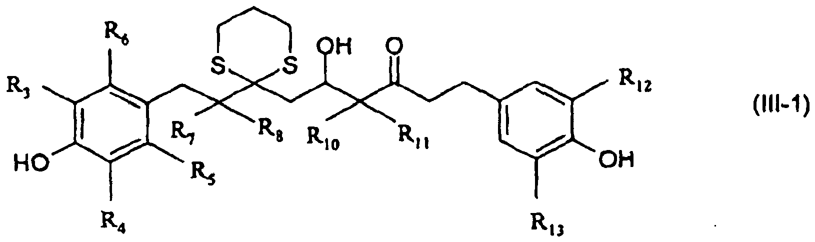 dans laquelle R₁₁ représente l'hydrogène.
R₃ à R₈, R₁₀ à R₁₃, R" et R"'étant tels que définis dans la revendication 1.

20. Procédé de préparation d'un composé de formule III selon la revendication 16 ou 19, dans lequel le composé I-Z , dans le cas où Z est un groupe OH, est obtenu par réaction d'un aldéhyde de formule (IV) suivante : 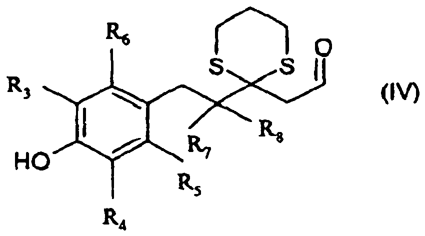 R₃ à R₈ étant tels que définis dans la revendication 1,
avec un composé de formule (VI) suivante : 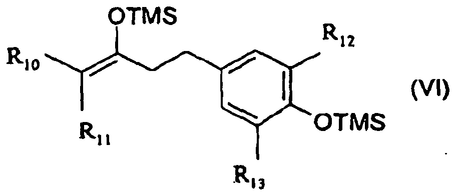 R₁₀ à R₁₃ étant tels que définis dans la revendication 1.

21. Procédé de préparation selon la revendication 16, des composés de formule III dans laquelle R₉ représente :
• un groupe OH,
un groupe O-alkyle, un groupe O-CO-alkyle, un groupe O-COCH₂CO-alkyle, ledit alkyle étant saturé ou non saturé, linéaire ou ramifié de 1 à 20 atomes de carbone, un groupe O-CO-cycloalkyle de 3 à 8 atomes de carbone, comprenant les étapes suivantes :
• réaction d'un aldéhyde de formule (IV) suivante : 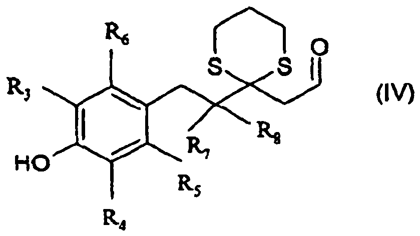 R₃ à R₈ étant tels que définis dans la revendication 1,
avec un composé de formule (VI) suivante : 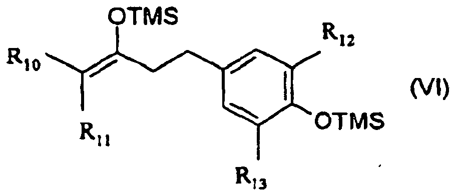 R₁₀ à R₁₃ étant tels que définis dans la revendication 1,
pour obtenir un composé I-Z dans lequel Z=OH de formule (III-1) suivante: 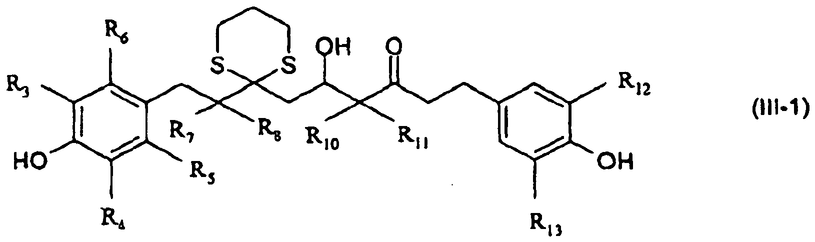
• oxydation phénolique avec un oxydant choisi parmi le PIFA et le PIDA, du composé (III-1) obtenu, afin d'obtenir un composé III dans lequel R₉ est OH,
• éventuellement, réaction d'acylation ou d'alkylation du composé III obtenu, dans lequel R₉ est un groupe OH, pour obtenir un composé de formule III désiré dans lequel R₉ est un groupe O-allyle, un groupe O-benzyle, un groupe O-CO-alkyle, un groupe OCOCH₂CO-alkyle, un groupe O-CO-cycloalkyle.

22. Procédé de préparation selon la revendication 19 ou 20, des composés de formule III où R9 est un groupe alkyle tel que défini dans la revendication 1, comprenant les étapes suivantes :
• addition d'un nucléophile CuXRa par addition de type Michael, Ra représentant un groupe alkyle tel que défini dans la revendication 1, sur le composé III-3 ci-dessus obtenu pour conduire au composé I-Z dans lequel Z=Ra de formule (III-4) suivante : 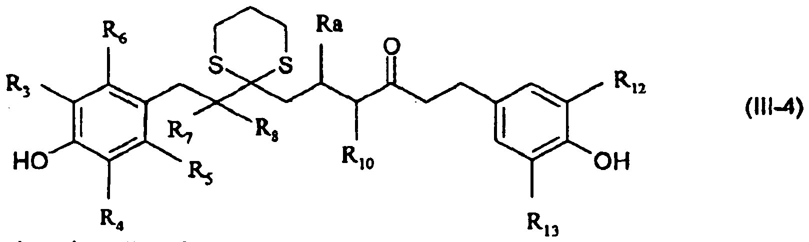 dans laquelle R₃ à R₈, R₁₀, R₁₂ et R₁₃ sont tels que définis dans la revendication 1 et Ra représente un groupe alkyle tel que défini dans la revendication 1.
• oxydation phénolique avec un oxydant choisi parmi le PIFA et le PIDA, du composé (III-4) obtenu, afin d'obtenir un composé III dans lequel R₉ est un groupe alkyle tel que défini dans la revendication 1.

23. Procédé de préparation selon la revendication 19 ou 20, des composés de formule III dans lesquels R9 est un groupe N(R")(R"'), R" et R"' étant tels que définis ci-dessus, comprenant les étapes suivantes :
• addition d'un nucléophile HN(R")(R"') par addition de type hétéro-Michael sur le composé III-3 ci-dessus obtenu pour conduire au composé 1-Z dans lequel Z=N(R")(R"') de formule (III-5) suivante : 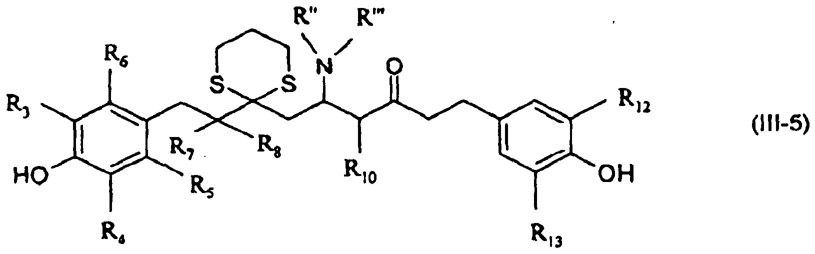 dans laquelle R₃ à R₈, R₁₀, R₁₂ et R₁₃ sont tels que définis dans la revendication 1,
• oxydation phénolique avec un oxydant choisi parmi le PIFA et le PIDA, du composé (III-5) obtenu, afin d'obtenir un composé III dans lequel R9 est un groupe N(R")(R"') tel que défini ci-dessus.

24. Procédé de préparation selon la revendication 19 ou 20, des composés de formule III dans lesquels R9 est un groupe S(O)-R", R" étant tel que défini ci-dessus, comprenant les étapes suivantes :
• addition d'un nucléophile R"SH par addition de type hétéro-Michael, R" étant tel que défini ci-dessus, sur le composé III-3 ci-dessus obtenu pour conduire au composé I-Z dans lequel Z= SR" de formule (III-6) suivante : 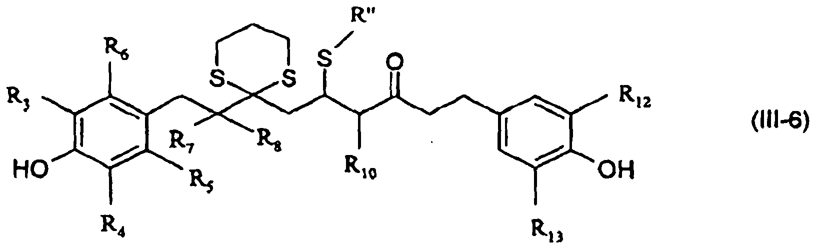 dans laquelle R₃ à R₈, R₁₀, R₁₂, R₁₃ et R"sont tels que définis dans la revendication 1.
• oxydation phénolique avec un oxydant choisi parmi le PIFA et le PIDA, du composé (III-6) obtenu, afin d'obtenir un composé III dans lequel R9 est un groupe -S(O)R", R" étant tel que défini dans la revendication 1.

25. Procédé de préparation selon la revendication 19 ou 20, des composés de formule III dans lesquels R₉ est un groupe O-alkyle tel que défini dans la revendication 1, un groupe O-CH₂-CH₂-N(CH₃)₂; un groupe O-CH₂-CCH,
un groupe 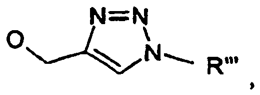 R"' étant tel que défini dans la revendication 1, comprenant les étapes suivantes :
• addition d'un nucléophile ROH par addition de type hétéro-Michael, R étant un alkyle tel que défini dans la revendication 1, un groupe -CN₂-CH₂-N(CH₃)₂ ; un groupe -CH₂-CCH, sur le composé III-3 ci-dessus obtenu pour conduire au composé I-Z dans lequel Z=OR de formule (III-2) suivante: 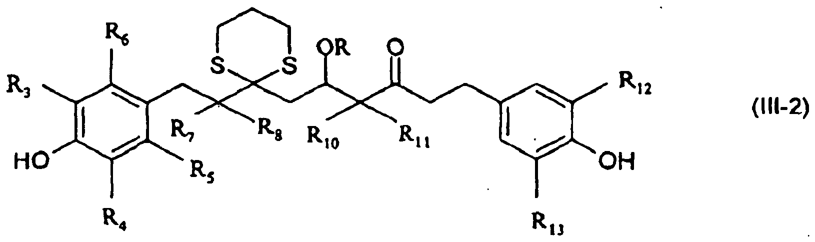 dans laquelle R₃ à R₈ et R₁₀, R₁₂ et R₁₃ sont tels que définis dans la revendication 1 et R₁₁ représente l'hydrogène,
• oxydation phénolique avec un oxydant choisi parmi le PIFA et le PIDA, du composé (III-2) obtenu, afin d'obtenir un composé **III** dans lequel R₉ est un groupe O-alkyle tel que défini dans la revendication 1, un groupe O=CH₂-CH₂-N(CH₃)₂ ; un groupe O-CH₂-CCH,
• éventuellement, réaction d'addition du composé III obtenu ci-dessus dans lequel R₉ est un groupe O-CH₂-CCH, avec N₃-R"' pour obtenir le composé de formule III désiré dans lequel R₉ est un groupe 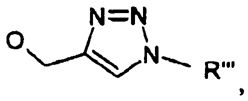 R"' étant tel que défini dans la revendication 1.

## Claims

1. Polyspirane compound of formula (I) below: 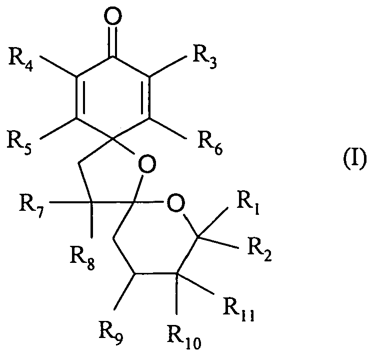 in which:
- R₁ represents hydrogen and R₂ represents a-CH₂COX-R' group, in which X= N, O, CH₂;
when X=O, R' represents a linear or branched, saturated or unsaturated alkyl of 1 to 20 carbon atoms or a cycloalkyl of 3 to 8 carbon atoms;
when X=CH₂, R' represents hydrogen, a linear or branched, saturated or unsaturated alkyl of 1 to 20 carbon atoms or a cycloalkyl of 3 to 8 carbon atoms;
when X=N, N can be monosubstituted by R' or disubstituted by R' and R" which represent, independently of each other, hydrogen or a linear or branched, saturated or unsaturated alkyl of 1 to 20 carbon atoms or a cycloalkyl of 3 to 8 carbon atoms;
R₉ represents:
• an OH group,
• an O-allyl group, an O-benzyl group, an O-CO-alkyl group, an O-COCH₂CO-alkyl group, said alkyl residue being saturated or unsaturated, linear or branched with 1 to 20 carbon atoms, or an OCO-cycloalkyl group of 3 to 8 carbon atoms,
or,
- R₁ and R₂ together represent a G-1 group: 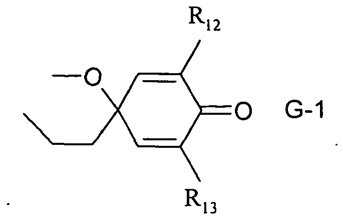 R₁₂ and R₁₃ represent independently of each other hydrogen, a linear or branched, saturated or unsaturated alkyl group of 1 to 6 carbon atoms, a cycloalkyl group of 3 to 8 carbon atoms, a linear or branched, saturated or unsaturated, O-alkyl group of 1 to 6 carbon atoms, an O-cycloalkyl group of 3 to 8 carbon atoms, a halogen atom;
R₉ represents:
• an OH group,
• a linear or branched, saturated or unsaturated alkyl group of 1 to 20 carbon atoms, a cycloalkyl group of 3 to 8 carbon atoms,
• an S(O)-R" group in which R" is a linear or branched, saturated or unsaturated alkyl of 1 to 20 carbon atoms or a cycloalkyl group of 3 to 8 carbon atoms,
• an O-alkyl group, an O-CO-alkyl group, an O-COCH₂CO-alkyl group, said alkyl residue being saturated or unsaturated, linear or branched with 1 to 20 carbon atoms, an O-cycloalkyl group of 3 to 8 carbon atoms, an O-CH₂-CH₂-N(CH₃)₂ group; a group O-CH₂-CCH,
• an 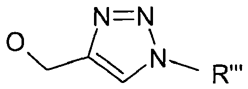 group in which R"' is a linear or branched, saturated or unsaturated alkyl of 1 to 20 carbon atoms,
• an N(R")(R"') group, R" and R'" being as defined above,
and whatever R₁ and R₂ may be:
- R₃, R₄, R₅, R₆ represent independently of each other hydrogen, a linear or branched, saturated or unsaturated alkyl group of 1 to 6 carbon atoms, a cycloalkyl group of 3 to 8 carbon atoms, a linear or branched, saturated or unsaturated, O-alkyl group of 1 to 6 carbon atoms, an O-cycloalkyl group of 3 to 8 carbon atoms, a halogen atom;
- R₇ and R₈ represent independently of each other hydrogen, a linear or branched, saturated or unsaturated alkyl group of 1 to 6 carbon atoms, a cycloalkyl group of 3 to 8 carbon atoms, a linear or branched, saturated or unsaturated, O-alkyl group of 1 to 6 carbon atoms, an O-cycloalkyl group of 3 to 8 carbon atoms, a linear or branched, saturated or unsaturated NH-alkyl group of 1 to 6 carbon atoms, an NH-cycloalkyl group of 3 to 8 carbon atoms, an NHCO-R group, in which R can be a linear or branched, saturated or unsaturated alkyl group of 1 to 6 carbon atoms, a cycloalkyl group of 3 to 8 carbon atoms,
- R₁₀ and R₁₁ represent independently of each other hydrogen, a hydroxyl group, an O-alkyl group, the alkyl residue being saturated or unsaturated, linear or branched with 1 to 8 carbon atoms, an O-cycloalkyl group of 3 to 8 carbon atoms, a tertiary amine.

2. Polyspirane compound according to claim 1, of general formula (II) below: 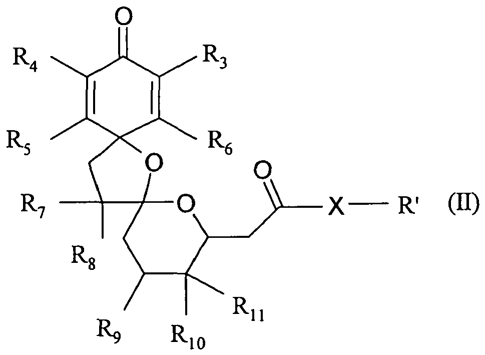 in which R₃ to R₈, R₁₀ to R₁₁, X and R' are as defined in claim 7 and, R₉ represents an OH group, an O-allyl group, an O-benzyl group, an O-CO-alkyl group, said alkyl being saturated or unsaturated, linear or branched with 1 to 20 carbon atoms, or an O-CO-cycloalkyl group comprising 3 to 8 carbon atoms.

3. Polyspirane compound according to claim 2, of general formula (III) below: 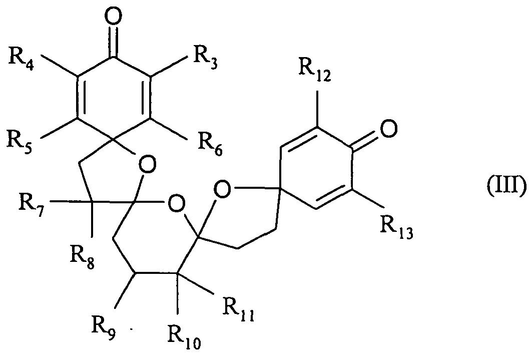 in which R₃ to R₈ and R₁₀ to R₁₃ are as defined in claim 7 when
R₁ and R₂ form together a G-1 group, and
R₉ represents:
• an OH group,
• a linear or branched, saturated or unsaturated alkyl group of 1 to 20 carbon atoms, a cycloalkyl group of 3 to 8 carbon atoms,
• an S(O)-R" group in which R" is a linear or branched, saturated or unsaturated alkyl of 1 to 20 carbon atoms or a cycloalkyl group of 3 to 8 carbon atoms,
• an O-alkyl group, an O-CO-alkyl group, an O-COCH₂CO-alkyl group, said alkyl residue being saturated or unsaturated, linear or branched with 1 to 20 carbon atoms, an O-cycloalkyl group of 3 to 8 carbon atoms, an O-CH₂-CH₂-N(CH₃)₂ group; an O-CH₂-CCH group,
• an 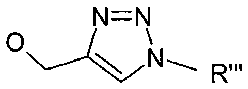 group in which R"' is a linear or branched, saturated or unsaturated alkyl of 1 to 20 carbon atoms,
an N(R")(R"') group, R" and R'" being as defined above.

4. Polyspirane compound of general formula (II) according to claim 2, in which R₃ to R₈ and R₁₀ to R₁₁ represent hydrogen, R₉ represents OH, X= O and R' is a linear, saturated alkyl with 3 carbon atoms, 10 carbon atoms or 18 carbon atoms.

5. Polyspirane compound of general formula (III) according to claim 3, in which:
R₃ to R₈ and R₁₀ to R₁₃ represent the hydrogen,
R₉ is an OH group, or O-CO-alkyl in which the alkyl is linear and has 7 carbon atoms, 14 carbon atoms or 16 carbon atoms.

6. Polyspirane compound of general formula (III) according to claim 5, constituted by a mixture of (8R, 10S, 21 S) and (8S, 10R, 21 R) enantiomers.

7. Polyspirane compound of general formula (III) according to claim 5, constituted by the (8R, 10R, 21S) enantiomer, or by the (8*R*, 10*S*, 21*S*) enantiomer or also by the (8*S*, 10*R*, 21*R*) enantiomer.

8. Use of at least one polyspirane compound, or physiologically acceptable salts thereof, of formula (I), such as defined in claim 1, for the preparation of a medicament intended for the prevention or treatment of pathologies involving the parasites belonging to the Apicomplexa phylum.

9. Use of at least one polyspirane compound according to claim 8, of general formula (II), such as defined in claim 2.

10. Use of at least one polyspirane compound according to claim 8, of general formula (III), such as defined in claim 3.

11. Use of at least one polyspirane compound according to one of claims 8 to 10, in which said parasites are species of *Plasmodium.*

12. Use of at least one polyspirane compound according to one of claims 8 to 10, in which said parasites are of the genus *Toxoplasma.*

13. Use of at least one compound of polyspirane formula according to claim 12, in which said disorders are malaria or toxoplasmosis.

14. Pharmaceutical composition comprising as active ingredient at least one polyspirane compound of general formula I, such as defined in claim 1, in combination with a pharmaceutically acceptable excipient.

15. Pharmaceutical composition according to claim 14, being presented in a form which can be administered by oral route at a rate of from 0.1 mg/kg/d to 100 mg/kg/d of active ingredient.

16. Method for preparing the compounds of formula I:
comprising a stage of phenolic oxidation with an oxidizing agent chosen among PIFA or PIDA, of a compound of formula (I-Z) below: 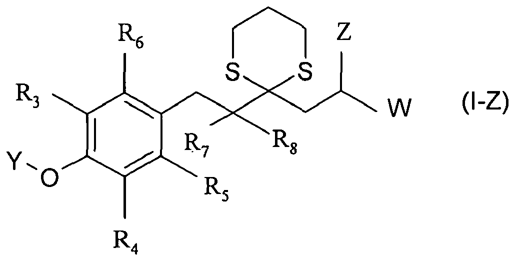 in which:
- either W represents a group of formula (II-W) below: 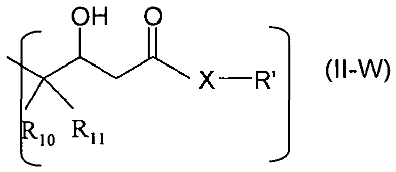 and X = O or N,
Y represents hydrogen,
and Z represents:
• an OH group,
R₃ to R₈, R₁₀, R₁₁, X, R' and R"' being as defined in claim 1,
in order to obtain a compound of general formula II in which X = O or N and R9 represents an OH group,
and then optionally comprising a stage of acylation or alkylation in order to obtain a compound of general formula II in which R₉ is an O-CO-alkyl group, an O-allyl group, an O-benzyl group, an O-COCH₂CO-alkyl group, said alkyl residue being saturated or unsaturated, linear or branched with 1 to 20 carbon atoms, or an O-CO-cycloalkyl group comprising 3 to 8 carbon atoms,
- or W represents a group of formula II'-W below: 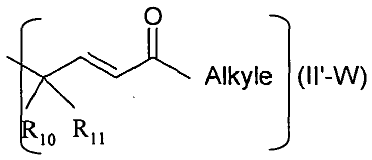 said alkyl being saturated or unsaturated, linear or branched with 1 to 17 carbon atoms or a cycloalkyl of 3 to 8 carbon atoms, and X = CH₂;
Y represents a (Pr₃)Si- group,
and Z represents a (Pr₃)Si-O- group,
in order to obtain a compound of general formula II in which X = CH₂ and R9 represents an OH group,
and then optionally comprising a stage of acylation or alkylation in order to obtain a compound of general formula II in which R₉ is an O-CO-alkyl group, an O-allyl group, an O-benzyl group, an O-COCH₂CO-alkyl group, said alkyl residue being saturated or unsaturated, linear or branched with 1 to 20 carbon atoms, or an O-CO-cycloalkyl group comprising 3 to 8 carbon atoms,
- or W represents a III-W group: 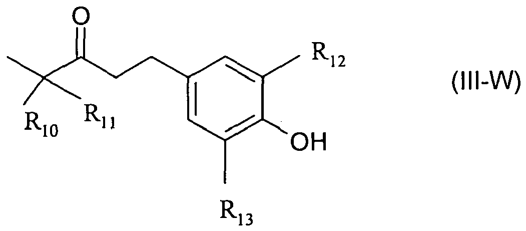 and Z represents:
• an OH group,
• a linear or branched, saturated or unsaturated alkyl group of 1 to 20 carbon atoms, a cycloalkyl group of 3 to 8 carbon atoms,
• a group S-R" in which R" is a linear or branched, saturated or unsaturated alkyl of 1 to 20 carbon atoms or a cycloalkyl group of 3 to 8 carbon atoms,
• an O-alkyl group, the alkyl being saturated or unsaturated, linear or branched with 1 to 20 carbon atoms, an O-cycloalkyl group of 3 to 8 carbon atoms, an O-CH₂-CH₂-N(CH₃)₂ group; an O-CH₂-CCH group,
• an N(R")(R"') group, R" and R"' being as defined above,
R₃ to R₈ and R₁₀ to R₁₃ being as defined in claim 1,
in order to obtain a compound of general formula III in which R9 represents:
• an OH group,
• a linear or branched, saturated or unsaturated alkyl group of 1 to 20 carbon atoms, a cycloalkyl group of 3 to 8 carbon atoms,
• an S(O)-R" group in which R" is a linear or branched, saturated or unsaturated alkyl of 1 to 20 carbon atoms or a cycloalkyl group of 3 to 8 carbon atoms,
• an O-alkyl group, the alkyl being saturated or unsaturated, linear or branched with 1 to 20 carbon atoms, an O-cycloalkyl group of 3 to 8 carbon atoms, an O-CH₂-CH₂-N(CH₃)₂ group; an O-CH₂-CCH group,
• an N(R")(R"') group, R" and R'" being as defined above,
and, when Z is an OH group (compound III-1), optionally comprising a stage of acylation or alkylation in order to obtain a compound of general formula III in which R₉ is an O-alkyl group, an O-cycloalkyl group, an O-CO-alkyl group, an O-COCH₂CO-alkyl group, an O-CH₂-CH₂-N(CH₃)₂ group; an O-CH₂-CCH group,
and/or, when Z represents an -O-CH₂-CCH group, optionally comprising an addition reaction with N₃-R"' in order to obtain a compound of general formula III in which R₉ is an 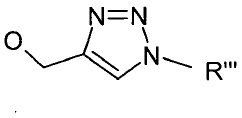 group, R"' being as defined in claim 1.

17. Method for preparing the compounds of formula II according to claim 16, in which X = O, N and comprising the following stages:
• Reaction of an aldehyde of formula (IV) below: 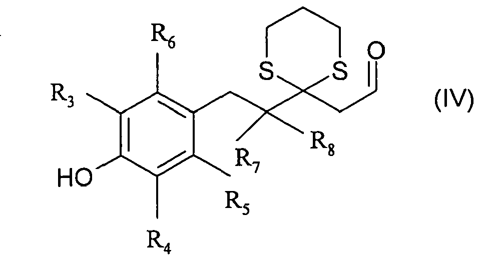 R₃ to R₈ being as defined in claim 1,
with a compound of formula VII below: 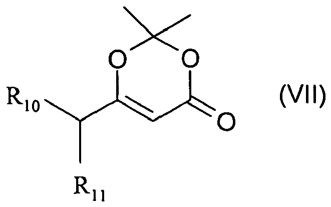 in order to obtain the compound of formula (V) below: 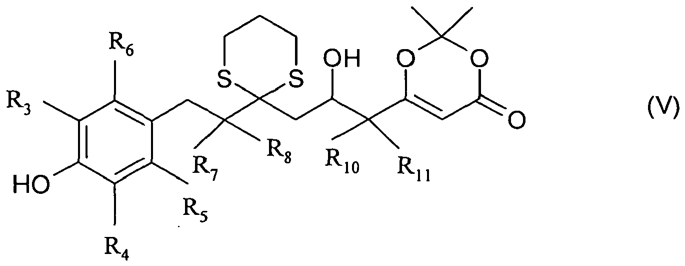 R₃ to R₈ and R₁₀ to R₁₁ being as defined in claim 1,
• reaction of compound (V) obtained with a compound R'-X-H, (X= 0) or (R')(R")-X-H (X=N), R' and R" being as defined in claim 1, then reduction with a reducing agent, chosen among NaBH₄ or NaBH₄ and Et₃B, or Me₄NBH(OAc)₃, in order to obtain a compound of formula (I-Z) in which W represents the II-W group;
• phenolic oxidation of the compound (I-Z) obtained with an oxidizing agent chosen among PIFA and PIDA, in order to obtain a compound II in which R₉ is OH;
• optionally, acylation or alkylation reaction of compound II in which R₉ is OH, in order to obtain a compound of general formula II in which R9 represents an O-allyl group, an O-benzyl group, an O-CO-alkyl group, or an O-CO-cycloalkyl group as defined in claim 1.

18. Method for preparing the compounds of formula II according to claim 16, in which X = CH₂ and comprising the following stages:
• reaction of an ester of formula (VIII) below: 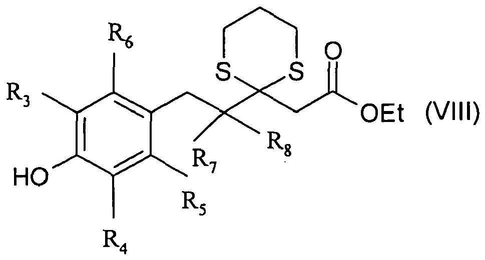 R₃ to R₈ being as defined in claim 1,
with an acetate of tert-butyl 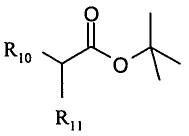 in the presence of a base such as LDA, in order to obtain the compound of formula (IX) below: 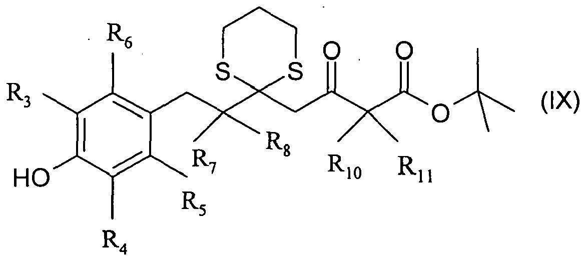 R₃ to R₈ being as defined in claim 1,
• reduction of the compound (IX) obtained then reaction with the tripropyl silyl chloride in order to produce the compound of formula X below: 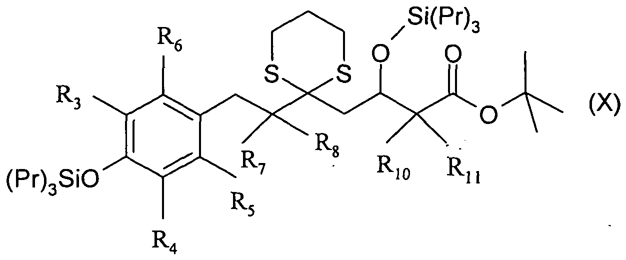
• reduction of compound (X) obtained with a reducing agent in order to produce the compound of formula (XI) below: 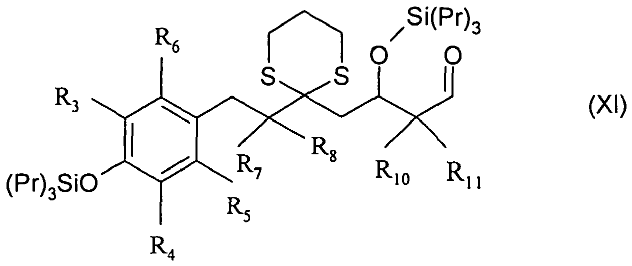
• Wittig reaction on the compound XI obtained in order to obtain a compound of formula (I-Z) in which W represents the II'-W group;
• phenolic oxidation of compound (I-Z) obtained with an oxidizing agent chosen among PIFA and PIDA, in order to obtain a compound II in which R₉ is OH and X = CH₂;
• optionally, acylation or alkylation reaction of compound II in which R₉ is OH and X= CH₂, in order to obtain a compound of general formula II in which X= CH₂ and R₉ represents an O-allyl group, an O-benzyl group, an O-CO-alkyl group, or an O-CO-cycloalkyl group as defined in claim 1.

19. Method for preparing the compounds of formula III according to claim 16, in which the compound I-Z is obtained by adding a nucleophile by Michael-type addition to a compound of formula III-3 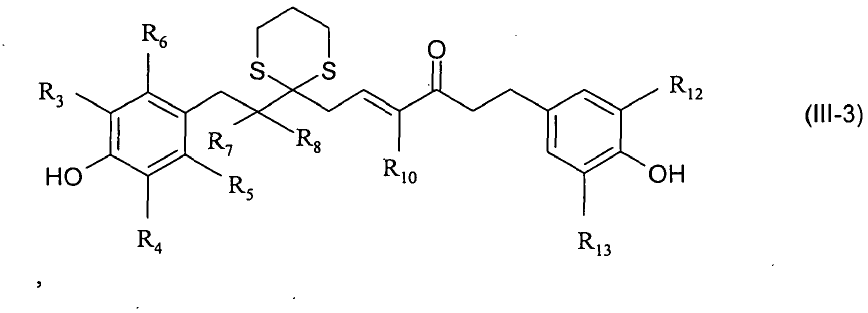 W representing the III-W group and Z representing:
• a linear or branched, saturated or unsaturated alkyl group of 1 to 20 carbon atoms, a cycloalkyl group of 3 to 8 carbon atoms,
• an S-R" group in which R" is a linear or branched, saturated or unsaturated alkyl of 1 to 20 carbon atoms or a cycloalkyl group of 3 to 8 carbon atoms,
• an O-alkyl group as defined in claim 1, an O-cycloalkyl group of 3 to 8 carbon atoms, an O-CH₂-CH₂-N(CH₃)₂ group;
an O-CH₂-CCH group,
• an N(R")(R"') group, R" and R"' being as defined above,
said compound III-3 being obtained by dehydration of a compound I-Z in which Z= OH of formula (III-1) below: 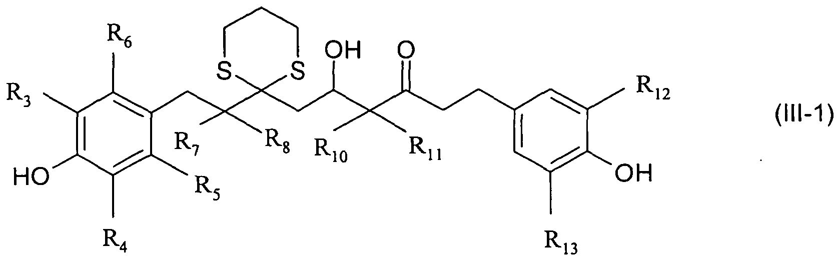 in which R₁₁ represents hydrogen.
R₃ to R₈, R₁₀ to R₁₃, R" and R"'being as defined in claim 1.

20. Method for preparing a compound of formula III according to claim 16 or 19, in which the compound I-Z, in the case where Z is an OH group, is obtained by reaction of an aldehyde of formula (IV) below: 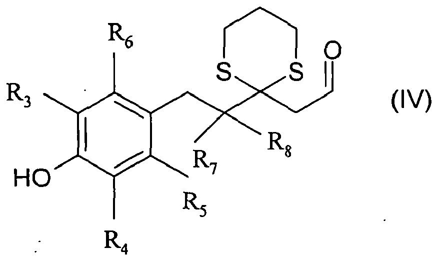 R₃ to R₈ being as defined in claim 1,
with a compound of formula (VI) below: 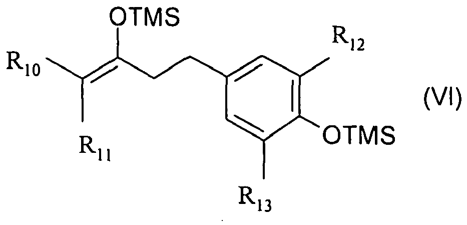 R₁₀ to R₁₃ being as defined in claim 1.

21. Method according to claim 16, for preparing compounds of formula III in which R₉ represents:
• an OH group,
an O-alkyl group, an O-CO-alkyl group, an O-COCH₂CO-alkyl group, said alkyl being saturated or unsaturated, linear or branched with 1 to 20 carbon atoms, an O-CO-cycloalkyl group of 3 to 8 carbon atoms, comprising the following stages:
• reaction of an aldehyde of formula (IV) below: 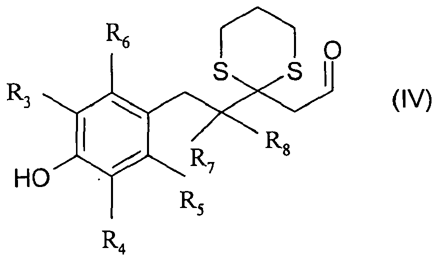 R₃ to R₈ being as defined in claim 1,
with a compound of formula (VI) below: 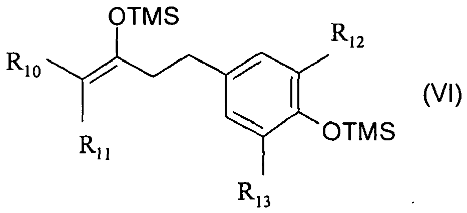 R₁₀ to R₁₃ being as defined in claim 1,
in order to obtain a compound I-Z in which Z=OH of formula (III-1) below: 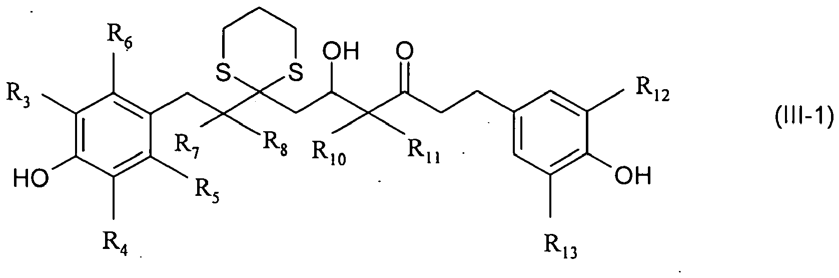
• phenolic oxidation with an oxidizing agent chosen among PIFA and PIDA, of the compound (III-1) obtained, in order to obtain a compound III in which R₉ is OH,
• optionally, acylation or alkylation reaction of compound III obtained, in which R₉ is an OH group, in order to obtain a desired compound of formula III in which R₉ is an O-allyl group, an O-benzyl group, an O-CO-alkyl group, an O-COCH₂CO-alkyl group, an O-CO-cycloalkyl group.

22. Method according to claim 19 or 20, for preparing compounds of formula III where R9 is an alkyl group as defined in claim 1, comprising the following stages:
• addition of a CuXRa nucleophile by Michael-type addition, Ra representing an alkyl group as defined in claim 1, to the above compound III-3 obtained in order to produce the compound I-Z in which Z=Ra of formula (III-4) below: 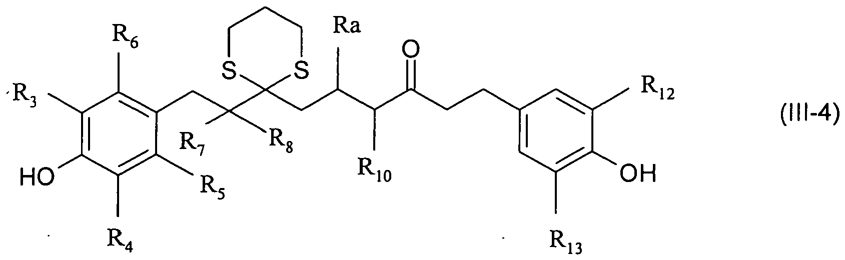 in which R₃ to R₈, R₁₀, R₁₂ and R₁₃ are as defined in claim 1 and Ra represents an alkyl group as defined in claim 1.
• phenolic oxidation with an oxidizing agent chosen among PIFA and PIDA, of the compound (III-4) obtained, in order to obtain a compound III in which R₉ is an alkyl group as defined in claim 1.

23. Method according to claim 19 or 20, for preparing compounds of formula III in which R9 is an N(R")(R"') group, R" and R"' being as defined above, comprising the following stages:
• addition of an HN(R")(R"') nucleophile by hetero-Michael type addition to the above compound III-3 obtained in order to produce the compound I-Z in which Z=N(R")(R"') of formula (III-5) below: 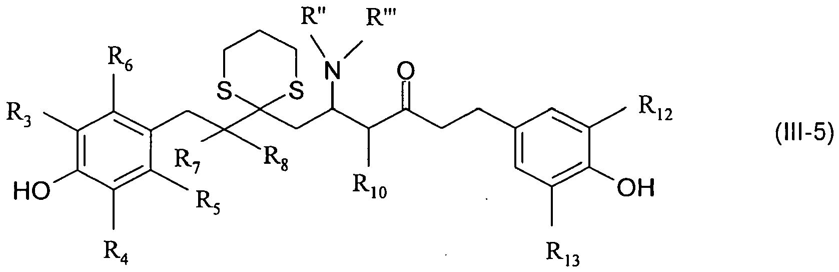 in which R₃ to R₈, R₁₀, R₁₂ and R₁₃ are as defined in claim 1,
• phenolic oxidation with an oxidizing agent chosen among PIFA and PIDA, of the compound (III-5) obtained, in order to obtain a compound III in which R9 is an N(R")(R"') group as defined above.

24. Method according to claim 19 or 20, for preparing compounds of formula III in which R9 is an S(O)-R" group, R" being as defined above, comprising the following stages:
• addition of an R"SH nucleophile by hetero-Michael type addition, R" being as defined above, to the compound III-3 obtained above in order to produce compound I-Z in which Z= SR" of formula (III-6) below: 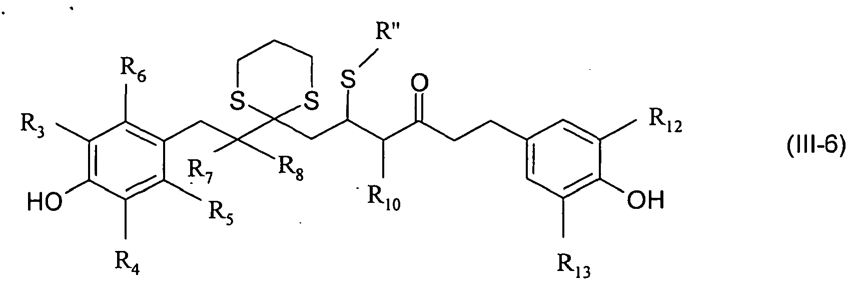 in which R₃ to R₈, R₁₀, R₁₂, R₁₃ and R"are as defined in claim 1.
• phenolic oxidation with an oxidizing agent chosen among PIFA and PIDA, of the obtained compound (III-6), in order to obtain a compound III in which R9 is an -S(O)R" group, R" being as defined in claim 1.

25. Method according to claim 19 or 20, for preparing compounds of formula III in which R₉ is an O-alkyl group as defined in claim 1, an O-CH₂-CH₂-N(CH₃)₂ group; an O-CH₂-CCH group,
an 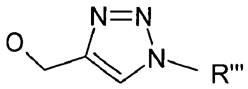 group, R"' being as defined in claim 1, comprising the following stages:
• addition of an ROH nucleophile by hetero-Michael type addition, R being an alkyl as defined in claim 1, an -CH₂-CH₂-N(CH₃)₂ group; a -CH₂-CCH group, to the compound III-3 obtained above in order to produce compound I-Z in which Z=OR of formula (III-2) below: 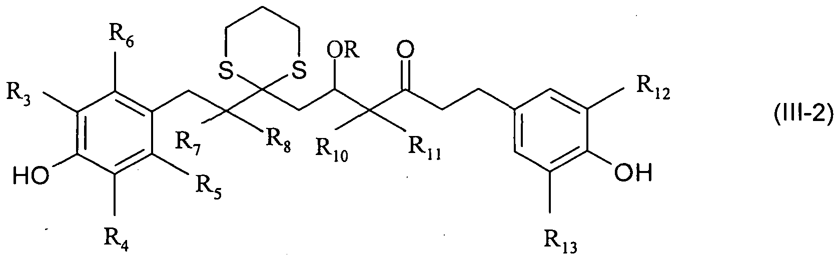 in which R₃ to R₈ and R₁₀, R₁₂ and R₁₃ are as defined in claim 1 and R₁₁ represents hydrogen,
• phenolic oxidation with an oxidizing agent chosen among PIFA and PIDA, of compound (III-2) obtained, in order to obtain a compound III in which R₉ is an O-alkyl group as defined in claim 1, a group O-CH₂-CH₂-N(CH₃)₂; a group O-CH₂-CCH,
• optionally, addition reaction of compound III obtained above in which R₉ is an O-CH₂-CCH group, with N₃-R"' in order to obtain the desired compound of formula III in which R₉ is a 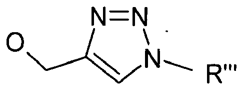 group, R"' being as defined in claim 1.

## Patentansprüche

1. Polyspiranverbindung nach der folgenden Formel (I): 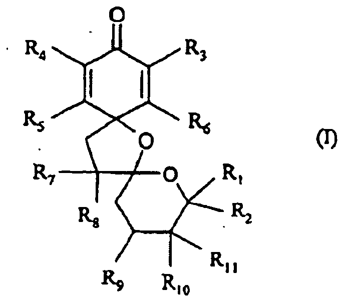 wobei:
- R₁ für Wasserstoff steht und R₂ für eine Gruppe - CH₂COX-R' steht, wobei X = N, O, CH₂;
wenn X = O, steht R' für ein gesättigtes oder ungesättigtes Alkyl, das geradkettig oder verzweigt mit 1 bis 20 Kohlenstoffatomen ist, oder für ein Cycloalkyl mit 3 bis 8 Kohlenstoffatomen;
wenn X = CH₂, steht R' für Wasserstoff, für ein gesättigtes oder ungesättigtes Alkyl, das geradkettig oder verzweigt mit 1 bis 20 Kohlenstoffatomen ist, oder für ein Cycloalkyl mit 3 bis 8 Kohlenstoffatomen;
wenn X = N, kann N mit R' einfach substituiert oder mit R' und R" zweifach substituiert sein, wobei diese unabhängig voneinander für Wasserstoff oder für ein gesättigtes oder ungesättigtes Alkyl, das geradkettig oder verzweigt mit 1 bis 20 Kohlenstoffatomen ist, oder für ein Cycloalkyl mit 3 bis 8 Kohlenstoffatomen stehen.
R₉ für folgendes steht:
• eine OH-Gruppe,
• eine O-Allylgruppe, eine O-Benzylgruppe, eine O-CO-Alkylgruppe, eine O-COCH₂CO-Alkylgruppe, wobei der Alkylrest gesättigt oder ungesättigt, geradkettig oder verzweigt mit 1 bis 20 Kohlenstoffatomen ist, oder eine OCO-Cycloalkylgruppe mit 3 bis 8 Kohlenstoffatomen,
oder
- R₁ und R₂ gemeinsam eine Gruppe G-1 bilden: 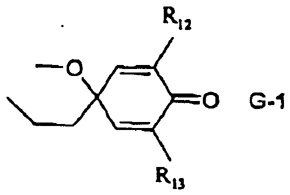 R₁₂ und R₁₃ unabhängig voneinander für Wasserstoff, für eine gesättigte oder ungesättigte Alkylgruppe, die geradkettig oder verzweigt mit 1 bis 6 Kohlenstoffatomen ist, für eine Cycloalkylgruppe mit 3 bis 8 Kohlenstoffatomen, für eine gesättigte oder ungesättigte O-Alkylgruppe, die geradkettig oder verzweigt mit 1 bis 6 Kohlenstoffatomen ist, für eine O-Cycloalkylgruppe mit 3 bis 8 Kohlenstoffatomen, für ein Halogenatom stehen;
R₉ für folgendes steht:
• eine OH-Gruppe,
• eine gesättigte oder ungesättigte Alkylgruppe, die geradkettig oder verzweigt mit 1 bis 20 Kohlenstoffatomen ist, eine Cycloalkylgruppe mit 3 bis 8 Kohlenstoffatomen;
• eine Gruppe S-(O)-R", wobei R" ein gesättigtes oder ungesättigtes Alkyl ist, das geradkettig oder verzweigt mit 1 bis 20 Kohlenstoffatomen ist, oder ein Cycloalkyl mit 3 bis 8 Kohlenstoffatomen,
• eine O-Alkylgruppe, eine O-CO-Alkylgruppe, eine O-COCH₂CO-Alkylgruppe, wobei der Alkylrest gesättigt oder ungesättigt, geradkettig oder verzweigt mit 1 bis 20 Kohlenstoffatomen ist, oder eine O-Cycloalkylgruppe mit 3 bis 8 Kohlenstoffatomen, eine Gruppe O-CH₂-CH₂-N(CH₃)₂; eine Gruppe O-CH₂-CCH,
• eine Gruppe 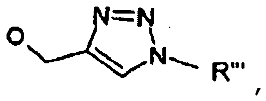 wobei R"' ein gesättigtes oder ungesättigtes Alkyl ist, das geradkettig oder verzweigt mit 1 bis 20 Kohlenstoffatomen ist,
• eine Gruppe N(R")(R"'), wobei R" und R"' den obigen Begriffsbestimmungen entsprechen,
und wobei unabhängig von der Beschaffenheit von R₁ und R₂:
- R₃, R₄, R₅, R₆ unabhängig voneinander für Wasserstoff, für eine gesättigte oder ungesättigte Alkylgruppe, die geradkettig oder verzweigt mit 1 bis 6 Kohlenstoffatomen ist, für eine Cycloalkylgruppe mit 3 bis 8 Kohlenstoffatomen, für eine gesättigte oder ungesättigte O-Alkylgruppe, die geradkettig oder verzweigt mit 1 bis 6 Kohlenstoffatomen ist, für eine O-Cycloalkylgruppe mit 3 bis 8 Kohlenstoffatomen, für ein Halogenatom stehen;
- R₇ und R₈ unabhängig voneinander für Wasserstoff, für eine gesättigte oder ungesättigte Alkylgruppe, die geradkettig oder verzweigt mit 1 bis 6 Kohlenstoffatomen ist, für eine Cycloalkylgruppe mit 3 bis 8 Kohlenstoffatomen, für eine gesättigte oder ungesättigte O-Alkylgruppe, die geradkettig oder verzweigt mit 1 bis 6 Kohlenstoffatomen ist, für eine O-Cycloalkylgruppe mit 3 bis 8 Kohlenstoffatomen, für eine gesättigte oder ungesättigte NH-Alkylgruppe, die geradkettig oder verzweigt mit 1 bis 6 Kohlenstoffatomen ist, für eine NH-Cycloalkylgruppe mit 3 bis 8 Kohlenstoffatomen, für eine NHCO-R-Gruppe steht, wobei R eine gesättigte oder ungesättigte Alkylgruppe, die geradkettig oder verzweigt mit 1 bis 6 Kohlenstoffatomen ist, eine Cycloalkylgruppe mit 3 bis 8 Kohlenstoffatomen sein kann,
- R₁₀ und R₁₁ unabhängig voneinander für Wasserstoff, für eine Hydroxylgruppe, für eine für O-Alkylgruppe, wobei der Alkylrest gesättigt oder ungesättigte, geradkettig oder verzweigt mit 1 bis 8 Kohlenstoffatomen ist, für eine O-Cycloalkylgruppe mit 3 bis 8 Kohlenstoffatomen, für ein tertiäres Amin stehen.

2. Polyspiranverbindung nach Anspruch 1, nach der folgenden allgemeinen Formel (II): 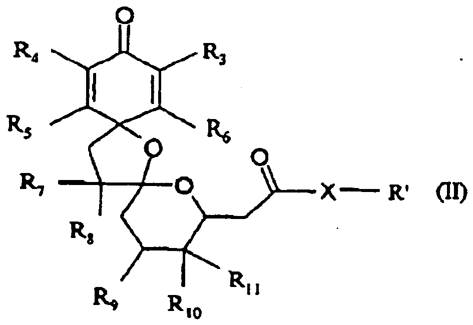 wobei R₃ bis R₈, R₁₀ bis R₁₁, X und R' den Begriffsbestimmungen in Anspruch 1 entsprechen und R₉ für eine OH-Gruppe, eine O-Allylgruppe, eine O-Benzylgruppe, eine O-CO-Alkylgruppe, wobei das Alkyl gesättigt oder ungesättigt, geradkettig oder verzweigt mit 1 bis 20 Kohlenstoffatomen ist, oder für eine C-CO-Cycloalkylgruppe, die 3 bis 8 Kohlenstoffatome aufweist, steht.

3. Polyspiranverbindung nach Anspruch 2, nach der folgenden allgemeinen Formel (III): 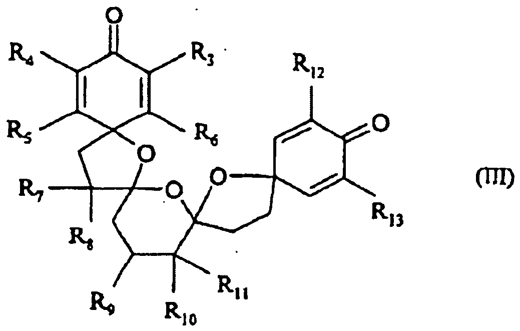 wobei R₃ bis R₈ und R₁₀ bis R₁₃ den Begriffsbestimmungen in Anspruch 1 entsprechen, wenn R₁ und R₂ gemeinsam eine Gruppe G-1 bilden, und
R₉ für folgendes steht:
• eine OH-Gruppe,
• eine gesättigte oder ungesättigte Alkylgruppe, die geradkettig oder verzweigt mit 1 bis 20 Kohlenstoffatomen ist, eine Cycloalkylgruppe mit 3 bis 8 Kohlenstoffatomen;
• eine Gruppe S-(O)-R", wobei R" ein gesättigtes oder ungesättigtes Alkyl ist, das geradkettig oder verzweigt mit 1 bis 20 Kohlenstoffatomen ist, oder eine Cycloalkylgruppe mit 3 bis 8 Kohlenstoffatomen,
• eine O-Alkylgruppe, eine O-CO-Alkylgruppe, eine O-COCH₂CO-Alkylgruppe, wobei der Alkylrest gesättigt oder ungesättigt, geradkettig oder verzweigt mit 1 bis 20 Kohlenstoffatomen ist, oder eine O-Cycloalkylgruppe mit 3 bis 8 Kohlenstoffatomen, eine Gruppe O-CH₂-CH₂-N(CH₃)₂; eine Gruppe O-CH₂-CCH,
• eine Gruppe 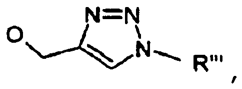 wobei R"' ein gesättigtes oder ungesättigtes Alkyl ist, das geradkettig oder verzweigt mit 1 bis 20 Kohlenstoffatomen ist,
eine Gruppe N(R")(R"'), wobei R" und R"' den obigen Begriffsbestimmungen entsprechen.

4. Polyspiranverbindung der Formel allgemeinen (II) gemäß Anspruch 2, wobei R₃ bis R₈ und R₁₀ bis R₁₁ für Wasserstoff stehen, R₉ für OH steht, X = O ist und R' ein gesättigtes geradkettiges Alkyl mit 3 Kohlenstoffatomen, mit 10 Kohlenstoffatomen oder mit 18 Kohlenstoffatomen ist.

5. Polyspiranverbindung der folgenden allgemeinen Formel (III) gemäß Anspruch 3, wobei:
R₃ bis R₈ und R₁₀ bis R₁₃ für Wasserstoff stehen,
R₉ eine OH- oder O-CO-Alkylgruppe ist, wobei das Alkyl geradkettig ist und 7 Kohlenstoffatome, 14 Kohlenstoffatome oder 16 Kohlenstoffatome besitzt.

6. Polyspiranverbindung der folgenden allgemeinen Formel (III) gemäß Anspruch 5, welche aus einer Mischung von Enantiomeren (8R, 10S, 21S) und (8S, 10R, 21R) besteht.

7. Polyspiranverbindung der folgenden allgemeinen Formel (III) gemäß Anspruch 5, welche aus dem Enantiomer (8R, 10R, 21S) oder dem Enantiomer (8R, 10S, 21S) oder auch dem Enantiomer (8S, 10R, 21R) besteht.

8. Verwendung mindestens einer Polyspiranverbindung, oder der physiologisch unbedenklichen Salze derselben, nach der Formel (I) entsprechend den Begriffsbestimmungen in Anspruch 1, zur Herstellung eines Arzneimittels, das zur Verhütung oder zur Behandlung von Erkrankungen bestimmt ist, an denen Parasiten beteiligt sind, die zum Stamm der Sporentierchen gehören.

9. Verwendung mindestens einer Polyspiranverbindung gemäß Anspruch 8, nach der allgemeinen Formel (II), entsprechend der Begriffsbestimmung in Anspruch 2,

10. Verwendung mindestens einer Polyspiranverbindung gemäß Anspruch 8, nach der allgemeinen Formel (III), entsprechend der Begriffsbestimmung in Anspruch 3.

11. Verwendung mindestens einer Polyspiranverbindung gemäß einem der Ansprüche 8 bis 10, wobei die Parasiten von der Art *Plasmodium* sind.

12. Verwendung mindestens einer Polyspiranverbindung gemäß einem der Ansprüche 8 bis 10, wobei die Parasiten von der Gattung *Toxoplasma* sind.

13. Verwendung mindestens einer Verbindung mit Polyspiranformel gemäß Anspruch 12, wobei es sich bei den Störungen um Malaria oder Toxoplasmose handelt.

14. Pharmazeutische Zusammensetzung, die als Wirkstoff mindestens eine Polyspiranverbindung nach der allgemeinen Formel I entsprechend der Begriffsbestimmung in 1 umfasst, in Verbindung mit einem pharmazeutisch unbedenklichen Träger.

15. Pharmazeutische Zusammensetzung nach Anspruch 14, die derart vorliegt, dass sie oral in Mengen von 0,1 mg/kg/Tag bis 100 mg/kg/Tag an Wirkstoff, verabreicht werden kann.

16. Verfahren zur Herstellung der Verbindungen nach Formel I:
umfassend einen Schritt der oxidativen Phenolkupplung einer Verbindung der folgenden Formel (I-Z) mit einem Oxidationsmittel, das aus PIFA oder PIDA ausgewählt ist: 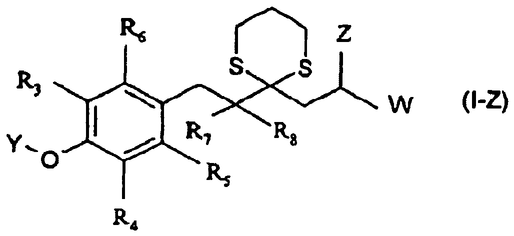
wobei:
- entweder W für eine Gruppe der folgenden Formel (II-W) steht: 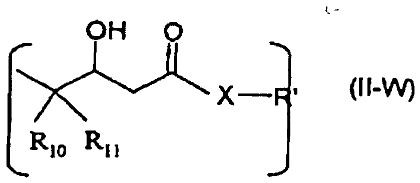 und X = O oder N ist,
Y für Wasserstoff steht,
und Z für Folgendes steht:
• eine OH-Gruppe,
wobei R₃ bis R₈, R₁₀, R₁₁, X, R' und R"' den Begriffsbestimmungen in Anspruch 1 entsprechen, um eine Verbindung nach der allgemeinen Formel II zu erhalten, wobei X = O oder N ist und R9 für eine OH-Gruppe steht,
und anschließend möglicherweise einen Acylierungs- oder Alkylierungsschritt umfassend, um eine Verbindung nach der allgemeinen Formel II zu erhalten, wobei R₉ eine O-CO-Alkylgruppe, eine O-Allylgruppe, eine O-Benzylgruppe, eine O-COCH₂CO-Alkylgruppe, wobei der Alkylrest
gesättigt oder ungesättigt, geradkettig oder verzweigt mit 1 bis 20 Kohlenstoffatomen ist, oder eine O-CO-Cycloalkylgruppe, die 3 bis 8 Kohlenstoffatome aufweist, ist,
- oder W für eine Gruppe der folgenden Formel II'-W steht: 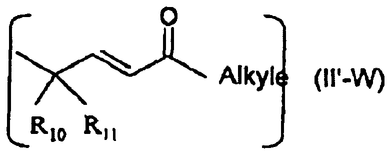 wobei das Alkyl gesättigt oder ungesättigt, geradkettig oder verzweigt mit 1 bis 17 Kohlenstoffatomen ist, oder für ein Cycloalkyl mit 3 bis 8 Kohlenstoffatomen, und X = CH₂ ist; Y für eine Gruppe (Pr₃)Si- steht,
und Z für eine Gruppe (Pr₃)Si-O- steht,
um eine Verbindung nach der allgemeinen Formel II zu erhalten, wobei X = CH₂ ist und R9 für eine OH-Gruppe steht,
und anschließend möglicherweise einen Acylierungs- oder Alkylierungsschritt umfassend, um eine Verbindung nach der allgemeinen Formel II zu erhalten, wobei R₉ eine O-CO-Alkylgruppe, eine O-Allylgruppe, eine O-Benzylgruppe, eine O-COCH₂CO-Alkylgruppe, wobei der Alkylrest gesättigt oder ungesättigt, geradkettig oder verzweigt mit 1 bis 20 Kohlenstoffatomen ist, oder eine O-CO-Cycloalkylgruppe, die 3 bis 8 Kohlenstoffatome aufweist, ist,
- oder W für eine Gruppe III-W steht: 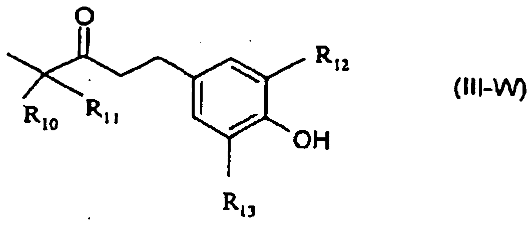 und Z für Folgendes steht:
• eine OH-Gruppe,
• eine gesättigte oder ungesättigte Alkylgruppe, die geradkettig oder verzweigt mit 1 bis 20 Kohlenstoffatomen ist, eine Cycloalkylgruppe mit 3 bis 8 Kohlenstoffatomen;
• eine Gruppe S-R", wobei R" ein gesättigtes oder ungesättigtes Alkyl ist, das geradkettig oder verzweigt mit 1 bis 20 Kohlenstoffatomen ist, oder eine Cycloalkylgruppe mit 3 bis 8 Kohlenstoffatomen,
• eine O-Alkylgruppe, wobei das Alkyl gesättigt oder ungesättigt, geradkettig oder verzweigt mit 1 bis 20 Kohlenstoffatomen ist, eine O-Cycloalkylgruppe mit 3 bis 8 Kohlenstoffatomen, eine Gruppe O-CH₂-CH₂-N(CH₃)₂; eine Gruppe O-CH₂-CCH,
• eine Gruppe N (R") (R"'), wobei R" und R"' den obigen Begriffsbestimmungen entsprechen,
wobei R₃ bis R₈ und R₁₀ bis R₁₃ den Begriffsbestimmungen in Anspruch 1 entsprechen,
um eine Verbindung nach der allgemeinen Formel III zu erhalten, wobei R9 für Folgendes steht:
• eine OH-Gruppe,
• eine gesättigte oder ungesättigte Alkylgruppe, die geradkettig oder verzweigt mit 1 bis 20 Kohlenstoffatomen ist, eine Cycloalkylgruppe mit 3 bis 8 Kohlenstoffatomen;
• eine Gruppe S-(O)-R", wobei R" ein gesättigtes oder ungesättigtes Alkyl ist, das geradkettig oder verzweigt mit 1 bis 20 Kohlenstoffatomen ist, oder ein Cycloalkyl mit 3 bis 8 Kohlenstoffatomen,
• eine O-Alkylgruppe, wobei das Alkyl gesättigt oder ungesättigt, geradkettig oder verzweigt mit 1 bis 20 Kohlenstoffatomen ist, eine O-Cycloalkylgruppe mit 3 bis 8 Kohlenstoffatomen, eine Gruppe O-CH₂-CH₂-N(CH₃)₂; eine Gruppe O-CH₂-CCH,
• eine Gruppe N (R") (R"'), wobei R" und R"' den obigen Begriffsbestimmungen entsprechen,
und, wenn Z eine OH-Gruppe ist (Verbindung III-1), möglicherweise einen Acylierungs- oder Alkylierungsschritt umfassend, um eine Verbindung nach der allgemeinen Formel III zu erhalten, wobei R₉ eine O-Alkylgruppe, eine O-Cycloalkylgruppe, eine O-CO-Alkylgruppe, eine O-COCH₂CO-Alkylgruppe, eine Gruppe O-CH₂-CH₂-N(CH₃)₂; eine Gruppe O-CH₂-CCH ist, und/oder, wenn Z für eine Gruppe -O-CH₂-CCH steht, möglicherweise eine Additionsreaktion mit N₃-R"' umfassend, um eine Verbindung nach der allgemeinen Formel III zu erhalten
wobei R₉ eine Gruppe 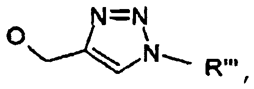 wobei R"' der Begriffsbestimmung in Anspruch 1 entspricht.

17. Verfahren zur Herstellung der Verbindungen nach Formel II gemäß dem Anspruch 16, wobei X = O, N und welches die folgenden Schritte umfasst:
• Reaktion eines Aldehyds nach der folgenden Formel (IV): 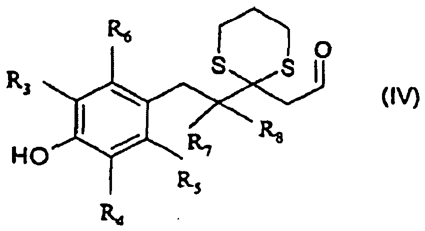 wobei R₃ bis R₈ den Begriffsbestimmungen in Anspruch 1 entsprechen,
mit einer Verbindung nach der folgenden Formel VII: 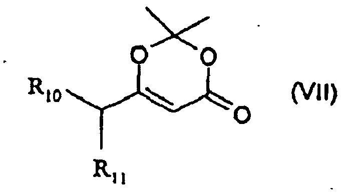 um eine Verbindung nach der folgenden Formel (V) zu erhalten: 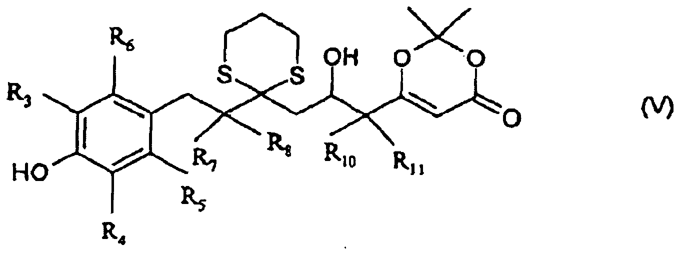 wobei R₃ bis R₈ und R₁₀ bis R₁₁ den Begriffsbestimmungen in Anspruch 1 entsprechen,
• Reaktion der erhaltenden Verbindung (V) mit einer Verbindung R'-X-H, (X = O) oder (R')(R")-X-H (X = N), wobei R' und R" den Begriffsbestimmungen in Anspruch 1 entsprechen, woraufhin eine Reduktion mit einem Reduktionsmittel erfolgt, das aus NaBH₄ oder NaBH₄ und Et₃B oder Me₄NBH(OAc)₃ ausgewählt ist, um eine Verbindung nach der Formel (I-Z) zu erhalten, wobei W für die Gruppe II-W steht;
• oxidative Phenolkupplung der erhaltenen Verbindung (I-Z) mit einem Oxidationsmittel, das aus PIFA und PIDA ausgewählt ist, um eine Verbindung II zu erhalten, wobei R₉ gleich OH ist;
• möglicherweise Acylierungs- oder Alkylierungsreaktion der Verbindung II, wobei R₉ gleich OH ist, um eine Verbindung nach der allgemeinen Formel II zu erhalten, wobei R₉ für eine O-Allylgruppe, eine O-Benzylgruppe, eine O-CO-Alkylgruppe oder eine O-CO-Cycloalkylgruppe gemäß den Begriffsbestimmungen in Anspruch 1 steht.

18. Verfahren zur Herstellung der Verbindungen nach Formel II gemäß dem Anspruch 16, wobei X = CH₂ und welches die folgenden Schritte umfasst:
• Reaktion eines Esters nach der folgenden Formel (VIII): 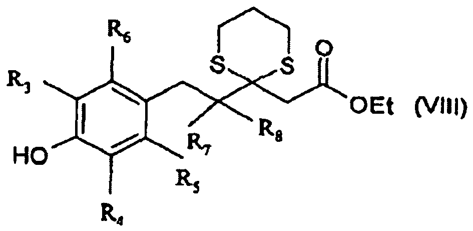 wobei R₃ bis R₈ den Begriffsbestimmungen in Anspruch 1 entsprechen,
mit einem tert.-Butylacetat 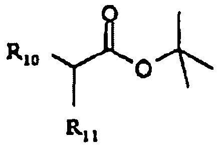 in Gegenwart einer Base wie etwa LDA, um die Verbindung nach der folgenden Formel (IX) zu erhalten: 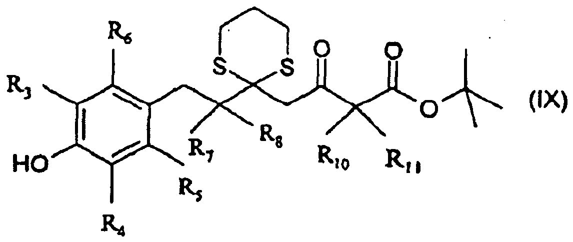 wobei R₃ bis R₈ den Begriffsbestimmungen in Anspruch 1 entsprechen,
• Reduktion der erhaltenen Verbindung (IX), woraufhin eine Reaktion mit Tripropylsilylchlorid zur Verbindung nach der folgenden Formel X führt: 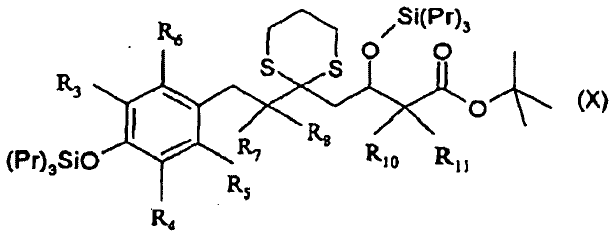
• Reduktion der erhaltenen Verbindung (X) mit einem Reduktionsmittel, was zur Verbindung nach der folgenden Formel (XI) führt: 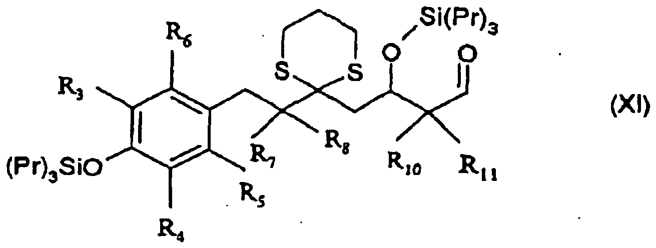
• Wittig-Reaktion mit der erhaltenen Verbindung XI, um eine Verbindung nach der Formel (I-Z) zu erhalten, wobei W für die Gruppe II'-W steht;
• oxidative Phenolkupplung der erhaltenen Verbindung (I-Z) mit einem Oxidationsmittel, das aus PIFA und PIDA ausgewählt ist, um eine Verbindung II zu erhalten, wobei R₉ gleich OH und X = CH₂ ist;
• möglicherweise Acylierungs- oder Alkylierungsreaktion der Verbindung II, wobei R₉ gleich OH und X = CH₂ ist, um eine Verbindung nach der allgemeinen Formel II zu erhalten, wobei X = CH₂ ist und R₉ für eine O-Allylgruppe, eine O-Benzylgruppe, eine O-CO-Alkylgruppe oder eine O-CO-Cycloalkylgruppe gemäß den Begriffsbestimmungen in Anspruch 1 steht.

19. Verfahren zur Herstellung von Verbindungen nach der Formel III gemäß Anspruch 16, wobei die Verbindung I-Z erhalten wird, indem ein Nukleophil mittels Michael-Addition an eine Verbindung nach der folgenden Formel III-3 angelagert wird 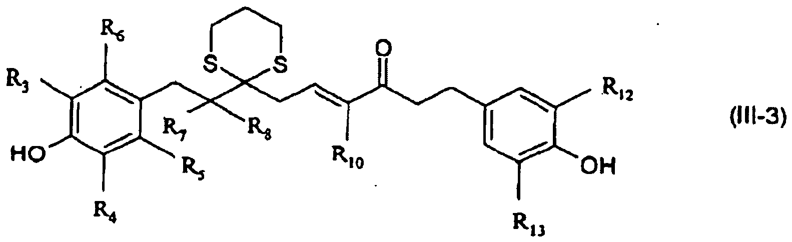 wobei W für die Gruppe III-W steht und Z für Folgendes steht:
• eine gesättigte oder ungesättigte Alkylgruppe, die geradkettig oder verzweigt mit 1 bis 20 Kohlenstoffatomen ist, eine Cycloalkylgruppe mit 3 bis 8 Kohlenstoffatomen;
• eine Gruppe S-R", wobei R" ein gesättigtes oder ungesättigtes Alkyl ist, das geradkettig oder verzweigt mit 1 bis 20 Kohlenstoffatomen ist, oder eine Cycloalkylgruppe mit 3 bis 8 Kohlenstoffatomen,
• eine O-Alkylgruppe gemäß der Begriffsbestimmung in Anspruch 1, eine O-Cycloalkylgruppe mit 3 bis 8 Kohlenstoffatomen, eine Gruppe O-CH₂-CH₂-N(CH₃)₂; eine Gruppe O-CH₂-CCH,
• eine Gruppe N(R") (R"'), wobei R" und R"' den obigen Begriffsbestimmungen entsprechen,
wobei die Verbindung III-3 durch Wasserabspaltung einer Verbindung I-Z erhalten wird, bei welcher Z = OH ist und welche die folgende Formel (III-1) hat: 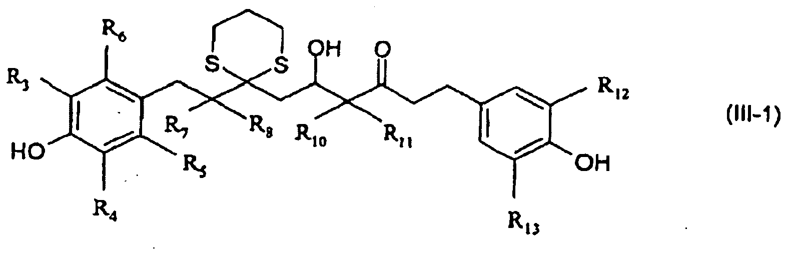 wobei R₁₁ für Wasserstoff steht.
Wobei R₃ bis R₈, R₁₀ bis R₁₃, R" und R"' den Begriffsbestimmungen in Anspruch 1 entsprechen.

20. Verfahren zur Herstellung einer Verbindung nach der Formel III gemäß Anspruch 16 oder 19, wobei die Verbindung I-Z, falls Z eine OH-Gruppe ist, durch Reaktion eines Aldehyds nach der folgenden Formel (IV): 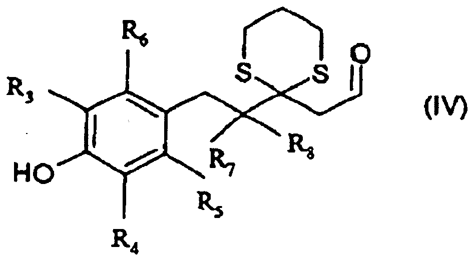 wobei R₃ bis R₈ den Begriffsbestimmungen in Anspruch 1 entsprechen, mit einer Verbindung nach der folgenden Formel (VI): 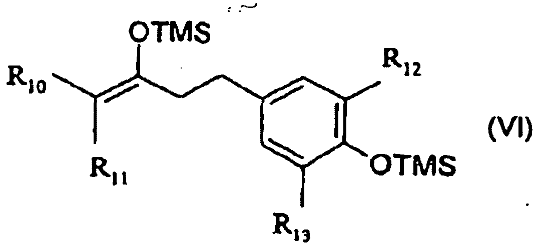 wobei R₁₀ bis R₁₃ den Begriffsbestimmungen in Anspruch 1 entsprechen, erhalten wird.

21. Verfahren gemäß Anspruch 16 zur Herstellung der Verbindungen nach der Formel III, wobei R₉ für Folgendes steht:
• eine OH-Gruppe,
eine O-Alkylgruppe, eine O-CO-Alkylgruppe, eine O-COCH₂CO-Alkylgruppe, wobei das Alkyl gesättigt oder ungesättigt, geradkettig oder verzweigt mit 1 bis 20 Kohlenstoffatomen ist, oder eine O-Cycloalkylgruppe mit 3 bis 8 Kohlenstoffatomen,
die folgenden Schritte umfassend:
• Reaktion eines Aldehyds nach der folgenden Formel (IV): 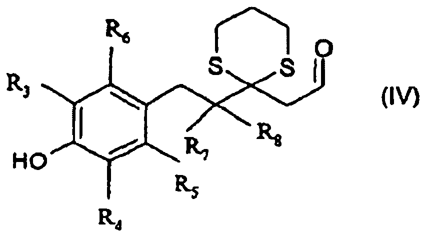 wobei R₃ bis R₈ den Begriffsbestimmungen in Anspruch 1 entsprechen,
mit einer Verbindung nach der folgenden Formel (VI): 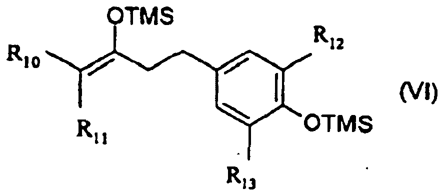 wobei R₁₀ bis R₁₃ den Begriffsbestimmungen in Anspruch 1 entsprechen,
um eine Verbindung I-Z nach der folgenden Formel (III-1) zu erhalten, wobei Z = OH ist: 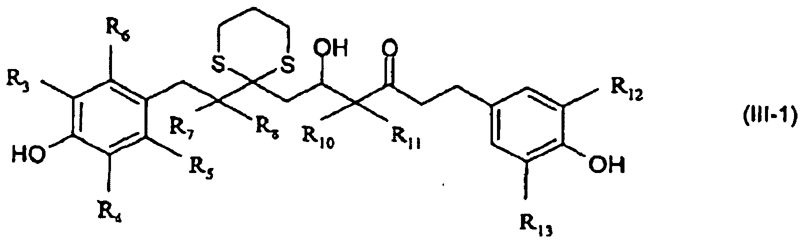
• oxidative Phenolkupplung der erhaltenen Verbindung (III-1) mit einem Oxidationsmittel, das aus PIFA und PIDA ausgewählt ist, um eine Verbindung III zu erhalten, wobei R₉ gleich OH ist;
• möglicherweise Acylierungs- oder Alkylierungsreaktion der Verbindung III, wobei R₉ eine OH-Gruppe ist, um eine Zielverbindung nach der Formel III zu erhalten, wobei R₉ eine O-Allylgruppe, eine O-Benzylgruppe, eine O-CO-Alkylgruppe, eine O-COCH₂CO-Alkylgruppe, eine O-CO-Cycloalkylgruppe ist.

22. Verfahren gemäß dem Anspruch 19 oder 20 zur Herstellung der Verbindungen nach der Formel III, wobei R9 eine Alkylgruppe gemäß der Begriffsbestimmung in Anspruch 1 ist, die folgenden Schritte umfassend:
• Anlagerung eines Nukleophils CuXRa mittels Michael-Addition an die oben erhaltene Verbindung III-3, wobei Ra für eine Alkylgruppe gemäß der Begriffsbestimmung in Anspruch 1 steht, was zur Verbindung I-Z nach der folgenden Formel (III-4) führt, wobei Z = Ra ist: 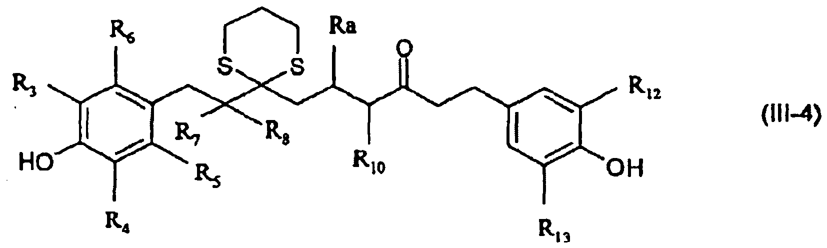 wobei R₃ bis R₈, R₁₀, R₁ und R₁₃ den Begriffsbestimmungen in Anspruch 1 entsprechen und Ra für eine Alkylgruppe gemäß der Begriffsbestimmung in Anspruch 1 steht.
• oxidative Phenolkupplung der erhaltenen Verbindung (III-4) mit einem Oxidationsmittel, das aus PIFA und PIDA ausgewählt ist, um eine Verbindung III zu erhalten, wobei R₉ eine Alkylgruppe gemäß der Begriffsbestimmung in Anspruch 1 ist.

23. Verfahren gemäß dem Anspruch 19 oder 20 zur Herstellung der Verbindungen nach der Formel III, wobei R9 eine Gruppe N(R") (R"') ist, wobei R" und R"' den obigen Begriffsbestimmungen entsprechen, die folgenden Schritte umfassend:
• Anlagerung eines Nukleophils HN(R")(R"') mittels Michael-Addition vom Heterotyp an die oben erhaltene Verbindung III-3, was zur Verbindung I-Z nach der folgenden Formel (III-5) führt, wobei Z = N(R")(R"') ist: 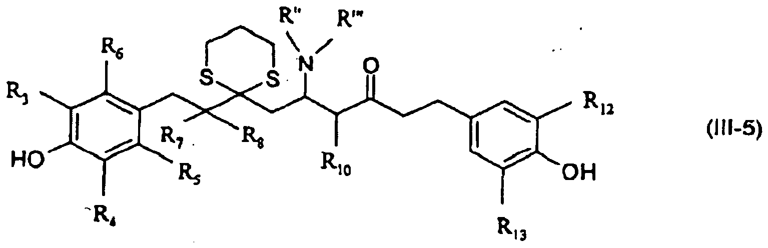 wobei R₃ bis R₈, R₁₀, R₁₂ und R₁₃ den Begriffsbestimmungen in Anspruch 1 entsprechen,
• oxidative Phenolkupplung der erhaltenen Verbindung (III-5) mit einem Oxidationsmittel, das aus PIFA und PIDA ausgewählt ist, um eine Verbindung III zu erhalten, wobei R9 eine Gruppe N(R")(R"') gemäß der obigen Begriffsbestimmung ist.

24. Verfahren gemäß dem Anspruch 19 oder 20 zur Herstellung der Verbindungen nach der Formel III, wobei R9 eine Gruppe S(O)-R" ist, wobei R" der obigen Begriffsbestimmung entspricht, die folgenden Schritte umfassend:
• Anlagerung eines Nukleophils R"SH, wobei R" der obigen Begriffsbestimmung entspricht, mittels Michael-Addition vom Hetero-Typ an die oben erhaltene Verbindung III-3, was zur Verbindung I-Z nach der folgenden Formel (III-6) führt, wobei Z = SR" ist: 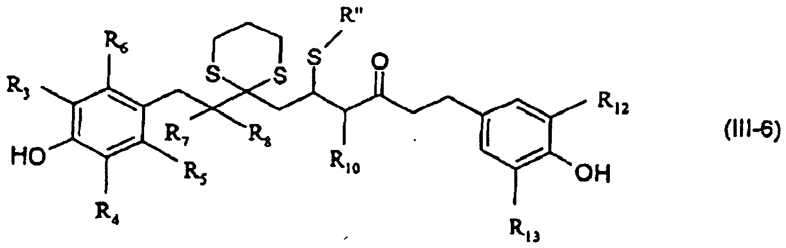 wobei R₃ bis R₈, R₁₀, R₁₂, R₁₃ und R" den Begriffsbestimmungen in Anspruch 1 entsprechen,
• oxidative Phenolkupplung der erhaltenen Verbindung (III-6) mit einem Oxidationsmittel, das aus PIFA und PIDA ausgewählt ist, um eine Verbindung III zu erhalten, wobei R9 eine Gruppe -S(O)R" ist, wobei R" der Begriffsbestimmung in Anspruch 1 entspricht.

25. Verfahren gemäß dem Anspruch 19 oder 20 zur Herstellung der Verbindungen nach der Formel III, wobei R₉ eine O-Alkylgruppe gemäß der Begriffsbestimmung in Anspruch 1, eine Gruppe O-CH₂-N(CH₃)₂; eine Gruppe O-CH₂-CCH,
eine Gruppe 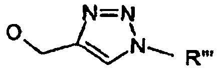 ist, wobei R"' der Begriffsbestimmung in Anspruch 1 entspricht, die folgenden Schritte umfassend:
• Anlagerung eines Nukleophils ROH mittels Michael-Addition vom Heterotyp an die oben erhaltene Verbindung III-3, wobei R ein Alkyl gemäß der Begriffsbestimmung in Anspruch 1, eine Gruppe - CH₂-CH₂- N(CH₃)2; eine Gruppe -CH₂-CCH ist, was zur Verbindung I-Z nach der folgenden Formel (III-2) führt, wobei Z = OR ist: 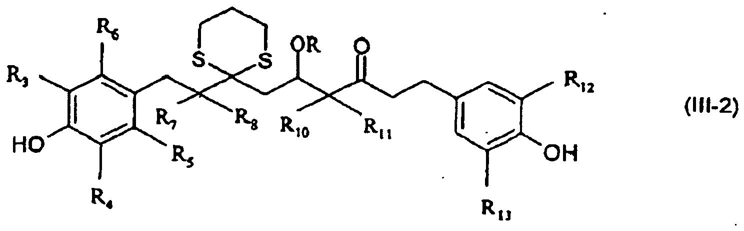 wobei R₃ bis R₈ sowie R₁₀, R₁₂ und R₁₃ den Begriffsbestimmungen in Anspruch 1 entsprechen und R₁₁ für Wasserstoff steht,
• oxidative Phenolkupplung der erhaltenen Verbindung (III-2) mit einem Oxidationsmittel, das aus PIFA und PIDA ausgewählt ist, um eine Verbindung III zu erhalten, wobei R₉ eine O-Alkylgruppe gemäß der Begriffsbestimmung in Anspruch 1 ist, eine Gruppe O-CH₂-CH₂-N(CH₃)_{2;} eine Gruppe O-CH₂-CCH ist,
• möglicherweise Additionsreaktion der oben erhaltenen Verbindung III, wobei R₉ eine Gruppe O-CH₂-CCH ist, mit N₃-R"', um die Zielverbindung nach der Formel III zu erhalten, wobei R₉ eine Gruppe 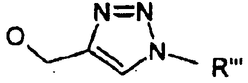 ist, wobei R"' der Begriffsbestimmung in Anspruch 1 entspricht.
